# EUROPEAN PATENT SPECIFICATION

(11) **EP 4 310 077 B1**
(45) Date of publication and mention of the grant of the patent: **08.07.2026**
(21) Application number: 22770630.6
(22) Date of filing: 18.03.2022
(51) Int. Cl.: C07D 271/07, C07D 413/12, C07D 417/12, A61K 31/4245, A61P 1/16, A61P 5/14

(54) **BICYCLIC PHENOL COMPOUNDS AND USE THEREOF**
BICYCLISCHE PHENOLVERBINDUNGEN UND IHRE VERWENDUNG
COMPOSÉS PHÉNOLIQUES BICYCLIQUES ET LEUR UTILISATION

(30) Priority: 19.03.2021 CN 202110298570; 23.04.2021 CN 202110443424; 28.01.2022 CN 202210107896
(43) Date of publication of application: 24.01.2024
(73) Proprietor: Guangdong Raynovent Biotech Co., Ltd., Huangpu District Guangzhou 510700 (CN)
(72) Inventor: ZHANG, Shengbin, Shanghai 200131 (CN); YU, Tao, Shanghai 200131 (CN); WU, Chengde, Shanghai 200131 (CN); LIU, Jinxin, Shanghai 200131 (CN); LI, Jian, Shanghai 200131 (CN); CHEN, Shuhui, Shanghai 200131 (CN)
(74) Representative: Dehns
(86) International application number: PCT/CN2022/081686
(87) International publication number: WO 2022/194278

(56) References cited:
- WO-A1-2020/041741
- WO-A1-2021/041237
- WO-A1-2021/050945
- US-A1- 2008 090 840

## Description

The present application claims the right of the following priorities:
CN202110298570.3, application date: March 19, 2021;
CN202110443424.5, application date: April 23, 2021;
CN202210107896.8, application date: January 28, 2022.

### TECHNICAL FIELD

The present disclosure relates to a series of bicyclic phenol compounds and a use thereof, specifically to a compound of formula (III) and a pharmaceutically acceptable salt thereof.

### BACKGROUND

There are two subtypes of thyroid hormone receptors (THR): THRα and THRβ. Over the past several decades, various THRβ agonists (such as GC-1, KB141, KB2115) have been developed for the treatment of metabolic disorders such as dyslipidemia, non-alcoholic fatty liver, and non-alcoholic steatohepatitis. However, skeletal and cardiac side effects have hindered their further development. For instance, KB2115 was halted in Phase III clinical studies due to cartilage damage found in dogs. THRα is mainly distributed in the brain, heart, and skeletal muscles, and it can enhance the activity of osteoclasts, leading to a reduction in bone density. Therefore, people believe these side effects are caused by the agonism of THRα subtype, and hope to avoid them by increasing target selectivity and liver tissue selectivity. The representative agonists of this strategy are MGL-3196 and VK-2809, of which the safety and efficacy are both preliminarily validated through clinical trials. Therefore, the development of thyroid hormone analogs with specific distribution in liver tissue and high selectivity for thyroid hormone receptor subtypes holds significant clinical value.

According to the literature (J. Med. Chem. 2014, 57, 3912-3923), the structure of the THRβ agonist MGL-3196 is as follows:

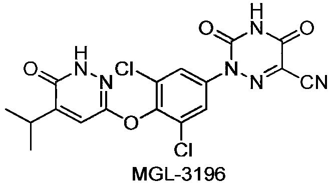

WO2021/041237 discloses compounds as thyroid hormone receptor agonists.

### CONTENT OF THE PRESENT INVENTION

The invention is defined in the appended claims. That is to say, only compounds of formula (I) and subject-matter referring to them form part of the invention. Compounds of formula (III) which do not fall within formula (I) are not part of the invention. Embodiments not encompassed by the claims are provided for reference purposes. Any references in the description to methods of treatment refer to the compounds or compositions of the invention for use in a method of treatment of the human (or animal) body by therapy.

The present disclosure provides a compound of formula (III) or a pharmaceutically acceptable salt thereof, 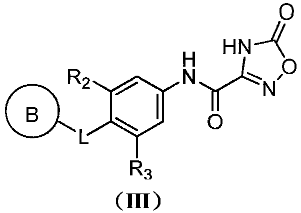 wherein
ring B is selected from 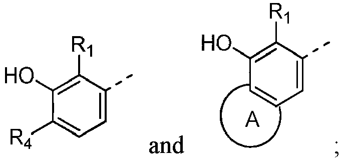
R₁ is independently selected from H, F, Cl, Br, I, and C₁₋₃ alkyl, and the C₁₋₃ alkyl is optionally substituted by 1, 2, or 3 Rₐ;
R₂ and R₃ are each independently selected from H, F, Cl, Br, I, OH, NH₂, CN, C₁₋₃ alkyl, and C₁₋₃ alkoxy, and the C₁₋₃ alkyl and C₁₋₃ alkoxy are optionally substituted by 1, 2, or 3 R_{b};
R₄ is selected from C₁₋₃ alkyl-phenyl;
L is selected from -O- and -CH₂-;
ring A is selected from phenyl, 5- to 6-membered heteroaryl, 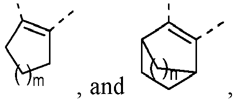 and the phenyl, 5- to 6-membered heteroaryl, 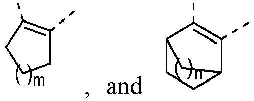 are optionally substituted by 1, 2, or 3 R_{c};
n and m are each independently selected from 0, 1, and 2;
Rₐ and R_{b} are each independently selected from F, Cl, Br, and I;
R_{c} is independently selected from F, Cl, Br, I, =O, =N-C₁₋₃ alkoxy, C₁₋₃ alkyl, and C₁₋₃ alkoxy, and the =N-C₁₋₃ alkoxy, C₁₋₃ alkyl, and C₁₋₃ alkoxy are optionally substituted by 1, 2, or 3 halogens.

In some embodiments of the present disclosure, the R₁ is independently selected from H, F, Cl, Br, I, and CH₃, and the CH₃ is optionally substituted by 1, 2, or 3 Rₐ, and other variables are as defined in the present disclosure.

In some embodiments of the present disclosure, the R₁ is independently selected from H, F, Cl, Br, I, CH₃, and CF₃, and other variables are as defined in the present disclosure.

In some embodiments of the present disclosure, the R₂ and R₃ are each independently selected from H, F, Cl, Br, I, OH, NH₂, CN, CH₃, and OCH₃, and the CH₃ and OCH₃ are optionally substituted by 1, 2, or 3 R_{b}, and other variables are as defined in the present disclosure.

In some embodiments of the present disclosure, the R₂ and R₃ are each independently selected from H, F, Cl, Br, I, OH, NH₂, CN, CH₃, CH₂F, CHF₂, CF₃, and OCH₃, and other variables are as defined in the present disclosure.

In some embodiments of the present disclosure, the R_{c} is independently selected from F, Cl, Br, I, =O, =N-O-CH₃, =N-O-CH₂CH₃, CH₃, OCH₃, and OCH₂CH₃, and the =N-O-CH₃, =N-O-CH₂CH₃, CH₃, OCH₃, and OCH₂CH₃ are optionally substituted by 1, 2, or 3 halogens, and other variables are as defined in the present disclosure.

In some embodiments of the present disclosure, the R_{c} is independently selected from F, Cl, Br, I, =O, =N-O-CH₃, =N-O-CH₂CH₃, CH₃, OCH₃, and OCH₂CH₃, and the =N-O-CH₃, =N-O-CH₂CH₃, CH₃, OCH₃, and OCH₂CH₃ are optionally substituted by 1, 2, or 3 R, and other variables are as defined in the present disclosure.

In some embodiments of the present disclosure, the R_{c} is independently selected from F, Cl, Br, I, =O, =N-O-CH₃, =N-O-CH₂CH₃, CH₃, OCH₃, and OCH₂CH₃, and other variables are as defined in the present disclosure.

In some embodiments of the present disclosure, the ring A is selected from 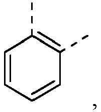 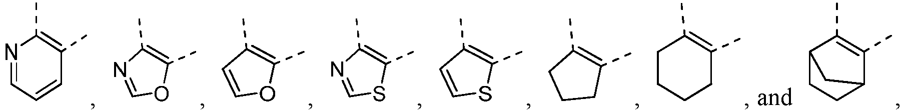 and the 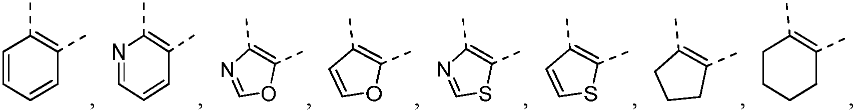 and 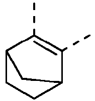 are optionally substituted by 1, 2, or 3 R_{c}, and other variables are as defined in the present disclosure.

In some embodiments of the present disclosure, the ring A is selected from 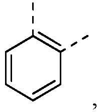 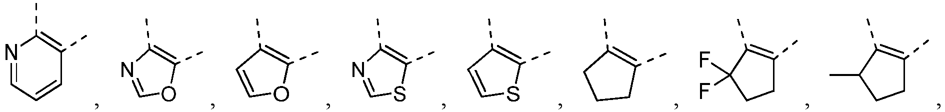 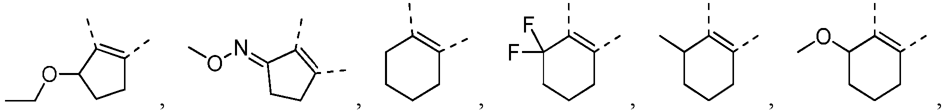 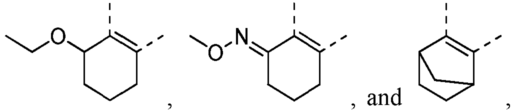 and other variables are as defined in the present disclosure.

In some embodiments of the present disclosure, the R₄ is selected from 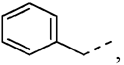 and other variables are as defined in the present disclosure.

In some embodiments of the present disclosure, the structural moiety 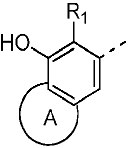 is selected from 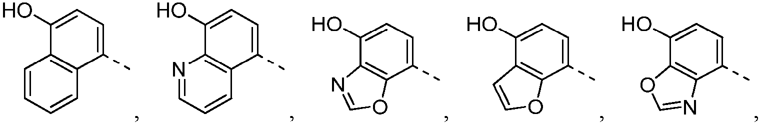 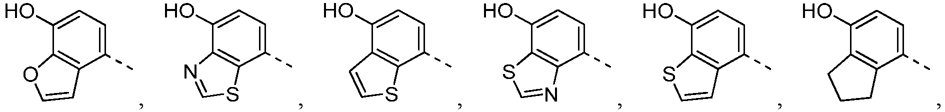 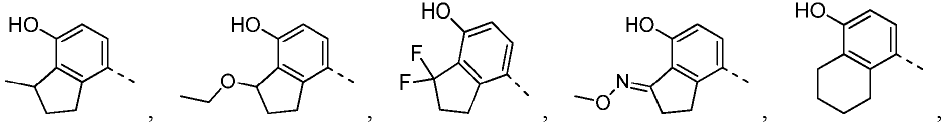 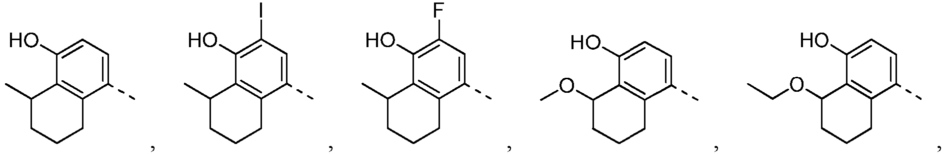 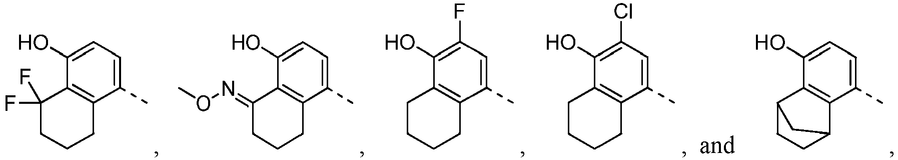 and other variables are as defined in the present disclosure.

The present disclosure provides a compound of formula (I) or a pharmaceutically acceptable salt thereof, 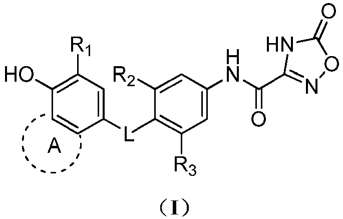 wherein
R₁ is independently selected from H, F, Cl, Br, I, and C₁₋₃ alkyl, and the C₁₋₃ alkyl is optionally substituted by 1, 2, or 3 Rₐ;
R₂ and R₃ are each independently selected from H, F, Cl, Br, I, OH, NH₂, CN, C₁₋₃ alkyl, and C₁₋₃ alkoxy, and the C₁₋₃ alkyl and C₁₋₃ alkoxy are optionally substituted by 1, 2, or 3 R_{b};
L is selected from -O- and -CH₂-;
ring A is selected from phenyl, 5- to 6-membered heteroaryl, 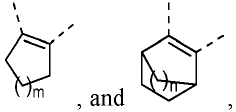 and the phenyl, 5- to 6-membered heteroaryl, 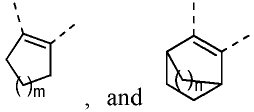 are optionally substituted by 1, 2, or 3 R_{c};
n and m are each independently selected from 0, 1, and 2;
Rₐ and R_{b} are each independently selected from F, Cl, Br, and I;
R_{c} is independently selected from F, Cl, Br, I, =O, =N-C₁₋₃ alkoxy, C₁₋₃ alkyl, and C₁₋₃ alkoxy, and the =N-C₁₋₃ alkoxy, C₁₋₃ alkyl, and C₁₋₃ alkoxy are optionally substituted by 1, 2, or 3 R;
R is independently selected from F, Cl, Br, and I.

In some embodiments of the present disclosure, the R₁ is independently selected from H, F, Cl, Br, I, and CH₃, and the CH₃ is optionally substituted by 1, 2, or 3 Rₐ, and other variables are as defined in the present disclosure.

In some embodiments of the present disclosure, the R₁ is independently selected from H, F, Cl, Br, I, CH₃, and CF₃, and other variables are as defined in the present disclosure.

In some embodiments of the present disclosure, the R₂ and R₃ are each independently selected from H, F, Cl, Br, I, OH, NH₂, CN, CH₃, and OCH₃, and the CH₃ and OCH₃ are optionally substituted by 1, 2, or 3 R_{b}, and other variables are as defined in the present disclosure.

In some embodiments of the present disclosure, the R₂ and R₃ are each independently selected from H, F, Cl, Br, I, OH, NH₂, CN, CH₃, CH₂F, CHF₂, CF₃, and OCH₃, and other variables are as defined in the present disclosure.

In some embodiments of the present disclosure, the R_{c} is independently selected from F, Cl, Br, I, =O, =N-O-CH₃, =N-O-CH₂CH₃, CH₃, OCH₃, and OCH₂CH₃, and the =N-O-CH₃, =N-O-CH₂CH₃, CH₃, OCH₃, and OCH₂CH₃ are optionally substituted by 1, 2, or 3 R, and other variables are as defined in the present disclosure.

In some embodiments of the present disclosure, the R_{c} is independently selected from F, Cl, Br, I, =O, =N-O-CH₃, =N-O-CH₂CH₃, CH₃, OCH₃, and OCH₂CH₃, and other variables are as defined in the present disclosure.

In some embodiments of the present disclosure, the ring A is selected from 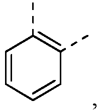 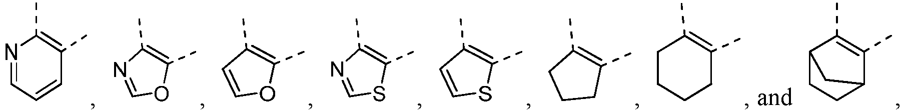 and the 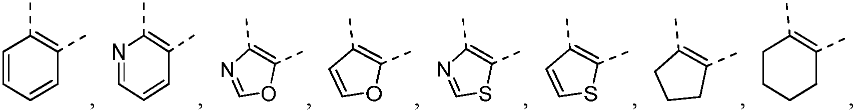 and 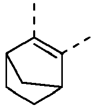 are optionally substituted by 1, 2, or 3 R_{c}, and other variables are as defined in the present disclosure.

In some embodiments of the present disclosure, the ring A is selected from 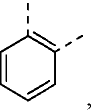 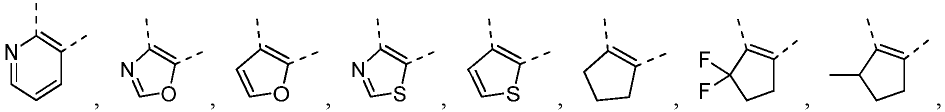 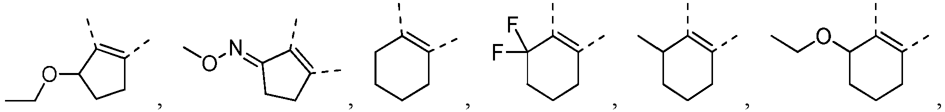 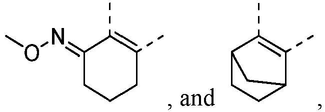 and other variables are as defined in the present disclosure.

In some embodiments of the present disclosure, the structural moiety 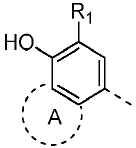 is selected from 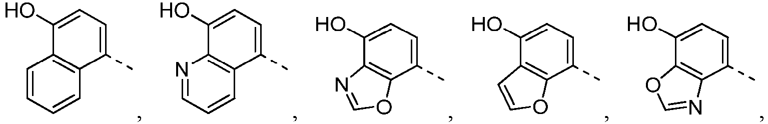 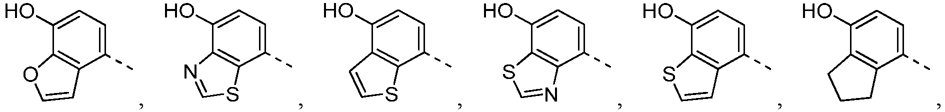 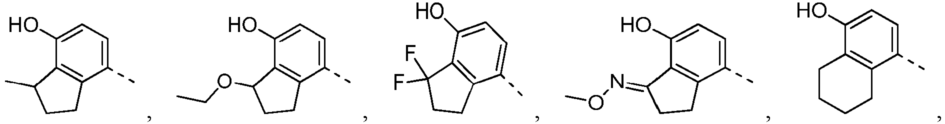 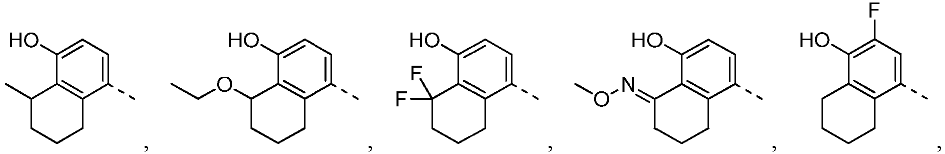 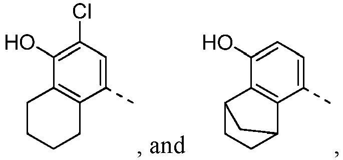 and other variables are as defined in the present disclosure.

In some embodiments of the present disclosure, the compound or the pharmaceutically acceptable salt thereof is selected from: 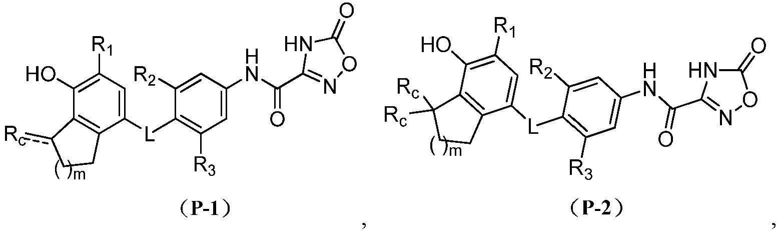 wherein
when 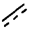 is a single bond, R_{c} is F, Cl, Br, I, C₁₋₃ alkyl, and C₁₋₃ alkoxy, and the C₁₋₃ alkyl and C₁₋₃ alkoxy are optionally substituted by 1, 2, or 3 halogens;
when 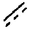 is a double bond, R_{c} is =O or =N-C₁₋₃ alkoxy, and the =N-C₁₋₃ alkoxy is optionally substituted by 1, 2, or 3 halogens;
R₁, R₂, R₃, L, and m are as defined in the present disclosure.

In some embodiments of the present disclosure, the compound or the pharmaceutically acceptable salt thereof is selected from: 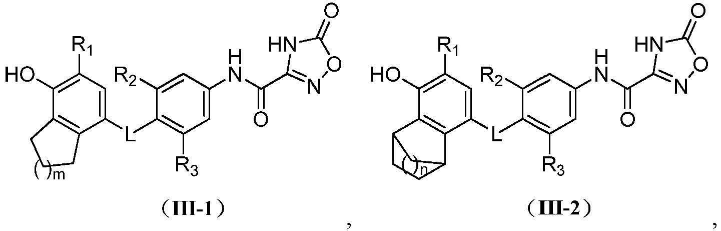 wherein R₁, R₂, R₃, L, m, and n are as defined in the present disclosure.

There are still some embodiments of the present disclosure which are obtained by any combination of the above variables.

The present disclosure also provides a compound of the following formula or a pharmaceutically acceptable salt thereof,

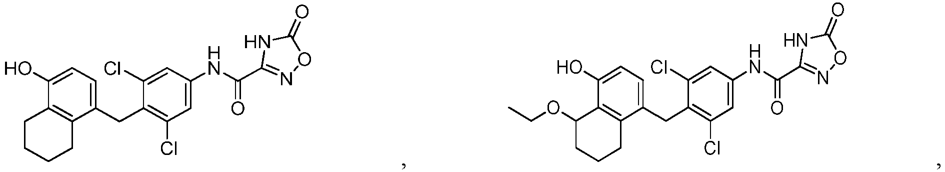

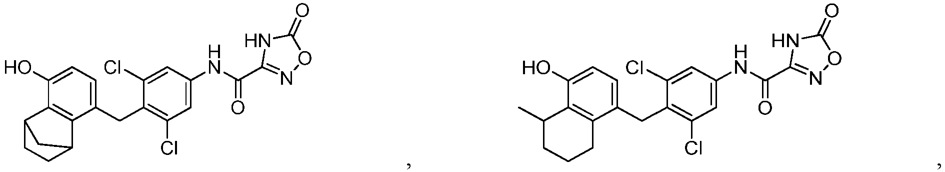

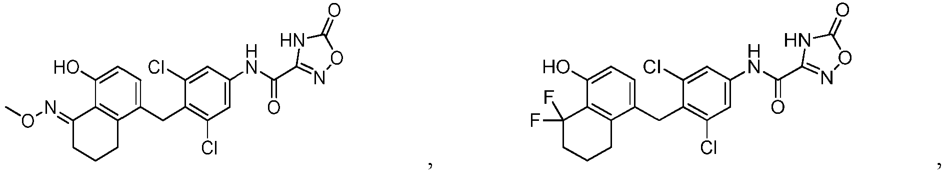

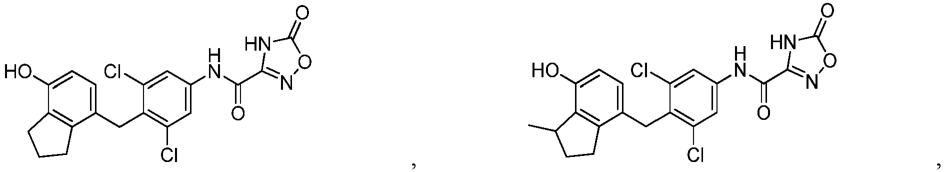

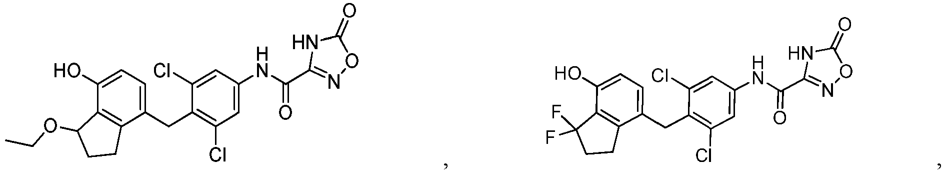

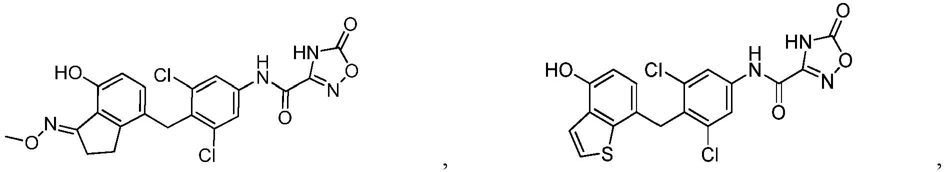

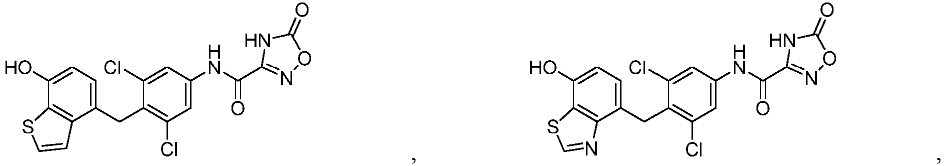

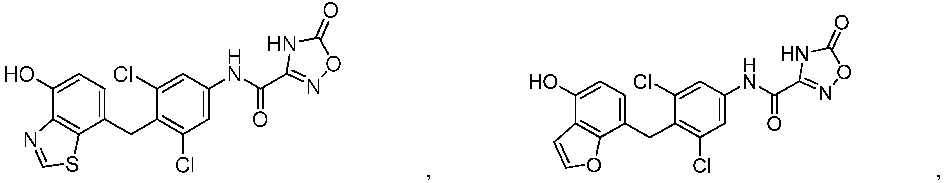

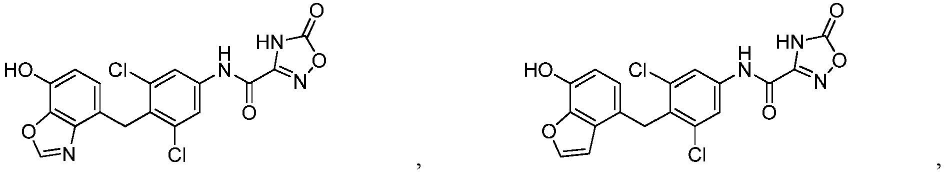

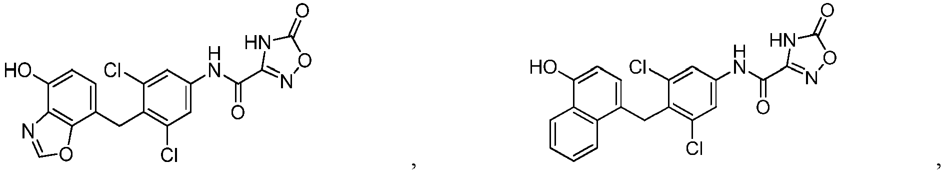

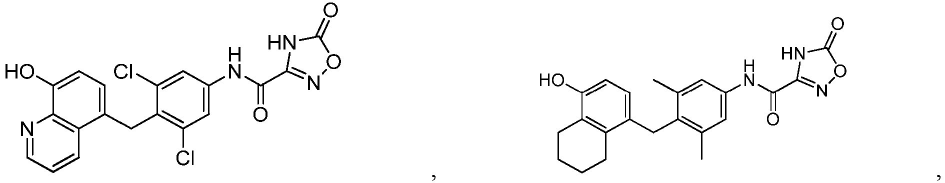

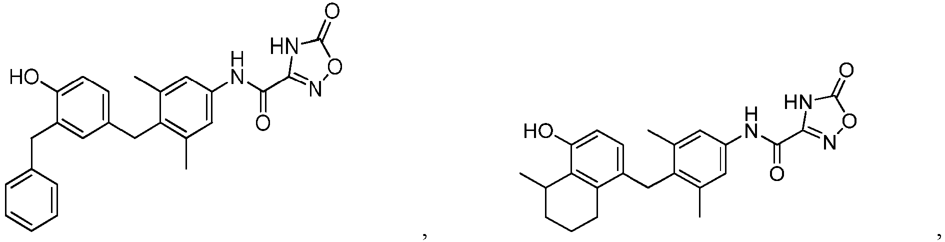

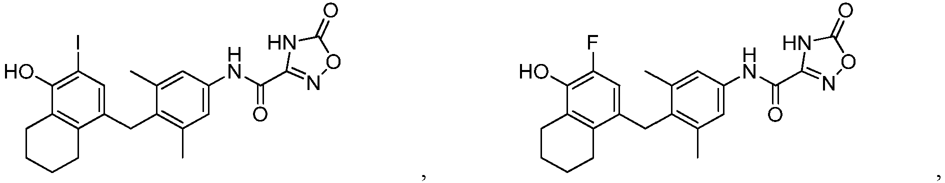

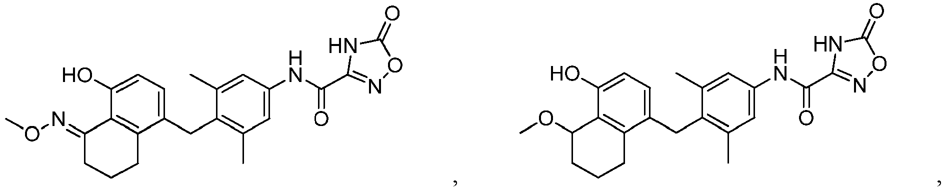

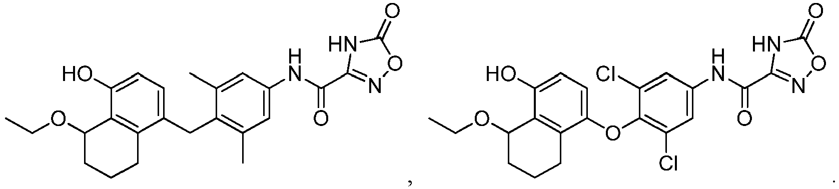

In some embodiments of the present disclosure, the compound or the pharmaceutically acceptable salt thereof is selected from:

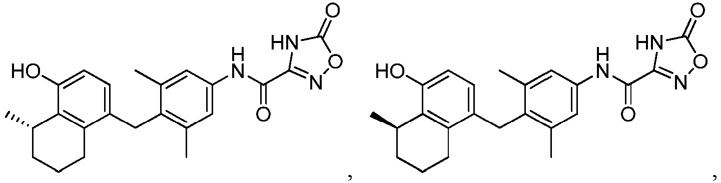

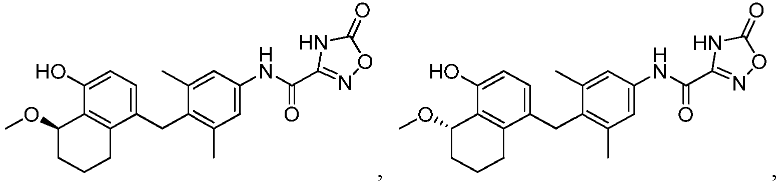

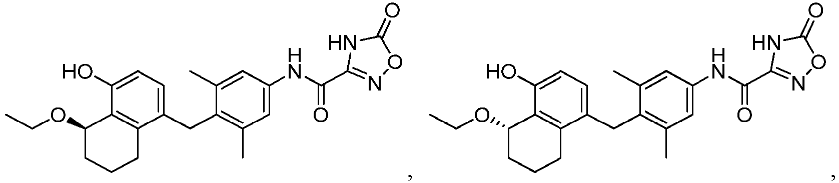

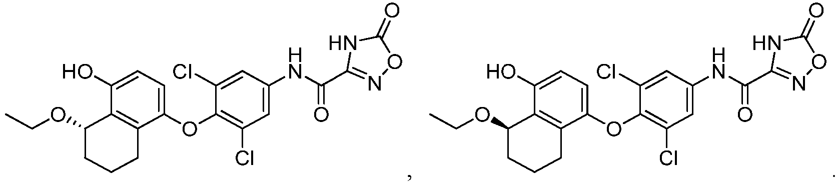

The present disclosure also provides a use of the compound or the pharmaceutically acceptable salt thereof in the manufacture of a medicament for treating non-alcoholic steatohepatitis.

The present disclosure also provides a synthesis method as follows: 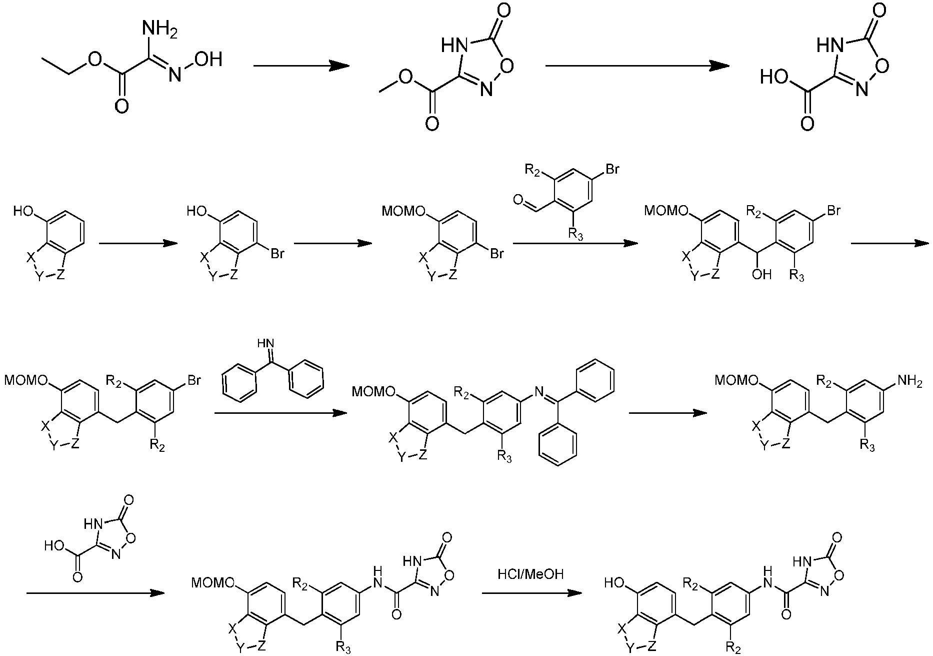 wherein
-X-Y-Z- is selected from cyclopentenyl, cyclohexenyl, phenyl, thienyl, thiazolyl, furyl, and oxazolyl;
R₂ and R₃ are each independently selected from H, F, Cl, Br, I, OH, NH₂, CN, C₁₋₃ alkyl, and C₁₋₃ alkoxy, and the C₁₋₃ alkyl and C₁₋₃ alkoxy are optionally substituted by 1, 2, or 3 R_{b};
each R_{b} is independently selected from F, Cl, Br, and I.

### Definition and description

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the compound into contact with a sufficient amount of base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by bringing the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of amino acid (such as arginine), and a salt of an organic acid such as glucuronic acid. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereomers isomers, (*D*)-isomers, (*L*)-isomers, and racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 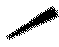 ) and a wedged dashed bond ( 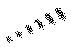 ), and the relative configuration of a stereogenic center is represented by a straight solid bond ( 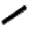 ) and a straight dashed bond ( 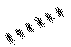 ), a wave line ( 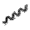 ) is used to represent a wedged solid bond ( 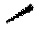 ) or a wedged dashed bond ( 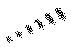 ), or the wave line ( 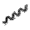 ) is used to represent a straight solid bond ( 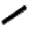 ) and a straight dashed bond ( 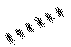 ).

Unless otherwise specified, when a double bond structure, such as carbon-carbon double bond, carbon-nitrogen double bond, and nitrogen-nitrogen double bond, exists in the compound, and each of the atoms on the double bond is connected to two different substituents (including the condition where a double bond contains a nitrogen atom, the lone pair of electrons attached on the nitrogen atom is regarded as a substituent connected), if the atom on the double bond in the compound is connected to its substituent by a wave line ( 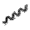 ), this refers to the (Z) isomer, (E) isomer or a mixture of two isomers of the compound. For example, the following formula (A) means that the compound exists as a single isomer of formula (A-1) or formula (A-2) or as a mixture of two isomers of formula (A-1) and formula (A-2); the following formula (B) means that the compound exists in the form of a single isomer of formula (B-1) or formula (B-2) or in the form of a mixture of two isomers of formula (B-1) and formula (B-2). The following formula (C) means that the compound exists as a single isomer of formula (C-1) or formula (C-2) or as two a mixture of two isomers of formula (C-1) and formula (C-2).

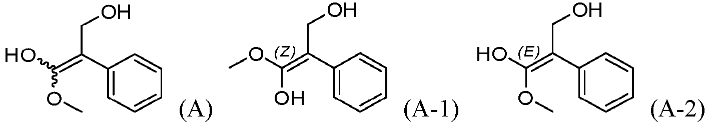

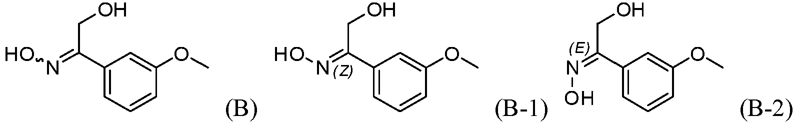

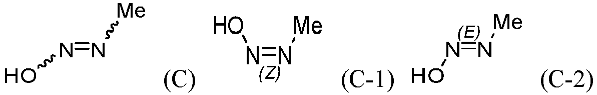

Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as ketoenol isomerization and imine-enamine isomerization. Valence tautomer includes some recombination of bonding electrons for mutual transformation. A specific example of ketoenol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer" or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomer, as well as (*D*)- and (*L*)-isomer can be prepared using chiral synthesis or chiral reagents or other conventional techniques. If one kind of enantiomer of certain compound of the present disclosure is to be obtained, the pure desired enantiomer can be obtained by asymmetric synthesis or derivatization of chiral auxiliary followed by separating the resulting diastereomeric mixture and cleaving the auxiliary group. Alternatively, when the molecule contains a basic functional group (such as amino) or an acidic functional group (such as carboxyl), the molecule reacts with an appropriate optically active acid or base to form a salt of the diastereoisomer, and then the diastereoisomer is resolved through the conventional method known in the art and recovered to obtain the pure enantiomer. In addition, the enantiomer and the diastereoisomer are generally separated through chromatography which uses a chiral stationary phase and optionally combines with chemical derivatization method (such as carbamate generated from amine).

The compound of the present disclosure may contain an unnatural proportion of atomic isotope at one or more than one atom that constitute the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted with the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone.

The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0 to 2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent is absent, for example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in 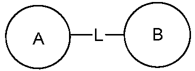 is -M-W-, then -M-W- can link ring A and ring B to form 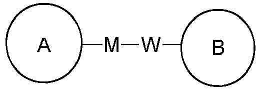 in the direction same as left-to-right reading order, and form 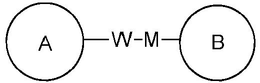 in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond ( 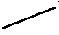 ), a straight dashed bond ( 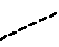 ), or a wavy line ( 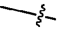 ). For example, the straight solid bond in -OCH₃ means that it is linked to other groups through the oxygen atom in the group; the straight dashed bond in 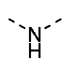 means that it is linked to other groups through the two ends of the nitrogen atom in the group; the wave lines in 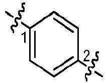 means that the phenyl group is linked to other groups through carbon atoms at position 1 and position 2; 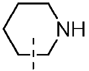 means that it can be linked to other groups through any linkable sites on the piperidinyl by one chemical bond, including at least four types of linkage, including 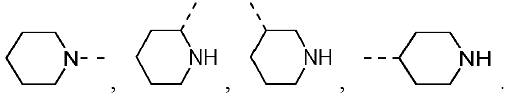 Even though the H atom is drawn on the -N-, 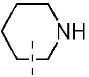 still includes the linkage of 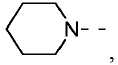 merely when one chemical bond was connected, the H of this site will be reduced by one to the corresponding monovalent piperidinyl.

Unless otherwise specified, the term "C₁₋₃ alkyl" refers to a linear or branched saturated hydrocarbon group consisting of 1 to 3 carbon atoms. The C₁₋₃ alkyl includes C₁₋₂ and C₂₋₃ alkyl, etc.; it can be monovalent (such as methyl), divalent (such as methylene) or multivalent (such as methine). Examples of C₁₋₃ alkyl include but are not limited to methyl (Me), ethyl (Et), propyl (including *n*-propyl and isopropyl) and the like.

Unless otherwise specified, the term "C₁₋₃ alkoxy" refers to an alkyl group consisting of 1 to 3 carbon atoms that are connected to the rest of the molecule through an oxygen atom. The C₁₋₃ alkoxy includes C₁₋₂, C₂₋₃, C₃, and C₂ alkoxy, etc. Examples of C₁₋₃ alkoxy include, but are not limited to, methoxy, ethoxy, propoxy (including *n*-propoxy and isopropoxy) and the like.

Unless otherwise specified, the terms "5- to 6-membered heteroaryl ring" and "5- to 6- membered heteroaryl" are used interchangeably. The term "5- to 6-membered heteroaryl" refers to a monocyclic group consisting of 5 to 6 ring atoms with a conjugated π-electron system, in which 1, 2, 3, or 4 ring atoms are heteroatoms independently selected from O, S, and N, and the rest are carbon atoms, wherein the nitrogen atoms are optionally quaternized, and the nitrogen and sulfur heteroatoms can be optionally oxidized (i.e., NO and S(O)ₚ, p is 1 or 2). 5- to 6-membered heteroaryl can be connected to the rest of the molecule through a heteroatom or a carbon atom. The 5- to 6-membered heteroaryl includes 5-membered and 6-membered heteroaryl. Examples of the 5- to 6-membered heteroaryl include, but are not limited to, pyrrolyl (including *N*-pyrrolyl, 2-pyrrolyl, and 3-pyrrolyl, etc.), pyrazolyl (including 2-pyrazolyl and 3-pyrazolyl, etc.), imidazolyl (including *N*-imidazolyl, 2-imidazolyl, 4-imidazolyl, and 5-imidazolyl, etc.), oxazolyl (including 2-oxazolyl, 4-oxazolyl, and 5-oxazolyl, etc.), triazolyl (including 1*H*-1,2,3-triazolyl, 2*H*-1,2,3-triazolyl, 1*H*-1,2,4-triazolyl, and 4*H-*1,2,4-triazolyl, etc.), tetrazolyl, isoxazolyl (including 3-isoxazolyl, 4-isoxazolyl, and 5-isoxazolyl, etc.), thiazolyl (including 2-thiazolyl, 4-thiazolyl, and 5-thiazolyl, etc.), furyl (including 2-furyl and 3-furyl, etc.), thienyl (including 2-thienyll and 3-thienyl, etc.), pyridyl (including 2-pyridyl, 3-pyridyl, and 4-pyridyl, etc.), pyrazinyl, pyrimidinyl (including 2-pyrimidinyl, 4-pyrimidinyl, etc.).

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁, and C₁₂, and any range from n to n+m is also included, for example C₁₋₁₂ includes C₁₋₃, C₁₋₆, C₁₋₉, C₃₋₆, C₃₋₉, C₃₋₁₂, C₆₋₉, C₆₋₁₂, C₉₋₁₂ and the like; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, and 6- to 10-membered ring, etc.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure.

The solvent used in the present disclosure is commercially available. The present disclosure uses the following abbreviations: aq stands for water; eq stands for equivalent; DCM stands for dichloromethane; PE stands for petroleum ether; DMF stands for *N,N-*dimethylformamide; DMSO stands for dimethyl sulfoxide; EtOAc stands for ethyl acetate; EtOH stands for ethanol; MeOH stands for methanol; CBz stands for benzyloxycarbonyl, which is an amine protecting group; BOC stands for tert-butoxycarbonyl, which is an amine protecting group; r.t. stands for room temperature; O/N stands for overnight; THF stands for tetrahydrofuran; Boc₂O stands for di-tert-butyl dicarbonate; TFA stands for trifluoroacetic acid; DIPEA stands for diisopropylethylamine; Xantphos stands for 4,5-bis(diphenylphosphino)-9,9-dimethoxyxanthene; NBS stands for N-bromosuccinimide; BINAP stands for 2,2-bis(diphenylphosphino)-1,1-binaphthyl; TEA stands for triethylamine; Pd₂(dba)₃ stands for tris(dibenzylideneacetone)dipalladium.

The compounds of the present disclosure are named according to the conventional naming principles in the art or by ChemDraw^{®} software, and the commercially available compounds use the supplier catalog names.

### Technical effect

The compounds of the present disclosure exhibit significant THRβ activity and selectivity, with no drug-drug interactions; the compounds of the present disclosure have high exposure and good oral bioavailability, exhibiting excellent pharmacokinetic properties; the compounds of the present disclosure can significantly reduce the level of LDL-C in rat plasma.

### BRIEF DESCRIPTION OF THE DRAWINGS

Figure 1: Predicted binding mode of compound A with MGL-3196;
Figure 2: Predicted binding mode of compound B with MGL-3196;
Figure 3: Predicted binding mode of compound C with MGL-3196;
Figure 4: Change rates of LDL-C in rats after one week of administration compared with that before administration.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for one skilled in the art, it is obvious to make various modifications and improvements to the examples of the present disclosure without departing from the scope of the invention which is defined in the appended claims.

### Calculation example 1

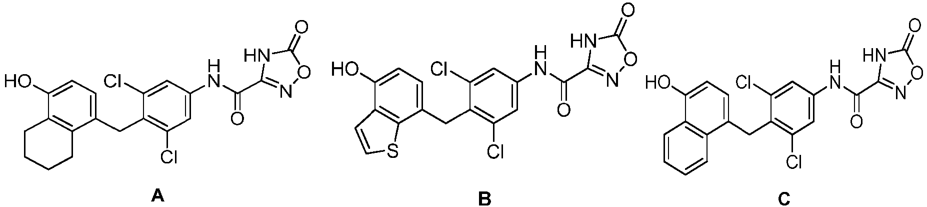

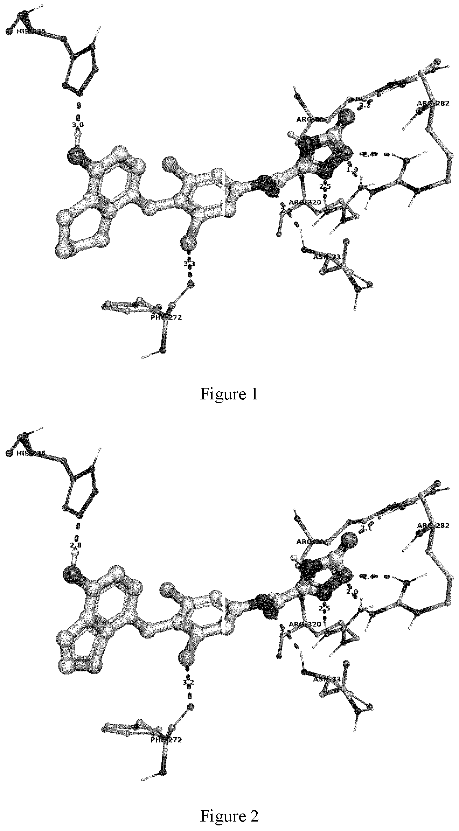

Molecular docking process: carried out using GlideSP[1] in Maestro (Schrödinger version 2017-2) with default settings. MGL-3196 was chosen as the docking template. To prepare protein, hydrogen atoms were added using the Protein Preparation Wizard module of Maestro[2], and the OPLS3 force field was utilized. For preparing the ligand, a 3D structure was generated, and energy minimization was performed using LigPrep[3]. A 30Å docking grid was generated using the ligand centroid in the 1Q4X crystal structure. Through Induced Fit Docking, amino acid side chains within a 5Å range of the ligand center were constrained to move within the B-factor range, generating a complex model. Subsequently, the ligand was removed, and the example compounds were placed during the molecular docking process. The types of interactions between the protein receptor and the ligand were analyzed, and then reasonable docking conformations were selected and saved based on the calculated docking score and global Strain values. The binding modes of compounds A to C with MGL-3196 are as shown in Figures 1 to 3.
[1] Glide, Schrödinger, LLC, New York, NY, 2017.
[2] Maestro, Schrödinger, LLC, New York, NY, 2017.
[3] LigPrep, Schrödinger, LLC, New York, NY, 2017.

**Conclusion:** The compounds of the present disclosure have good binding with THRβ protein. The compounds of the present disclosure occupy a binding pocket of THRβ with thyroxine, and the binding pocket is a closed and hydrophobic pocket composed of multilayered α-helices. Above the pocket, there is a positively charged sub-pocket composed of three arginines (Arg282, Arg316, and Arg320). The 6-azauracil acidic group of the original reference compound MGL-3196 is bound in this sub-pocket. The cyano group forms a hydrogen bond with Arg316, the carbonyl and nitrogen atom of 6-azauracil form a hydrogen bond with Arg320, and the carbonyl oxygen of pyridazinone forms a hydrogen bond with His435. The intermediate benzene ring, the terminal pyridazinone, and isopropyl can all form hydrophobic interactions with surrounding amino acids. The polar head of the compound of the present disclosure, 1,2,4-oxadiazolin-5-one, is bound in this sub-pocket, similarly forming hydrogen bonds with the three arginines. By adjusting the angle of the dichlorobenzene through the amide, it is easier to form a halogen bond with Phe272. The carbonyl or hydroxyl at the tail can form hydrogen bonds through interactions with His435, and the fused ring effectively provides hydrophobic interactions. The compound exhibits good selectivity.

### Example 1

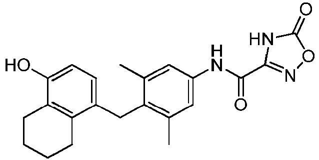

### Synthetic route:

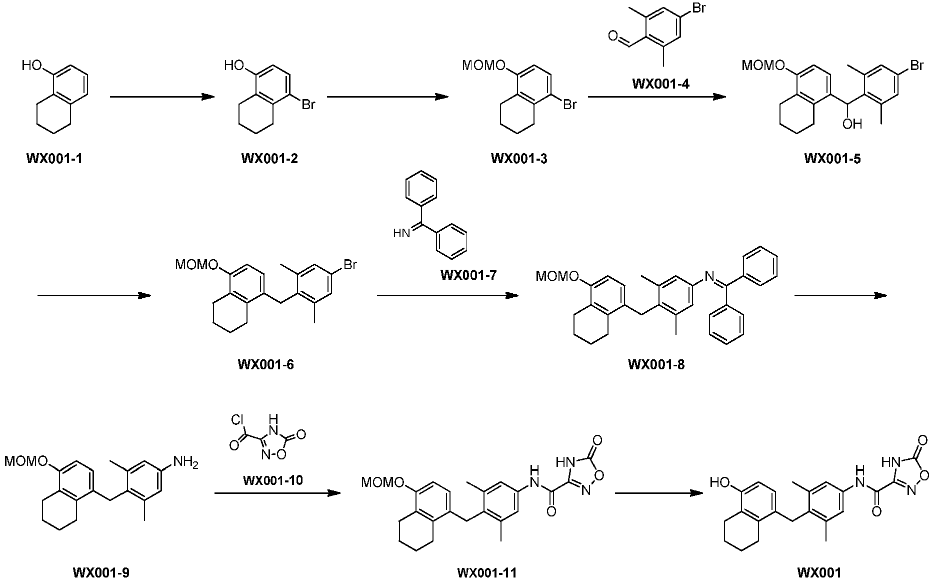

### Step 1: Synthesis of compound WX001-2

To a pre-dried reaction flask were added **WX001-1** (5 g, 33.74 mmol) and acetonitrile (50 mL), then added NBS (6.61 g, 37.11 mmol). The system was replaced with nitrogen three times, and stirred at 25°C for 16 hours. After the reaction was completed, the reaction mixture was directly concentrated under reduced pressure. The concentrate was slurried with methyl tert-butyl ether (200 mL) at 25°C for 30 minutes, and filtered. The filtrate was collected, washed with saturated brine (200 mL * 2). The organic phase was collected, dried over anhydrous sodium sulfate, and filtered. The filtrate was concentrated under reduced pressure to obtain **WX001-2.** ¹H NMR (400MHz, chloroform-d) δ 7.29-7.30 (d, J=2.4 Hz, 1H), 6.55-6.57 (d, J=8.4 Hz,1H), 4.91 (s, 1H), 2.73-2.74 (m,2H), 2.66-2.68 (m,2H), 1.81-1.84 (m, 4H).

### Step 2: Synthesis of compound WX001-3

To a pre-dried reaction flask were added **WX001-2** (6.2 g, 27.30 mmol) and DMF (140 mL). Cesium carbonate (22.24 g, 68.25 mmol) was added thereto in batches at 0°C. The system was replaced with nitrogen three times, and chloromethyl methyl ether (3.30 g, 40.95 mmol, 3.11 mL) was slowly added dropwise thereto at 0°C. The mixture was stirred at 0°C for 2 hours, additionally added with chloromethyl methyl ether (1.76 g, 21.84 mmol, 1.66 mL, 0.8 eq), and stirred at 0°C for another 1 hour. After the reaction was completed, the reaction mixture was quenched by pouring it into water (100 mL). The aqueous phase was extracted with methyl tert-butyl ether (100 mL * 3). The organic phases were combined, washed with saturated brine (150 mL * 3). The resulting organic phase was collected, dried over anhydrous sodium sulfate, filtered, and concentrated under reduced pressure to obtain a crude product. The crude product was purified by column chromatography (petroleum ether: ethyl acetate = 1:0 to 20:1) to obtain **WX001-3.** ¹H NMR (400MHz, chloroform-d) δ 7.31-7.33 (d, J=8.4 Hz, 1H), 6.78-6.81 (d, J= 8.4 Hz, 1H), 5.18 (s, 2H), 3.48 (s, 3H), 2.68-2.74 (m, 4H), 1.76-1.79 (m, 4H).

### Step 3: Synthesis of compound WX001-5

To a pre-dried reaction flask were added **WX001-3** (6 g, 22.13 mmol) and THF (60 mL). The mixture was cooled to -78°C, added with n-butyllithium (2.5 M, 9.29 mL), maintained at -78°C, and stirred for 1 hour. A mixture solution of THF (6 mL) and **WX001-4** (4.71 g, 22.13 mmol) was added thereto, and the resulting mixture was stirred at -78°C for another 1 hour. After the reaction was completed, the reaction mixture was poured into 20 mL of saturated aqueous solution of ammonium chloride, and the mixture was extracted with ethyl acetate (20 mL * 3). The organic phases were combined, washed with 20 mL of saturated brine. After separating the phases, the organic phase was dried over anhydrous sodium sulfate, and filtered. The filtrate was dried under reduced pressure by rotary evaporation to obtain a crude product. The crude product was purified by column chromatography (ethyl acetate: petroleum ether = 2% to 5%) to obtain **WX001-5.** ¹H NMR (400MHz, chloroform-d) δ 7.10 (s, 2H), 6.77-6.79 (d, J=8.4 Hz, 1H), 6.67-6.69 (d, J=8.8 Hz, 1H), 6.13-6.14 (d, J=4.8 Hz, 1H), 5.07-5.11 (m, 2H), 3.46 (s, 3H), 2.92-2.97 (m, 1H), 2.71-2.74 (m, 2H), 2.57-2.62 (m, 1H),2.26 (s, 6H), 1.88-1.89 (d, J=4.4 Hz, 1H), 1.76-1.83 (m, 4H).

### Step 4: Synthesis of compound WX001-6

To a pre-dried reaction flask were added **WX001-5** (8.4g, 20.72 mmol) and DCM (100 mL). The mixture was cooled to 0°C, then added with triethylsilane (3.61 g, 31.09 mmol, 4.97 mL) and TFA (3.54 g, 31.09 mmol, 2.30 mL). The reaction system was stirred at 0°C for 1 hour. After the reaction was completed, the reaction mixture was poured into 50 mL of saturated aqueous solution of sodium carbonate. The mixture was extracted with DCM (50 mL * 3). The organic phases were combined, and washed with saturated brine (100 mL). After separating the phases, the organic phase was dried over anhydrous sodium sulfate, and filtered. The filtrate was dried under reduced pressure by rotary evaporation to obtain a crude product. The crude product was purified by column chromatography (ethyl acetate: petroleum ether = 2% to 5% to 10%) to obtain **WX001-6.** ¹H NMR (400MHz, chloroform-d) δ 7.22 (s, 2H), 6.66-6.68 (d,J=8.4 Hz, 1H), 6.20-6.22 (d, J=8.4 Hz, 1H), 5.13 (s, 2H), 3.73 (s, 2H), 3.46 (s, 3H), 2.73-2.78 (m, 4H), 2.15 (s, 6H), 1.80-1.90 (m, 4H).

### Step 5: Synthesis of compound WX001-8

To a solution of **WX001-7** (2.05 g, 11.30 mmol, 1.90 mL) in THF (40 mL) were added **WX001-6** (4 g, 10.27mmol), cesium carbonate (5.02 g, 15.41 mmol), BINAP (319.87 mg, 513.71 µmol), and palladium (II) acetate (115.33 mg, 513.71 µmol). The mixture was reacted at 65°C for 12 hours. After the reaction was completed, the reaction mixture was added with water (50 mL) and extracted with ethyl acetate (50 mL * 3). The organic phases were combined, and washed with saturated brine (50 mL). After separating the phases, the organic phase was dried over anhydrous sodium sulfate, and filtered. The filtrate was dried under reduced pressure by rotary evaporation to obtain **WX001-8.**

### Step 6: Synthesis of compound WX001-9

To a pre-dried reaction flask were added **WX001-8** (4.5 g, 9.19 mmol), HCl (8 mL), and THF (40 mL). The mixture was stirred at 25°C for 1 hour. After the reaction was completed, the reaction mixture was added with 50 mL of saturated aqueous solution of sodium carbonate, and extracted with ethyl acetate (50 mL * 3). The organic phases were combined, and washed with saturated brine (50 mL). After separating the phases, the organic phase was dried over anhydrous sodium sulfate, and filtered. The filtrate was dried under reduced pressure by rotary evaporation. The crude product was purified by column chromatography (ethyl acetate: petroleum ether = 5% to 10% to 20%) to obtain **WX001-9.** ¹H NMR (400MHz, chloroform-d) δ 6.66-6.68(d, J=8.4 Hz, 1H), 6.46 (s, 2H), 6.31-6.34 (d, J=8.4 Hz,1H), 5.14 (s, 2H), 3.69 (s, 2H), 3.46 (s, 3H), 2.73-2.80 (m, 4H), 2.09 (s, 6H),1.80-1.87 (m, 4H).

### Step 7: Synthesis of compound WX001-11

To a dry reaction flask were sequentially added **WX001-9** (450 mg, 1.38 mmol, 1 *eq),* THF (4 mL), and a solution of TEA (419.75 mg, 4.15 mmol, 577.37 µL, 3 *eq)* and **WX001-10** (308.01 mg, 2.07 mmol, 1.5 *eq)* in THF (3 mL). The system was replaced with nitrogen, and the mixture was stirred at 20°C for 1 hour. After the reaction was completed, the mixture was diluted with water (10 mL), and extracted with ethyl acetate (10 mL * 3). After separating the phases, the organic phase was collected, sequentially washed with saturated brine (10 mL * 3), dried over anhydrous sodium sulfate, and concentrated under reduced pressure to obtain a crude product. The crude product was purified by a thin-layer chromatography silica gel plate (DCM: MeOH = 20:1). Thus **WX001-11** was obtained. ¹H NMR (400 MHz, chloroform-d) δ 8.51 (br s, 1H), 7.31 (s, 2H), 6.65 (d, *J* = 8.4 Hz, 1H), 6.24 (d, *J =* 8.4 Hz, 1H), 5.17 - 5.08 (m, 2H), 3.78 - 3.73 (m, 2H), 3.48 - 3.43 (m, 3H), 2.79 - 2.74 (m, 4H), 2.19 - 2.14 (m, 6H), 1.88 (br d, *J=* 4.5 Hz, 2H), 1.84 - 1.79 (m, 2H).

### Step 8: Synthesis of compound WX001

To a dry reaction flask were sequentially added **WX001-11** (400 mg, 914.31 µmol, 1 *eq),* MeOH (4 mL), and HCl (0.8 mL, purity of 37%). The mixture was heated to 50°C and stirred for 2 hours. After the reaction was completed, the mixture was diluted with water (10 mL), and extracted with ethyl acetate (10 mL*3). After separating the phases, the organic phase was collected. The organic phase was sequentially washed with a saturated aqueous solution of sodium bicarbonate (20 mL) and saturated brine (10 mL * 3), dried over anhydrous sodium sulfate, and concentrated under reduced pressure to obtain a crude product. The crude product was purified by preparative HPLC (chromatographic column: Waters Xbridge BEH C18 100 * 30 mm * 10 µm; mobile phase: [water (10 mM ammonium bicarbonate) - acetonitrile]; acetonitrile%: 25% to 55%, 10 minutes) to obtain **WX001.** ¹H NMR (400 MHz, deuterated methanol) δ 7.41 (s, 2H), 6.36 (d, *J=* 8.3 Hz, 1H), 6.05 (d, *J* = 8.3 Hz, 1H), 3.77 - 3.73 (m, 2H), 2.80 - 2.74 (m, 2H), 2.69 - 2.64 (m, 2H), 2.15 (s, 6H), 1.88 - 1.88 (m, 1H), 1.90 - 1.85 (m, 2H), 1.82 - 1.78 (m, 2H). MS-ESI *m*/*z:* 392.5 [M-H]⁻.

### Example 2

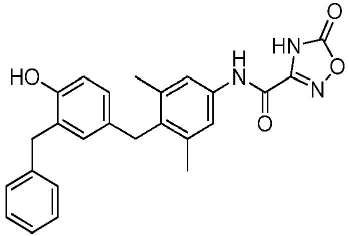

### Synthetic route:

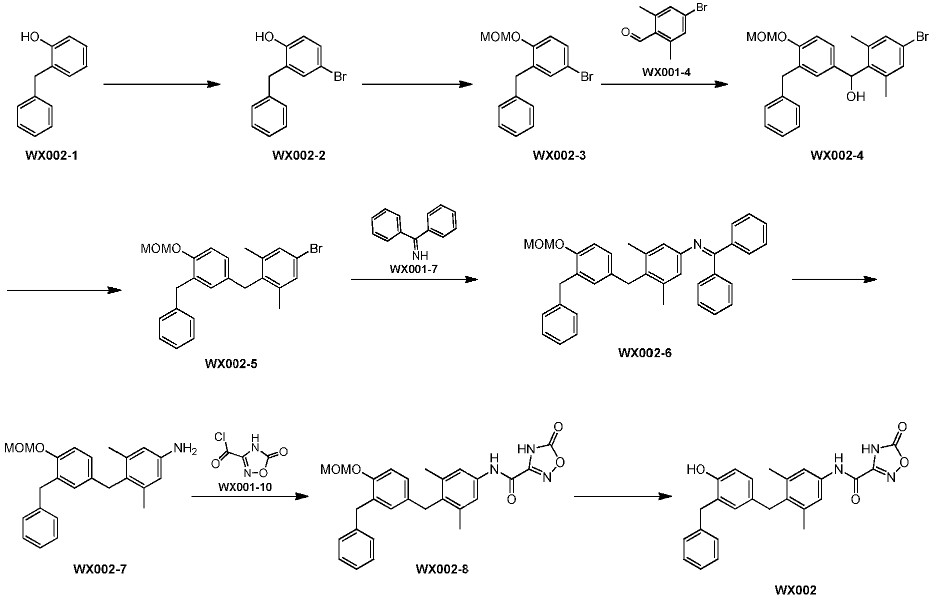

### Step 1: Synthesis of compound WX002-2

To a pre-dried reaction flask were added **WX002-1** (1 g, 5.43 mmol, 1 eq), chloroform (67 mL), and tetrabutylammonium tribromide (3.14 g, 6.51 mmol, 1.2 eq). The reaction system was replaced with nitrogen three times, and stirred at 25°C for 3 hours. After the reaction was completed, the reaction mixture was directly concentrated under reduced pressure. The resulting concentrate was diluted with ethyl acetate (30 mL), and added with water (30 mL). The phases were separated. The aqueous phase was extracted with ethyl acetate (30 mL * 2). The organic phases were combined, and sequentially washed with 1 N hydrochloric acid (30 mL) and saturated brine (30 mL). The resulting organic phase was collected, dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1) to obtain **WX002-2.** ¹H NMR (400 MHz, chloroform-d) δ 7.35 - 7.29 (m, 2H), 7.27 - 7.20 (m, 5H), 6.71 - 6.66 (m, 1H), 4.74 (s, 1H), 3.96 (s, 2H).

### Step 2: Synthesis of compound WX002-3

To a pre-dried reaction flask were added **WX002-2** (2.46 g, 9.35 mmol, 66.63 mL, 1 eq) and DMF (24.6 mL). The reaction system was replaced with nitrogen three times, placed at 0°C, and added with cesium carbonate (13.07 g, 40.11 mmol, 4.29 eq). Chloromethyl methyl ether (2.15 g, 26.70 mmol, 2.03 mL, 2.86 eq) was added dropwise thereto, and the mixture was stirred at 25°C for 2 hours. After the reaction was completed, the reaction mixture was quenched by pouring it into ice water (30 mL), and added with ethyl acetate (20 mL) to separate the phases. The organic phase was collected, and the aqueous phase was extracted with ethyl acetate (2 * 20 mL). The resulting organic phases were combined, washed with saturated brine (20 mL * 3), dried over anhydrous sodium sulfate, and filtered. The filtrate was concentrated under reduced pressure to obtain a crude product. The crude product was purified by column chromatography (petroleum ether: ethyl acetate = 1:0 to 10:1 to 5:1) to obtain **WX002-3.** ¹H NMR (400 MHz, chloroform-d) δ 7.32 - 7.27 (m, 3H), 7.25 - 7.17 (m, 4H), 6.97 (d, J = 8.7 Hz, 1H), 5.15 (s, 2H), 3.96 (s, 2H), 3.37 (s, 3H).

### Step 3: Synthesis of compound WX002-4

To a pre-dried reaction flask were added **WX002-3** (2.34 g, 7.62 mmol, 1 eq) and THF (23.4 mL). The reaction system was replaced with nitrogen three times and placed at -78°C. n-Butyllithium (2.5 M, 3.35 mL, 1.1 eq) was slowly added dropwise thereto. The mixture was stirred at -78°C for 1 hour, then a mixture solution of **WX001-4** (1.46 g, 6.86 mmol, 0.9 eq) and THF (23.4 mL) was dropwise added thereto at -78°C. The mixture was stirred at - 78°C for another 1 hour. After the reaction was completed, the reaction mixture was poured into a saturated solution of ammonium chloride (20 mL), and added with ethyl acetate (20 mL) to separate the phases. The organic phase was collected, and the aqueous phase was extracted with ethyl acetate (20 mL * 2). The resulting organic phases were combined, sequentially washed with saturated brine (20 mL * 2), dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1 to 5:1) to obtain **WX002-4.** ¹H NMR (400 MHz, chloroform-d) δ 7.18 - 7.14 (m, 2H), 7.12 - 7.05 (m, 5H), 6.99 (s, 1H), 6.93 - 6.86 (m, 2H), 6.14 (d, J = 4.1 Hz, 1H), 5.23 (s, 1H), 5.05 (s, 2H), 3.89 (d, J = 5.6 Hz, 2H), 3.26 (s, 3H), 2.15 (s, 6H).

### Step 4: Synthesis of compound WX002-5

To a pre-dried reaction flask were added **WX002-4** (2.23 g, 5.05 mmol, 1 eq) and DCM (23 mL). The reaction system was replaced with nitrogen three times and placed at 0°C. Et₃SiH (881.28 mg, 7.58 mmol, 1.21 mL, 1.5 eq) and trifluoroacetic acid (864.15 mg, 7.58 mmol, 561.13 µL, 1.5 eq) were sequentially and slowly added thereto in drops, and the mixture was stirred at 0°C for another 1 hour. After the reaction was completed, the reaction mixture was poured into 50 mL of saturated aqueous solution of sodium carbonate, and extracted with DCM (50 mL * 3). The organic phases were combined, and washed with saturated brine (50 mL). After separating the phases, the organic phase was dried over anhydrous sodium sulfate, and filtered. The filtrate was dried under reduced pressure by rotary evaporation. The crude product was purified by column chromatography (petroleum ether: ethyl acetate = 1:0 to 20:1 to 10:1) to obtain **WX002-5.** ¹H NMR (400 MHz, chloroform-d) δ 7.24 (d, J = 7.4 Hz, 2H), 7.21 - 7.12 (m, 5H), 6.93 (d, J = 8.4 Hz, 1H), 6.78 (d, J = 1.9 Hz, 1H), 6.72 (dd, J = 2.0, 8.4 Hz, 1H), 5.10 (s, 2H), 3.93 (s, 2H), 3.89 (s, 2H), 3.34 (s, 3H), 2.19 (s, 6H).

### Step 5: Synthesis of compound WX002-6

To a pre-dried reaction flask were added **WX002-5** (1.8 g, 4.23 mmol, 1 eq), **WX001-**7 (843.62 mg, 4.65 mmol, 781.13 µL, 1.1 eq), and dioxane (36 mL). Then cesium carbonate (2.07 g, 6.35 mmol, 1.5 eq) was added thereto. The reaction system was replaced with nitrogen three times, and sequentially added with Xantphos (195.89 mg, 338.54 µmol, 0.08 eq) and Pd₂(dba)₃ (232.51 mg, 253.90 µmol, 0.06 eq), again replaced with nitrogen three times, and stirred at 100°C for 16 hours. After the reaction was completed, the reaction mixture was added with water (200 mL) and ethyl acetate (200 mL). The phases were separated. The aqueous phase was extracted with ethyl acetate (200 mL * 3). The organic phases were combined, and washed with saturated brine (300 mL * 2). The resulting organic phase was collected, dried over anhydrous sodium sulfate, and filtered. The filtrate was concentrated under reduced pressure to obtain **WX002-6,** which was directly used in the next step without further purification.

### Step 6: Synthesis of compound WX002-7

To a pre-dried reaction flask were added **WX002-6** (2.22 g, 4.22 mmol, 1 eq) and THF (25 mL). Then 2 N hydrochloric acid (5.78 mL, 2.74 eq) was added thereto. The reaction system was replaced with nitrogen three times, and stirred at 25°C for 1 hour. After the reaction was completed, the reaction mixture was quenched with a saturated solution of sodium bicarbonate (100 mL). The aqueous phase was extracted with ethyl acetate (100 mL * 3). The organic phases were combined, and washed with saturated brine (150 mL * 2). The resulting organic phase was collected, dried over anhydrous sodium sulfate, and filtered. The filtrate was concentrated under reduced pressure to obtain a crude product. The crude product was purified by column chromatography (petroleum ether: ethyl acetate = 1:0 to 10:1 to 5:1 to 2:1) to obtain **WX002-7.** ¹H NMR (400 MHz, chloroform-d) δ 7.24 (d, J = 7.2 Hz, 2H), 7.17 (d, J = 7.5 Hz, 3H), 6.91 (d, J = 8.3 Hz, 1H), 6.86 (d, J = 1.8 Hz, 1H), 6.76 (dd, J = 1.8, 8.3 Hz, 1H), 6.43 (s, 2H), 5.09 (s, 2H), 3.94 (s, 2H), 3.85 (s, 2H), 3.50 (br s, 2H), 3.33 (s, 3H), 2.14 (s, 6H).

### Step 7: Synthesis of compound WX002-8

To a pre-dried reaction flask were added **WX001-10** (474.51 mg, 3.20 mmol, 1.50 eq) and THF (8 mL). The reaction system was replaced with nitrogen three times, and a mixture solution of **WX002-7** (770 mg, 2.13 mmol, 1 eq) and THF (8 mL) was dropwise added thereto. The mixture was added with TEA (646.65 mg, 6.39 mmol, 889.48 µL, 3 eq), and stirred at 25°C for 1 hour. After the reaction was completed, the reaction mixture was quenched by pouring it into water (30 mL). The aqueous phase was extracted with ethyl acetate (30 mL * 3). The organic phases were combined, and washed with saturated brine (50 mL * 2). The resulting organic phase was collected, dried over anhydrous sodium sulfate, and filtered. The filtrate was concentrated under reduced pressure to obtain **WX002-8,** which was directly used in the next step without further purification.

### Step 8: Synthesis of compound WX002

To a pre-dried reaction flask were added **WX002-8** (1.09 g, 2.30 mmol, 1 eq), MeOH (22 mL), and HCl (12 M, 4.36 mL, 22.73 eq). The reaction system was replaced with nitrogen three times, and stirred at 50°C for 3 hours. After the reaction was completed, the reaction mixture was added with a saturated sodium bicarbonate solution to adjust the pH to around 7, and extracted with ethyl acetate (30 mL * 3). After separating the phases, the organic phases were collected, combined, washed with saturated brine (40 mL * 3), dried over anhydrous sodium sulfate, and filtered. The filtrate was concentrated under reduced pressure to obtain a crude product. The crude product was purified by HPLC (chromatographic column: Waters Xbridge BEH C18 250 * 50 mm * 10 µm; mobile phase: [water (ammonium bicarbonate) - acetonitrile]; acetonitrile%: 25% to 65%, 10 minutes) to obtain **WX002.** ¹H NMR (400 MHz, deuterated methanol) δ 7.36 (s, 2H), 7.25 - 7.08 (m, 5H), 6.67 - 6.59 (m, 3H), 3.88 (s, 2H), 3.84 (s, 2H), 2.18 (s, 6H); MS-ESI *m*/*z:* 428.2 [M-H]⁻.

### Example 3

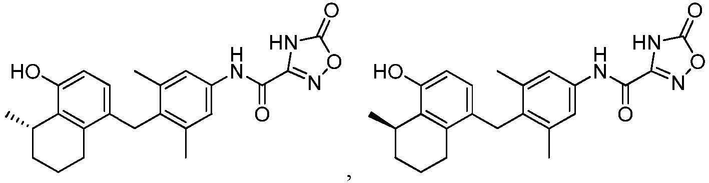

### Synthetic route:

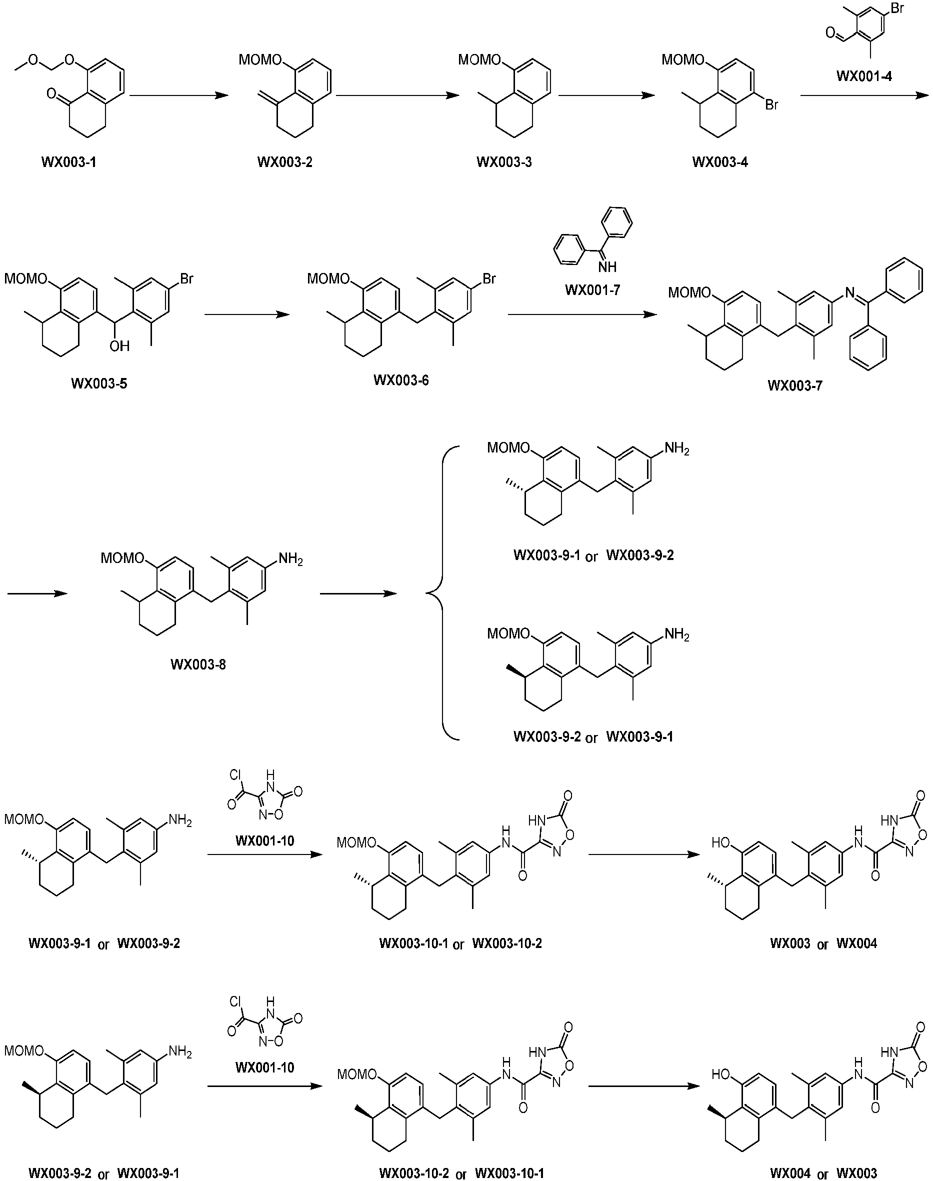

### Step 1: Synthesis of compound WX003-2

To a pre-dried reaction flask were added sodium hydride (5.82 g, 145.46 mmol, content of 60%, 3 eq) and DMSO (35 mL). The reaction system was replaced with nitrogen three times, and stirred at 75°C for 10 minutes. Then the reaction system was maintained at 25°C, slowly added with a solution of methyltriphenylphosphonium iodide (39.20 g, 96.98 mmol, 2 eq) in DMSO (25 mL), and stirred at 25°C for 20 minutes. A solution of **WX003-1** (10 g, 48.49 mmol, 1 eq) in DMSO (10 mL) was dropwise added thereto at 25°C, and the mixture was heated to 65°C and stirred for another 12 hours. After the reaction was completed, the reaction mixture was poured into ice water (200 mL), and added with methyl tert-butyl ether (200 mL) to separate the phases. The organic phase was collected. The aqueous phase was extracted with methyl tert-butyl ether (200 mL * 2). The resulting organic phases were combined, washed with saturated brine (600 mL * 3), dried over anhydrous sodium sulfate, and filtered. The filtrate was concentrated under reduced pressure to obtain a crude product. The crude product was purified by an automatic column chromatography machine (petroleum ether: ethyl acetate = 1:0 to 5:1) to obtain **WX003-2.** ¹H NMR (400MHz, chloroform-d) δ 7.14 - 7.06 (m, 1H), 6.99 (d, J = 8.1 Hz, 1H), 6.81 (d, J = 7.5 Hz, 1H), 5.94 (d, J = 2.4 Hz, 1H), 5.33 - 5.27 (m, 1H), 5.23 (s, 2H), 3.52 (s, 2H), 3.56 - 3.48 (m, 1H), 2.90 - 2.83 (m, 1H), 2.87 (t, J = 6.4 Hz, 1H), 2.56 - 2.50 (m, 1H), 2.57 - 2.48 (m, 1H), 1.89 (quin, J = 6.4 Hz, 1H), 1.96 - 1.84 (m, 1H).

### Step 2: Synthesis of compound WX003-3

To a pre-dried hydrogenation flask were sequentially added ethyl acetate (300 mL) and Pd/C (2.32 g, 20.56 mmol, content of 10%, 1 eq) under nitrogen atmosphere, then added **WX003-2** (4.2 g, 20.56 mmol, 1 eq). The reaction system was replaced with hydrogen three times, maintained under hydrogen atmosphere at 15 psi, and stirred at 25°C for 2 hours. After the reaction was completed, the reaction mixture was directly filtered through diatomite. The filtrate was concentrated under reduced pressure to obtain **WX003-3.** ¹H NMR (400MHz, chloroform-d) δ 7.09 - 7.02 (m, 1H), 6.90 (d, J = 8.1 Hz, 1H), 6.76 (d, J = 7.6 Hz, 1H), 5.23 (s, 2H), 3.51 (s, 2H), 3.57 (s, 1H), 3.30 - 3.16 (m, 1H), 2.88 - 2.67 (m, 2H), 1.93 - 1.70 (m, 3H), 1.96 - 1.64 (m, 1H), 1.25 (d, J = 7.0 Hz, 3H).

### Step 3: Synthesis of compound WX003-4

To a pre-dried reaction flask were added **WX003-3** (4 g, 19.39 mmol, 1 eq) and THF (40 mL), then added NBS (3.45 g, 19.39 mmol, 1 eq). The reaction system was replaced with nitrogen three times, and stirred at - 78°C for 0.5 hours. After the reaction was completed, the reaction mixture was added with water (100 mL) and ethyl acetate (100 mL) to separate the phases. The organic phase was collected, and the aqueous phase was extracted with ethyl acetate (100 mL * 2). The resulting organic phases were combined, washed with saturated brine (400 mL * 3), dried over anhydrous sodium sulfate, and filtered. The filtrate was concentrated under reduced pressure to obtain a crude product. The crude product was purified by an automatic column chromatography machine (petroleum ether: ethyl acetate = 1:0 to 10:1) to obtain **WX003-4.** ¹H NMR (400MHz, chloroform-d) δ 7.36 - 7.30 (m, 1H), 6.83 (d, J = 8.6 Hz, 1H), 5.20 (d, J = 0.9 Hz, 2H), 3.55 - 3.46 (m, 1H), 3.49 (s, 2H), 3.29 - 3.18 (m, 1H), 2.95 - 2.83 (m, 1H), 2.62 - 2.48 (m, 1H), 1.92 - 1.79 (m, 2H), 1.76 - 1.67 (m, 2H), 1.23 (d, J = 7.0 Hz, 3H).

### Step 4: Synthesis of compound WX003-5

To a pre-dried reaction flask were added **WX003-4** (4.9 g, 17.18 mmol, 1 eq) and THF (50 mL), then slowly added dropwise n-butyllithium (2.5 M, 7.56 mL, 1.1 eq) at -78°C. The mixture was stirred for 1 hour, and a solution of **WX001-4** (3.29 g, 15.46 mmol, 0.9 eq) in THF (25 mL) was slowly added dropwise thereto. The mixture was stirred at -78°C for 1 hour. After the reaction was completed, the reaction mixture was poured into a saturated solution of ammonium chloride (200 mL), and added with ethyl acetate (200 mL) to separate the phases. The organic phase was collected, and the aqueous phase was extracted with ethyl acetate (200 mL * 2). The resulting organic phases were combined, sequentially washed with saturated brine (600 mL * 2), dried over anhydrous sodium sulfate, and filtered. The filtrate was concentrated under reduced pressure to obtain a crude product. The crude product was purified by an automatic column chromatography machine (petroleum ether: ethyl acetate = 1:0 to 10:1 to 5:1) to obtain **WX003-5.** ¹H NMR (400MHz, chloroform-d) δ 7.19 (d, J = 8.5 Hz, 2H), 6.94 (d, J = 8.6 Hz, 1H), 6.86 - 6.74 (m, 1H), 6.21 (dd, J = 4.4, 10.0 Hz, 1H), 5.27 - 5.14 (m, 2H), 3.57 - 3.43 (m, 3H), 3.34 - 3.19 (m, 1H), 3.10 - 3.00 (m, 1H), 2.92 - 2.72 (m, 1H), 2.47 - 2.35 (m, 1H), 2.26 (d, J = 6.9 Hz, 6H), 1.92 - 1.84 (m, 1H), 1.79 - 1.69 (m, 2H), 1.29 - 1.22 (m, 3H).

### Step 5: Synthesis of compound WX003-6

To a pre-dried reaction flask were added **WX003-5** (2.45 g, 5.84 mmol, 1 eq) and DCM (25 mL). The mixture was maintained at -10°C, then added with Et₃SiH (1.02 g, 8.76 mmol, 1.40 mL, 1.5 eq) and TFA (999.25 mg, 8.76 mmol, 648.86 µL, 1.5 eq). The mixture was stirred at -10°C for 1 hour. After the reaction was completed, the reaction mixture was added with a saturated aqueous solution of sodium bicarbonate to adjust the pH to between 6 and 7, and added with water (30 mL) and dichloromethane (30 mL) to separate the phases. The organic phase was collected, and the aqueous phase was extracted with dichloromethane (30 mL * 2). The resulting organic phases were combined, washed with saturated brine (150 mL * 3), dried over anhydrous sodium sulfate, and filtered. The filtrate was concentrated under reduced pressure to obtain a crude product. The crude product was purified by an automatic column chromatography machine (petroleum ether: ethyl acetate = 1:0 to 10:1 to 5:1) to obtain **WX003-6.** ¹H NMR (400MHz, chloroform-d) δ 7.23 (s, 1H), 7.25 - 7.20 (m, 1H), 6.69 (d, J = 8.6 Hz, 1H), 6.21 (d, J = 8.4 Hz, 1H), 5.16 (s, 2H), 3.82 - 3.62 (m, 2H), 3.48 (s, 3H), 3.28 (td, J = 3.5, 6.8 Hz, 1H), 2.93 - 2.83 (m, 1H), 2.67 - 2.57 (m, 1H), 2.15 (s, 6H), 1.92 (br dd, J = 4.8, 10.1 Hz, 2H), 1.78 (td, J = 4.5, 9.4 Hz, 2H), 1.27 (d, J = 7.0 Hz, 3H).

### Step 6: Synthesis of compound WX003-7

To a pre-dried reaction flask were added **WX003-6(1.9** g, 4.71 mmol, 1 eq) and dioxane (20 mL), then added cesium carbonate (2.30 g, 7.07 mmol, 1.5 eq) and **WX001-7** (939.07 mg, 5.18 mmol, 869.51 µL, 1.1 eq). The reaction system was replaced with nitrogen three times, added with Xantphos (218.05 mg, 376.84 µmol, 0.08 eq) and Pd₂(dba)₃ (258.81 mg, 282.63 µmol, 0.06 eq), and again replaced with nitrogen three times. The mixture was stirred at 100°C for 12 hours. After the reaction was completed, the reaction mixture was cooled to room temperature, added with water (50 mL) and ethyl acetate (50 mL) to separate the phases. The organic phase was collected, and the aqueous phase was extracted with ethyl acetate (50 mL * 2). The resulting organic phases were combined, washed with saturated brine (300 mL * 3), dried over anhydrous sodium sulfate, and filtered. The filtrate was concentrated under reduced pressure to obtain **WX003-7,** and the crude product was directly used in the next step.

### Step 7: Synthesis of compound WX003-8

To a pre-dried reaction flask were added **WX003-7** (2.3 g, 4.57 mmol, 1 eq) and THF (25 mL), then added hydrochloric acid (2 N, 2.28 mL, 1 eq). The mixture was stirred at 25°C for 1 hour. After the reaction was completed, the reaction mixture was added with a saturated aqueous solution of sodium bicarbonate to adjust the pH to between 6 and 7, and added with water (30 mL) and ethyl acetate (30 mL) to separate the phases. The organic phase was collected, and the aqueous phase was extracted with ethyl acetate (30 mL * 2). The resulting organic phases were combined, washed with saturated brine (120 mL * 3), dried over anhydrous sodium sulfate, and filtered. The filtrate was concentrated under reduced pressure to obtain a crude product. The crude product was purified by an automatic column chromatography machine (petroleum ether: ethyl acetate = 1:0 to 10:1 to 5:1 to 3:1) to obtain **WX003-8.** ¹H NMR (400MHz, chloroform-d) δ 6.68 (d, J = 8.4 Hz, 1H), 6.53 (s, 2H), 6.30 (d, J = 8.5 Hz, 1H), 5.15 (s, 2H), 3.81 - 3.57 (m, 2H), 3.54 - 3.41 (m, 3H), 3.36 - 3.21 (m, 1H), 2.96 - 2.82 (m, 1H), 2.70 - 2.55 (m, 1H), 2.22 - 2.02 (m, 6H), 1.99 - 1.85 (m, 2H), 1.84 - 1.69 (m, 2H), 1.27 (d, J = 7.0 Hz, 3H).

### Step 8: Synthesis of compounds WX003-9-1 and WX003-9-2

**WX003-8** (250 mg, 736.44 µmol, 1 eq) was subjected to chiral resolution to obtain **WX003-9-1** and **WX003-9-2.** The separation method was: (chromatographic column: DAICEL CHIRALCEL OJ (250 mm * 30 mm, 10 µm); A: food-grade supercritical carbon dioxide; B: mobile phase: [0.1% ammonia water + isopropanol]; B%: 35% to 35%, 10 minutes). The retention time for **WX003-9-1** was 3.671 minutes, while the retention time for **WX003-9-2** was 4.112 minutes. Analysis method (instrument: CAS-TJ-ANA-SFC-C (Waters UPCC with PDA); chromatographic column: Chiralpak OJ-3,150 × 4.6 mm I.D., 3 µm; mobile phase: A: food-grade supercritical carbon dioxide; B: isopropanol (0.1% isopropylamine, by volume); gradient: The content of B was increased from 10% to 50% over 3.5 minutes and held for 1 minute, then elevated from 50% to 10% within 0.5 minutes; flow rate: 2.5 mL/min; column temperature: 35°C; detection wavelength: 220 nm; system back pressure: 100 bar). **WX003-9-1:** ¹H NMR (400MHz, chloroform-d) δ 6.68 (d, J = 8.4 Hz, 1H), 6.47 (s, 2H), 6.32 (d, J = 8.5 Hz, 1H), 5.15 (s, 2H), 3.79 - 3.57 (m, 2H), 3.47 (s, 3H), 3.32 - 3.23 (m, 1H), 2.93 - 2.84 (m, 1H), 2.62 (ddd, J = 7.3, 10.6, 17.6 Hz, 1H), 2.09 (s, 6H), 1.95 - 1.86 (m, 2H), 1.80 - 1.74 (m, 2H), 1.30 - 1.25 (d, J = 7.0 Hz, 3H). **WX003-9-2:** ¹H NMR (400MHz, chloroform-d) δ 6.68 (d, J = 8.5 Hz, 1H), 6.47 (s, 2H), 6.32 (d, J = 8.5 Hz, 1H), 5.15 (s, 2H), 3.77 - 3.58 (m, 2H), 3.47 (s, 3H), 3.32 - 3.23 (m, 1H), 2.94 - 2.84 (m, 1H), 2.62 (ddd, J = 7.4, 10.5, 17.5 Hz, 1H), 2.09 (s, 6H), 1.96 - 1.87 (m, 2H), 1.81 - 1.74 (m, 2H), 1.27 (d, J = 7.0 Hz, 3H).

### Step 9: Synthesis of compounds WX003-10-1 and WX003-10-2

To a dry reaction flask was added a solution of **WX001-10** (46.59 mg, 313.72 µmol, 1.5 eq) in THF (1 mL). **WX003-9-1** (71 mg, 209.15 µmol, 1 eq) was dissolved in THF (1 mL) and the solution was added to the reaction flask. TEA (63.49 mg, 627.45 µmol, 87.33 µL, 3 eq) was added thereto. The mixture was stirred at 20°C for 1 hour. After the reaction was completed, the reaction mixture was poured into water (5 mL), and extracted with ethyl acetate (5 mL) to separate the phases. The organic phase was collected, and the aqueous phase was extracted with ethyl acetate (5 mL * 2). The resulting organic phases were combined, sequentially washed with saturated brine (20 mL * 2), dried over anhydrous sodium sulfate, and filtered. The filtrate was concentrated under reduced pressure to obtain **WX003-10-1.**

To a dry reaction flask was added a solution of **WX001-10** (46.59 mg, 313.72 µmol, 1.5 eq) in THF (1 mL). **WX003-9-2** (71 mg, 209.15 µmol, 1 eq) was dissolved in THF (1 mL) and the solution was added to the reaction flask. TEA (63.49 mg, 627.45 µmol, 87.33 µL, 3 eq) was added thereto. The mixture was stirred at 20°C for 1 hour. After the reaction was completed, the reaction mixture was poured into water (5 mL), and extracted with ethyl acetate (5 mL) to separate the phases. The organic phase was collected, and the aqueous phase was extracted with ethyl acetate (5 mL * 2). The resulting organic phases were combined, sequentially washed with saturated brine (20 mL * 2), dried over anhydrous sodium sulfate, and filtered. The filtrate was concentrated under reduced pressure to obtain **WX003-10-2.**

### Step 10: Synthesis of compounds WX003 and WX004

To a pre-dried reaction flask were added **WX003-10-1** (100.00 mg, 221.48 µmol, 1 eq) and DMA (1 mL), then added HCl (0.2 mL, purity of 37%). The reaction system was replaced with nitrogen three times, and stirred at 50°C for 6 hours. After the reaction was completed, the reaction mixture was diluted with water (10 mL), added with a saturated aqueous solution of sodium bicarbonate to adjust the pH to neutrality, and extracted with ethyl acetate (10 mL * 3). After separating the phases, the organic phases were collected, combined, washed with saturated brine (50 mL * 3), dried over anhydrous sodium sulfate, and filtered. The filtrate was concentrated under reduced pressure to obtain a crude product. The crude product was purified by HPLC (chromatographic column: Waters Xbridge BEH C18 100 * 30 mm * 10 µm; mobile phase: [water (ammonium bicarbonate) - acetonitrile]; B%: 25% to 55%, 8 minutes) to obtain **WX003.** ¹H NMR (400MHz, dimethyl sulfoxide-d6) δ 10.34 (br s, 1H), 8.93 (s, 1H), 7.45 (s, 2H), 6.40 (d, J = 8.3 Hz, 1H), 5.96 (d, J = 8.3 Hz, 1H), 3.70 (s, 1H), 3.62 (s, 1H), 3.10 (td, J = 3.5, 6.8 Hz, 1H), 2.88 - 2.79 (m, 1H), 2.58 (br s, 1H), 2.09 (s, 6H), 1.89 - 1.78 (m, 2H), 1.68 (br d, J = 3.1 Hz, 2H), 1.18 (d, J = 6.9 Hz, 3H); MS-ESI *m*/*z:* 406.2 [M-H]⁻.

To a pre-dried reaction flask were added **WX003-10-2** (100.00 mg, 221.48 µmol, 1 eq) and DMA (1 mL), then added HCl (0.2 mL, purity of 37%). The reaction system was replaced with nitrogen three times, and stirred at 50°C for 6 hours. After the reaction was completed, the reaction mixture was diluted with water (10 mL), added with a saturated aqueous solution of sodium bicarbonate to adjust the pH to neutrality, and extracted with ethyl acetate (10 mL * 3). After separating the phases, the organic phases were collected, combined, washed with saturated brine (50 mL * 3), dried over anhydrous sodium sulfate, and filtered. The filtrate was concentrated under reduced pressure to obtain a crude product. The crude product was purified by HPLC (chromatographic column: Waters Xbridge BEH C18 100 * 30 mm * 10 µm; mobile phase: [A: water (ammonium bicarbonate) - B: acetonitrile]; acetonitrile%: 25% to 55%, 8 minutes) to obtain **WX004.** ¹H NMR (400MHz, dimethyl sulfoxide-d6) δ 10.23 (s, 1H), 8.92 (s, 1H), 7.45 (s, 2H), 6.39 (d, J = 8.3 Hz, 1H), 5.95 (d, J = 8.3 Hz, 1H), 3.69 (s, 1H), 3.60 (br s, 1H), 3.08 (td, J = 3.5, 6.8 Hz, 1H), 2.87 - 2.78 (m, 1H), 2.62 - 2.53 (m, 1H), 2.07 (s, 6H), 1.89 - 1.77 (m, 2H), 1.66 (br d, J = 3.3 Hz, 2H), 1.17 (d, J = 6.9 Hz, 3H); MS-ESI *m*/*z:* 406.2 [M-H]⁻.

### Example 4

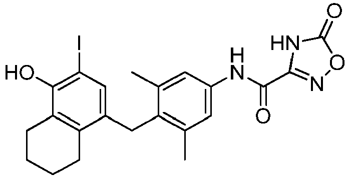

### Synthetic route:

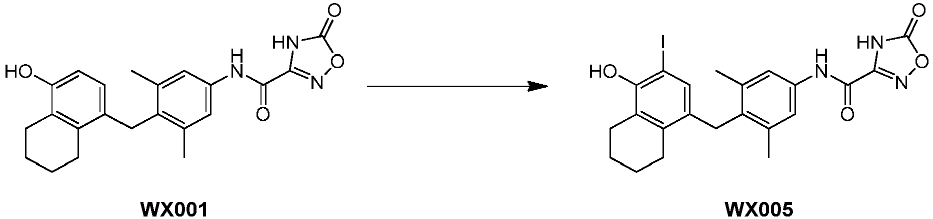

### Step 1: Synthesis of compound WX005

To a pre-dried reaction flask were added **WX001** (50 mg, 127.09 µmol, 1 eq) and THF (2.8 mL), then added TEA (0.28 mL). The reaction system was replaced with nitrogen three times, maintained at -78°C, and added with iodine monochloride (24.76 mg, 152.50 µmol, 7.78 µL, 1.20 eq). The mixture was stirred at -78°C for 2 hours. After the reaction was completed, the reaction mixture was added with 2 N hydrochloric acid to adjust the pH to between 6 and 7, and washed with a saturated sodium thiosulfate solution (10 mL * 2). The organic phase was collected, dried over anhydrous sodium sulfate, and filtered. The filtrate was concentrated under reduced pressure to obtain a crude product. The crude product was purified by HPLC (chromatographic column: Phenomenex luna 80 * 30 mm * 3 µm; mobile phase: [water (HCl) - acetonitrile]; acetonitrile%: 60% to 80%, 8 minutes) to obtain **WX005.** ¹H NMR (400 MHz, methanol-d4) δ 7.44 (s, 2H), 6.51 (s, 1H), 3.76 (s, 2H), 2.81 - 2.66 (m, 4H), 2.17 (s, 6H), 1.91 - 1.77 (m, 4H); MS-ESI *m*/*z*: 518.0 [M-H]⁻.

### Example 5

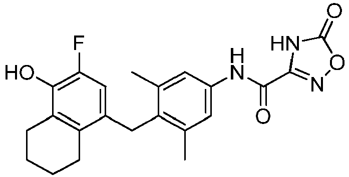

### Synthetic route:

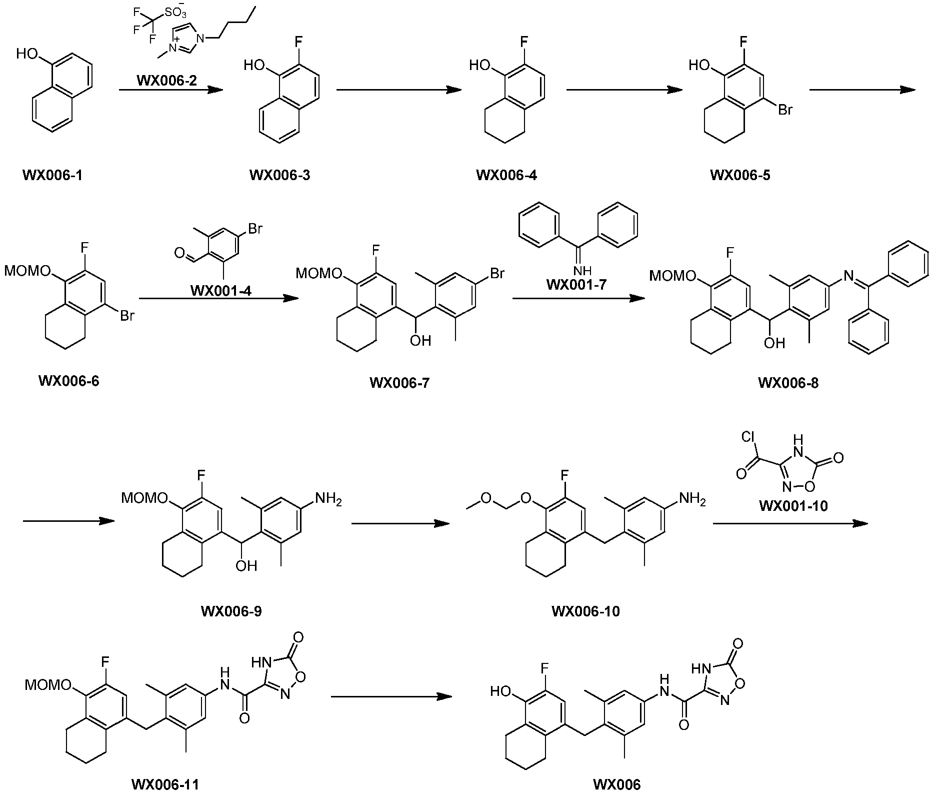

### Step 1: Synthesis of compound WX006-3

To a pre-dried reaction flask were added **WX006-1** (5.5 g, 38.15 mmol, 13.75 mL, 1 *eq)* and isopropanol (250 mL), then added **WX006-2** (12.14 g, 41.96 mmol, 1.1 *eq)* and 1-chloromethyl-4-fluoro-1,4-diazoniabicyclo[2.2.2]octane bis(tetrafluoroborate) (20.27 g, 57.22 mmol, 1.5 *eq).* The reaction system was replaced with nitrogen three times, and stirred at 80°C for 12 hours. After the reaction was completed, the reaction mixture was directly concentrated under reduced pressure to obtain a crude product. The crude product was purified by column chromatography (petroleum ether: ethyl acetate = 1:0 to 50:1) to obtain **WX006-3.** ¹H NMR (400 MHz, chloroform-d) δ 8.21 (d, *J*=8.19 Hz, 1H), 7.80 (d, *J*=8.19 Hz, 1H), 7.46-7.55 (m, 2H), 7.39 (dd, *J*=5.20, 8.99 Hz, 1H), 7.28-7.32 (m, 1H), 5.48 (d, *J=4.40* Hz, 1H). ¹⁹F NMR (377 MHz, chloroform-d) δ -145.48 (s, 1F).

### Step 2: Synthesis of compound WX006-4

To a pre-dried reaction flask were added **WX006-3** (3.0 g, 18.50 mmol, 1 *eq)* and ethanol (100 mL), then added palladium on carbon (1 g, 18.50 mmol, content of 10%, 1 *eq*). The reaction system was replaced with hydrogen three times. The mixture was maintained in a hydrogen (373.70 mg, 185.00 mmol, 10 *eq)* atmosphere at 50 psi, and stirred at 60°C for 16 hours. After the reaction was completed, the reaction mixture was directly filtered through diatomite. The filtrate was concentrated under reduced pressure to obtain a crude product. The crude product was purified by column chromatography (petroleum ether: ethyl acetate = 1:0) to obtain **WX006-4.** ¹H NMR (400 MHz, chloroform-d) δ 6.78-6.91 (m, 1H), 6.58 (dd, *J*=5.38, 8.38 Hz, 1H), 5.03 (d, *J*=5.13 Hz, 1H), 2.71 (s, 4H), 1.75-1.81 (m, 4H). ¹⁹F NMR (376 MHz, chloroform-d) δ -145.84 (s, 1F).

### Step 3: Synthesis of compound WX006-5

To a pre-dried reaction flask were added **WX006-4** (4 g, 24.07 mmol, 1 *eq)* and acetonitrile (80 mL), then added NBS (4.71 g, 26.48 mmol, 1.1 *eq).* The reaction system was replaced with nitrogen three times, and stirred at 25°C for 2 hours. After the reaction was completed, the reaction mixture was added with water (150 mL) and ethyl acetate (150 mL) to separate the phases. The organic phase was collected, and the aqueous phase was extracted with ethyl acetate (200 mL * 2). The resulting organic phases were combined, washed with saturated brine (300 mL * 3), dried over anhydrous sodium sulfate, and filtered. The filtrate was concentrated under reduced pressure to obtain a crude product. The crude product was purified by column chromatography (petroleum ether: ethyl acetate = 1:0 to 50:1) to obtain **WX006-5.** ¹H NMR (400 MHz, chloroform-d) δ 7.17 (d, *J*=9.51 Hz, 1H), 5.04 (s, 1H), 2.69 (td, *J*=5.85, 15.82 Hz, 4H), 1.73-1.85 (m, 1H), 1.77 (d, J=5.25 Hz, 3H). ¹⁹F NMR (377 MHz, dimethyl sulfoxide-d6) δ 0.00 (s, 1F).

### Step 4: Synthesis of compound WX006-6

To a pre-dried reaction flask were added **WX006-5** (6 g, 24.48 mmol, 1 *eq)* and DMF (120 mL). The reaction system was replaced with nitrogen three times, maintained at 0°C, and added with cesium carbonate (23.93 g, 73.44 mmol, 3 eq). Chloromethyl methyl ether (3.94 g, 48.96 mmol, 3.72 mL, 2 eq) was dropwise added thereto, and the mixture was stirred at 25°C for 2 hours. After the reaction was completed, the reaction mixture was poured into ice water (100 mL), and added with ethyl acetate (100 mL) to separate the phases. The organic phase was collected, and the aqueous phase was extracted with ethyl acetate (100 mL * 2). The resulting organic phases were combined, washed with saturated brine (100 mL * 3), dried over anhydrous sodium sulfate, and filtered. The filtrate was concentrated under reduced pressure to obtain a crude product. The crude product was purified by a thin-layer chromatography silica gel plate (petroleum ether: ethyl acetate = 10:1) to obtain **WX006-6.** ¹H NMR (400 MHz, chloroform-d) δ 7.20 (d, *J*=10.38 Hz, 1H), 5.11 (s, 2H), 3.53-3.65 (m, 3H), 2.78 (t, *J*=6.07 Hz, 2H), 2.67 (t, *J*=6.00 Hz, 2H), 1.70-1.82 (m, 4H). ¹⁹F NMR (376 MHz, chloroform-d) δ -132.23 (s, 1F).

### Step 5: Synthesis of compound WX006-7

To a pre-dried reaction flask were added **WX006-6** (1 g, 3.46 mmol, 1 *eq)* and tetrahydrofuran (10 mL). The reaction system was replaced with nitrogen three times and placed at -78°C. n-Butyllithium (2.5 M, 1.52 mL, 1.1 *eq)* was slowly added dropwise thereto. The mixture was stirred at -78°C for 1 hour, dropwise added with a mixture solution of **WX001-4** (663.22 mg, 3.11 mmol, 0.9 *eq)* and tetrahydrofuran (10 mL) at -78°C, and stirred at -78°C for another 1 hour. After the reaction was completed, the reaction mixture was poured into a saturated ammonium chloride solution (50 mL), and added with ethyl acetate (50 mL) to separate the phases. The organic phase was collected, and the aqueous phase was extracted with ethyl acetate (50 mL * 2). The resulting organic phases were combined, sequentially washed with saturated brine (50 mL * 2), dried over anhydrous sodium sulfate, and filtered. The filtrate was concentrated under reduced pressure to obtain a crude product. The crude product was purified by column chromatography (petroleum ether: ethyl acetate = 1:0 to 10:1 to 5:1) to obtain **WX006-7.** ¹H NMR (400 MHz, chloroform-d) δ 7.19 (s, 2H), 6.88 (d, *J*=12.88 Hz, 1H), 6.17 (d, *J*=3.50 Hz, 1H), 5.13 (s, 2H), 3.58 (s, 3H), 2.80 (d, *J*=3.00 Hz, 2H), 2.72-2.76 (m, 1H), 2.58 (d, *J*=17.64 Hz, 1H), 2.38 (s, 1H), 2.34 (s, 1H), 2.25 (s, 6H), 1.92 (d, *J*=4.00 Hz, 1H), 1.79 (d, *J*=5.63 Hz, 1H). ¹⁹F NMR (376 MHz, chloroform-d) δ -134.26 (s, 1F).

### Step 6: Synthesis of compound WX006-8

To a pre-dried reaction flask were added **WX006-7** (800 mg, 1.89 mmol, 1 *eq),* **WX001-7** (376.75 mg, 2.08 mmol, 348.84 µL, 1.1 *eq),* and 1,4-dioxane (16 mL), then added cesium carbonate (923.62 mg, 2.83 mmol, 1.5 *eq).* The reaction system was replaced with nitrogen three times, and sequentially added with Xantphos (87.48 mg, 151.19 µmol, 0.08 *eq)* and Pd₂(dba)₃ (103.83 mg, 113.39 µmol, 0.06 *eq),* and again replaced with nitrogen three times. The mixture was stirred at 100°C for 16 hours. After the reaction was completed, the reaction mixture was directly filtered through diatomite. The filtrate was concentrated under reduced pressure to obtain a crude product. The crude product was purified by column chromatography (petroleum ether: ethyl acetate = 1:0 to 20:1 to 10:1 to 3:1) to obtain **WX006-8.** ¹H NMR (400 MHz, chloroform-d) δ 7.84-7.92 (m, 1H), 7.87 (d, *J*=6.90 Hz, 1H), 7.62 (d, *J*=6.90 Hz, 2H), 7.48-7.54 (m, 3H), 7.32-7.38 (m, 3H), 7.21 (d, *J*=8.41 Hz, 2H), 6.85 (d, *J*=13.55 Hz, 1H), 6.11 (s, 1H), 5.13 (s, 2H), 3.59 (s, 3H), 2.79 (d, *J*=7.28 Hz, 3H), 2.62 (s, 1H), 2.12 (s, 6H), 1.81 (dd, *J*=3.39, 6.27 Hz, 2H), 1.69 (d, *J*=5.14 Hz, 1H), 1.55 (s, 1H). ¹⁹F NMR (376 MHz, chloroform-d) δ -133.29 (s, 1F).

### Step 7: Synthesis of compound WX006-9

To a pre-dried reaction flask were added **WX006-8** (600 mg, 1.15 mmol, 1 *eq)* and tetrahydrofuran (12 mL), then added hydrochloric acid (2 M, 572.92 µL, 1 *eq*)*.* The reaction system was replaced with nitrogen three times, then stirred at 25°C for 1 hour. After the reaction was completed, the reaction mixture was added with a saturated solution of sodium bicarbonate to adjust the pH to around 7. The aqueous phase was extracted with ethyl acetate (20 mL * 3). The organic phases were combined, and washed with saturated brine (20 mL * 2). The resulting organic phase was collected, dried over anhydrous sodium sulfate, and filtered. The filtrate was concentrated under reduced pressure to obtain a crude product. The crude product was purified by a thin-layer chromatography silica gel plate (petroleum ether: ethyl acetate = 1:1) to obtain **WX006-9.** ¹H NMR (400 MHz, chloroform-d) δ 7.05 (d, *J*=13.13 Hz, 1H), 6.47 (s, 2H), 6.12 (s, 1H), 5.12 (s, 2H), 3.59 (s, 3H), 2.78 (d, *J=*5.75 Hz, 2H), 2.25-2.32 (m, 2H), 2.23-2.24 (m, 1H), 2.19 (s, 6H), 1.66-1.76 (m, 4H). ¹⁹F NMR (377 MHz, chloroform-d) δ -134.74 (s, 1F).

### Step 8: Synthesis of compound WX006-10

To a pre-dried reaction flask were added MeOH (30 mL), glacial acetic acid (25 mL), and wet Pd/C (50 mg, content of 10%), then added **WX006-9** (500 mg, 1.39 mmol, 1 eq). The reaction system was replaced with hydrogen three times, and maintained in hydrogen atmosphere at 50 psi. The mixture was stirred at 25°C for 16 hours. After the reaction was completed, the reaction mixture was filtered through diatomite. The filtrate was concentrated under reduced pressure to obtain a crude product. The crude product was purified by column chromatography (petroleum ether: ethyl acetate = 20:1 to 10:1) to obtain **WX006-10.** ¹H NMR (400 MHz, chloroform-d) δ 6.47 - 6.37 (m, 2H), 6.15 (d, J = 13.0 Hz, 1H), 5.10 - 5.06 (m, 2H), 3.67 - 3.62 (m, 2H), 3.59 - 3.56 (m, 3H), 2.81 (br t, J = 6.1 Hz, 2H), 2.72 (br t, J = 6.1 Hz, 2H), 2.08 (s, 6H), 1.90 - 1.84 (m, 2H), 1.82 - 1.77 (m, 2H).

### Step 9: Synthesis of compound WX006-11

To a dry reaction flask was added a solution of **WX006-10** in THF (2 mL), and the reaction system was replaced with nitrogen three times. **WX001-10** was dissolved in THF (2 mL) and the solution was added to the reaction flask. TEA (150.27 mg, 1.49 mmol, 206.70 µL, 3 eq) was added thereto. The mixture was stirred at 20°C for 1 hour. After the reaction was completed, the reaction mixture was poured into water (5 mL), and extracted with ethyl acetate (5 mL) to separate the phases. The organic phase was collected, and the aqueous phase was extracted with ethyl acetate (5 mL * 2). The resulting organic phases were combined, sequentially washed with saturated brine (20 mL * 2), dried over anhydrous sodium sulfate, and filtered. The filtrate was dried under reduced pressure by rotary evaporation to obtain a crude product of **WX006-11,** which was directly used in the next step.

### Step 10: Synthesis of compound WX006

To a pre-dried reaction flask were added **WX006-11** and MeOH (5 mL), then added HCl (12 N, 60.19 µL, 1 *eq).* The reaction system was replaced with nitrogen three times, and stirred at 50°C for 0.5 hours. After the reaction was completed, the reaction mixture was quenched with a saturated solution of sodium bicarbonate, diluted with ethyl acetate (30 mL), and added with water (30 mL) to separate the phases. The organic phase was collected. The aqueous phase was extracted with ethyl acetate (30 mL * 3). The organic phases were combined, and washed with saturated brine (30 mL * 2). The resulting organic phase was collected, dried over anhydrous sodium sulfate, and filtered. The filtrate was concentrated under reduced pressure to obtain a crude product. The crude product was purified by HPLC (chromatographic column: Phenomenex luna C18 80 * 40 mm * 3 µm; mobile phase: [water (HCl) - acetonitrile]; acetonitrile%: 49% to 69%, 7 minutes) to obtain **WX006.** ¹H NMR (400 MHz, methanol-d4) δ 7.42 (s, 2H), 5.89 (d, *J* = 12.3 Hz, 1H), 3.77 (s, 2H), 2.76 - 2.67 (m, 4H), 2.17 (s, 6H), 1.90 - 1.77 (m, 4H); MS-ESI *m*/*z:* 410.1 [M-H]⁻.

### Example 6

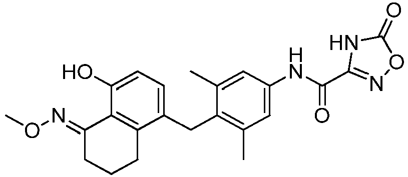

### Synthetic route:

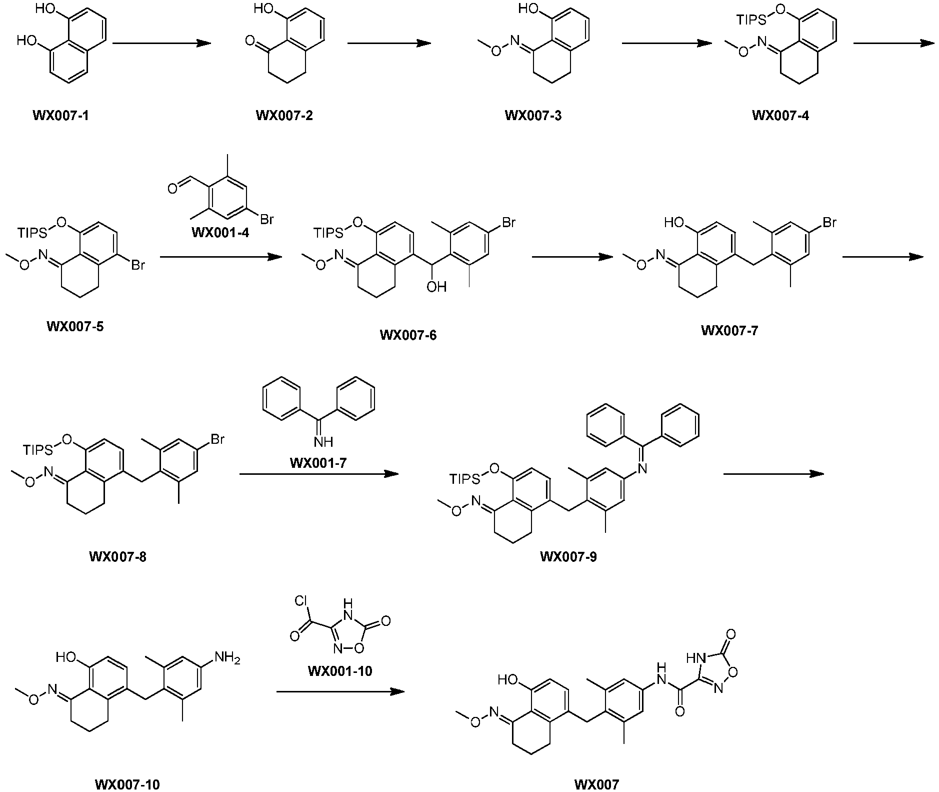

### Step 1: Synthesis of compound WX007-2

To two pre-dried hydrogenation flasks were respectively and sequentially added EtOH (1000 mL) and Pd/C (10 g, 312.17 mmol, content of 10%, 1 eq) under nitrogen atmosphere, then respectively added **WX007-1** (50 g, 312.17 mmol, 1 eq). The reaction system was replaced with hydrogen three times, maintained in a hydrogen atmosphere at 50 psi, and stirred at 60°C for 4 hours. After the reaction was completed, the reaction mixture was directly filtered through diatomite. The filtrate was concentrated under reduced pressure to obtain a crude product. The crude product was purified by column chromatography (petroleum ether: ethyl acetate = 1:0 to 10:1 to 5:1) to obtain **WX007-2.** ¹H NMR (400MHz, chloroform-d) δ 12.43 (s, 1H), 7.39 - 7.35 (m, 1H), 6.81 - 6.80 (m, 1H), 6.73 - 6.71 (m, 1H), 2.95 - 2.92 (m, 2H), 2.71 - 2.69 (m, 2H), 2.15 - 2.08 (m, 2H).

### Step 2: Synthesis of compound WX007-3

To a pre-dried reaction flask were added **WX007-2** (10 g, 61.66 mmol, 1 eq), methoxyamine hydrochloride (5.66 g, 67.82 mmol, 1.1 eq), and EtOH (100 mL), then added potassium carbonate (25.56 g, 184.97 mmol, 3 eq). The reaction system was replaced with nitrogen three times, then stirred at 60°C for 12 hours. After the reaction was completed, the reaction mixture was cooled to room temperature, and added with water (200 mL). The aqueous phase was extracted with ethyl acetate (200 mL * 3). The organic phases were combined, washed with saturated brine (300 mL * 1), dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1 to 5:1) to obtain **WX007-3.** ¹H NMR (400MHz, chloroform-d) δ 11.33 (s, 1H), 7.17 - 7.13 (m, 1H), 6.82-6.80 (d, 1H), 6.68 - 6.66 (d, 1H), 4.00 (s, 3H), 2.83 - 2.76 (m, 4H), 1.90 - 1.83 (m, 2H).

### Step 3: Synthesis of compound WX007-4

To a pre-dried reaction flask were added **WX007-3** (11 g, 57.52 mmol, 1 eq), DCM (200 mL), 2,6-lutidine (24.65 g, 230.09 mmol, 26.80 mL, 4 eq), and triisopropylsilyl trifluoromethanesulfonate (52.88 g, 172.57 mmol, 46.38 mL, 3 eq). The reaction system was replaced with nitrogen three times, and stirred at 25°C for 6 hours. After the reaction was completed, the reaction mixture was quenched with water (200 mL). The aqueous phase was extracted with dichloromethane (500 mL * 3). The organic phases were combined, washed with saturated brine (550 mL * 2), dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1 to 5:1) to obtain **WX007-4.** ¹H NMR (400MHz, chloroform-d) δ 7.07 - 7.03 (m, 1H), 6.77-6.72 (m, 2H), 3.97 (s, 3H), 2.76 - 2.72 (m, 2H), 2.61 - 2.58 (m, 2H), 1.75 - 1.70 (m, 2H), 1.35 - 1.29 (m, 3H), 1.28 - 1.10 (m, 18H).

### Step 4: Synthesis of compound WX007-5

To a pre-dried reaction flask were added **WX007-4** (4 g, 11.51 mmol, 1 eq) and acetonitrile (40 mL), then added NBS (2.25 g, 12.66 mmol, 1.1 eq). The reaction system was replaced with nitrogen three times, then stirred at 25°C for 2 hours. After the reaction was completed, the reaction mixture was quenched with water (50 mL). The aqueous phase was extracted with ethyl acetate (60 mL * 3). The organic phases were combined, washed with saturated brine (100 mL * 2). The resulting organic phase was collected, dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1) to obtain **WX007-5.** ¹H NMR (400MHz, chloroform-d) δ 7.32 - 7.30 (m, 1H), 6.67 - 6.65 (d, 1H), 3.97 (s, 3H), 2.75 - 2.71 (m, 4H), 1.75 - 1.72 (m, 2H), 1.33 - 1.25 (m, 3H), 1.11 - 1.09 (m, 18H).

### Step 5: Synthesis of compound WX007-6

To a pre-dried reaction flask were added **WX007-5** (0.5 g, 1.17 mmol, 1 eq) and THF (5 mL). The reaction system was replaced with nitrogen three times. n-Butyllithium (2.5 M, 515.87 µL, 1.1 eq) was slowly added dropwise thereto at -78°C, and the mixture was stirred at -78°C for 1 hour. The mixture was dropwise added with a mixture solution of **WX001-4** (249.81 mg, 1.17 mmol, 1 eq) and THF (5 mL) at -78°C, and stirred at -78°C for another 1 hour. After the reaction was completed, the reaction mixture was quenched by pouring it into a saturated solution of ammonium chloride (5 mL), and added with ethyl acetate (5 mL) to separate the phases. The organic phase was collected, and the aqueous phase was extracted with ethyl acetate (2 * 5 mL). The resulting organic phases were combined, sequentially washed with saturated brine (2 * 5 mL), dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1) to obtain **WX007-6.** ¹H NMR (400MHz, dimethyl sulfoxide-d6) δ 7.18 (s, 3H), 6.95 - 6.93 (m, 1H), 6.70 - 6.68 (m, 1H), 6.24 - 6.23 (d, 1H), 3.97 (s, 3H), 2.74 - 2.70 (m, 3H), 2.63 (m, 1H), 2.23 (s, 6H), 1.70 - 1.66 (m, 2H), 1.30 - 1.26 (m, 3H), 1.24 - 1.08 (m, 18H).

### Step 6: Synthesis of compound WX007-7

To a pre-dried reaction flask were added **WX007-6** (470 mg, 838.33 µmol, 1 eq) and DCM (10 mL). The reaction system was replaced with nitrogen three times and placed at 0°C. Et₃SiH (146.22 mg, 1.26 mmol, 200.85 µL, 1.5 eq) and TFA (143.38 mg, 1.26 mmol, 93.10 µL, 1.5 eq) were sequentially and slowly added thereto in drops, and the mixture was stirred at 0°C for 1 hour. After the reaction was completed, the reaction mixture was poured into a saturated aqueous solution of sodium carbonate (30 mL), and extracted with DCM (30 mL * 3). The organic phases were combined, and washed with saturated brine (30 mL). After separating the phases, the organic phase was dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1) to obtain **WX007-7.** ¹H NMR (400MHz, dimethyl sulfoxide-d6) δ 11.25 (s, 1H), 7.30 (s, 2H), 6.58 - 6.56 (m, 1H), 6.23 - 6.21 (m, 1H), 3.96 (s, 3H), 3.78 (s, 2H), 2.88 - 2.80 (m, 4H), 2.11 (s, 6H), 1.88 - 1.85 (m, 2H).

### Step 7: Synthesis of compound WX007-8

To a pre-dried reaction flask were added **WX007-7** (320 mg, 824.11 µmol, 1 eq), DCM (6.4 mL), 2,6-lutidine (353.21 mg, 3.30 mmol, 383.93 µL, 4 eq), and triisopropylsilyl trifluoromethanesulfonate (757.57 mg, 2.47 mmol, 664.54 µL, 3 eq). The reaction system was replaced with nitrogen three times, and stirred at 25°C for 1 hour. After the reaction was completed, the reaction mixture was quenched with water (200 mL). The aqueous phase was extracted with dichloromethane (500 mL * 3). The organic phases were combined, sequentially washed with saturated brine (550 mL * 2), dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1 to 5:1) to obtain **WX007-8.** ¹H NMR (400MHz, dimethyl sulfoxide-d6) δ 7.21 (s, 2H), 6.57 - 6.55 (d, 1H), 6.25 - 6.23 (m, 1H), 3.97 (s, 3H), 3.80 (s, 2H), 2.78 - 2.69 (m, 4H), 2.13 (s, 6H), 1.82 - 1.80 (m, 2H), 1.27 - 1.21 (m, 3H), 1.08 (m, 18H).

### Step 8: Synthesis of compound WX007-9

To a pre-dried reaction flask were added **WX007-8** (518 mg, 951.09 µmol, 1 eq), **WX001-7** (189.61 mg, 1.05 mmol, 175.56 µL, 1.1 eq), and dioxane (10 mL), then added cesium carbonate (464.83 mg, 1.43 mmol, 1.5 eq). The reaction system was replaced with nitrogen three times, and sequentially added with Xantphos (44.03 mg, 76.09 µmol, 0.08 eq) and Pd₂(dba)₃ (52.26 mg, 57.07 µmol, 0.06 eq), and again replaced with nitrogen three times. The mixture was stirred at 100°C for 6 hours. After the reaction was completed, the reaction mixture was directly filtered through diatomite. The filtrate was concentrated under reduced pressure to obtain **WX007-9.** Step 9: Synthesis of compound **WX007-10**

To a pre-dried reaction flask were added **WX007-9** (615 mg, 953.55 µmol, 1 eq), THF (10 mL), and HCl (2N, 1.91 mL, 4 eq). The reaction system was replaced with nitrogen three times, and stirred 25°C for 1 hour. After the reaction was completed, the reaction mixture was quenched with a saturated solution of sodium bicarbonate (100 mL). The aqueous phase was extracted with ethyl acetate (100 mL * 3). The organic phases were combined, and washed with saturated brine (150 mL * 2). The resulting organic phase was collected, dried over anhydrous sodium sulfate, and filtered. The filtrate was concentrated under reduced pressure to obtain a crude product. The crude product was purified by column chromatography (petroleum ether: ethyl acetate = 1:0 to 10:1 to 5:1 to 2:1) to obtain **WX007-10.** ¹H NMR (400MHz, chloroform-d) δ 6.64 - 6.62 (m, 1H), 6.47 - 6.45 (m, 3H), 3.99 (s, 3H), 3.72 (s, 2H), 3.53 (s, 1H), 2.89 - 2.83 (m, 4H), 2.08 (s, 6H), 1.96 - 1.95(m, 2H).

### Step 10: Synthesis of compound WX007

To a dry reaction flask was added a solution of **WX001-10** (32.96 mg, 221.94 µmol, 0.8 eq) in THF (1 mL). **WX007-10** (90 mg, 277.42 µmol, 1 eq) was dissolved in THF (1 mL) and the solution was added to the reaction flask. TEA (84.22 mg, 832.27 µmol, 115.84 µL, 3 eq) was added thereto. The mixture was stirred at 20°C for 1 hour. After the reaction was completed, the reaction mixture was poured into water (5 mL), and extracted with ethyl acetate (5 mL) to separate the phases. The organic phase was collected, and the aqueous phase was extracted with ethyl acetate (5 mL * 2). The resulting organic phases were combined, sequentially washed with saturated brine (20 mL * 2), dried over anhydrous sodium sulfate, and filtered. The filtrate was concentrated under reduced pressure to obtain a crude product. The crude product was purified by HPLC (chromatographic column: Waters Xbridge BEH C18 100 * 30 mm * 10 µm; mobile phase: [water (ammonium bicarbonate) - acetonitrile]; acetonitrile%: 35% to 55%, 8 minutes) to obtain a liquid. The liquid was extracted and the organic phase was collected, dried over anhydrous sodium sulfate, and filtered. The filtrate was concentrated under reduced pressure to obtain **WX007.** ¹H NMR (400MHz, methanol-d4) δ 7.41 (s, 2H), 6.51 (d, J = 8.5 Hz, 1H), 6.32 (d, J = 8.6 Hz, 1H), 3.98 (s, 3H), 3.85 (s, 2H), 2.92 - 2.85 (m, 4H), 2.16 (s, 6H), 1.97 - 1.91 (m, 2H); MS-ESI *m*/*z:* 437.2 [M+H]⁺.

### Example 7

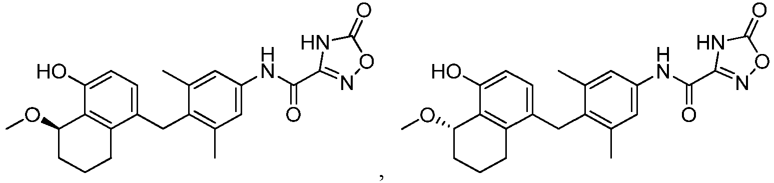

### Synthetic route:

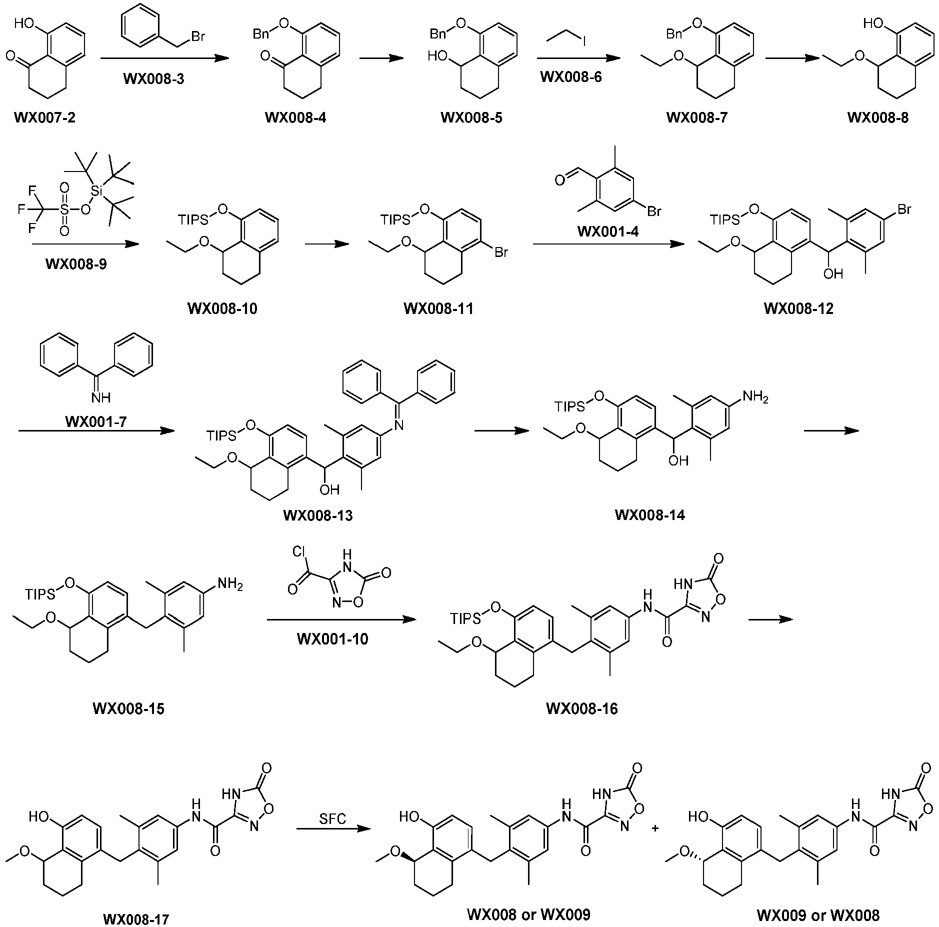

### Step 1: Synthesis of compound WX008-4

To a pre-dried reaction flask were added **WX007-2** (113 g, 696.74 mmol, 1 eq) and DMF (1050 mL), then added **WX008-3** (214.50 g, 1.25 mol, 148.96 mL, 1.8 eq) and potassium carbonate (173.33 g, 1.25 mol, 1.8 eq). The reaction system was replaced with nitrogen three times, and stirred at 25°C for 16 hours. After the reaction was completed, the system was added with water (1000 mL), and extracted with methyl tert-butyl ether (500 mL * 3). After separating the phases, the organic phases were combined, washed with saturated brine (500 mL * 2), dried over anhydrous sodium sulfate, and filtered. The filtrate was concentrated under reduced pressure using a water pump at 50°C to obtain a crude product in the form of a pale yellow solid. The crude product was purified by flash column chromatography (silica gel: 100 to 200 mesh; petroleum ether: ethyl acetate = 1:0 to 100:2) to obtain **WX008-4.**

### Step 2: Synthesis of compound WX008-5

To a pre-dried reaction flask were added **WX008-4** (90 g, 356.71 mmol, 1 eq) and MeOH (900 mL). The mixture was slowly added with sodium borohydride (16.19 g, 428.05 mmol, 1.2 eq) at 0°C, and stirred at 0°C for 0.5 hours. After the reaction was completed, the reaction mixture was quenched with a saturated solution of ammonium chloride (900 mL), and added with ethyl acetate (900 mL). The phases were separated. The aqueous phase was extracted with ehtyl acetate (900 mL * 2). The organic phases were combined, washed with saturated brine (900 mL * 2). The resulting organic phase was collected, dried over anhydrous sodium sulfate, and filtered. The filtrate was concentrated under reduced pressure. The crude product was purified by flash column chromatography (petroleum ether: ethyl acetate = 1:0 to 100:1) to obtain **WX008-5.** ¹H NMR (400MHz, chloroform-d) δ 7.48 - 7.32 (m, 5H), 7.16 (t, J = 8.0 Hz, 1H), 6.80 (dd, J = 8.1, 10.1 Hz, 2H), 5.20 - 5.08 (m, 3H), 3.10 (s, 1H), 2.88 - 2.77 (m, 1H), 2.75 - 2.63 (m, 1H), 2.10 - 1.98 (m, 1H), 1.98 - 1.88 (m, 2H), 1.80 - 1.70 (m, 1H).

### Step 3: Synthesis of compound WX008-7

To a pre-dried reaction flask were added **WX008-5** (20 g, 78.64 mmol, 1 eq) and THF (200 mL). The mixture was slowly added with sodium hydride (12.58 g, 314.56 mmol, content of 60%, 4 eq) at 0°C, and stirred at 0°C for 0.5 hours. **WX008-6** (36.80 g, 235.92 mmol, 18.87 mL, 3 eq) was dropwise added thereto at 0°C, and the mixture was stirred at 0°C for 0.5 hours. The reaction mixture was warmed to 25°C, and stirred for another 15 hours. After the reaction was completed, the reaction mixture was quenched with water (100 mL). The aqueous phase was extracted with ethyl acetate (100 mL * 3). The organic phases were combined, washed with saturated brine (100 mL * 3). The resulting organic phase was collected, dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1 to 5:1) to obtain **WX008-**7. ¹HNMR (400MHz, chloroform-d) δ 7.48 - 7.39 (m, 2H), 7.33 - 7.37 (m, 3H), 7.14 - 7.12 (m, 1H), 6.76 - 6.72 (m, 2H), 5.14 - 5.05 (m, 2H), 4.72 - 4.70 (m, 1H), 3.68 - 3.64 (m, 1H), 3.52 - 3.48 (m, 1H), 2.86 - 2.81 (m, 1H), 2.71 - 2.68 (m, 1H), 2.24 - 2.23 (m, 1H), 2.21 - 2.20 (m, 1H), 1.74 - 1.73 (m, 1H), 1.54 - 1.51 (m, 1H), 1.17 - 1.13 (m, 3H).

### Step 4: Synthesis of compound WX008-8

To a pre-dried reaction flask were added ethyl acetate (300 mL) and Pd/C (2 g, content of 10%), then added **WX008-7** (14.36 g, 50.85 mmol, 1 eq). The reaction system was replaced with hydrogen three times, maintained in a hydrogen atmosphere at 15 psi, and stirred at 25°C for 16 hours. After the reaction was completed, the reaction mixture was filtered through diatomite. The filtrate was concentrated under reduced pressure to obtain a crude product. The crude product was purified by column chromatography (petroleum ether: ethyl acetate = 1:0 to 20:1 to 10:1) to obtain **WX008-8.** ¹H NMR (400MHz, chloroform-d) δ 8.33 (s, 1H), 7.11 - 7.07 (m, 1H), 6.73 - 6.71 (m, 1H), 6.65 - 6.63 (m, 1H), 4.91 - 4.88 (m, 1H), 3.80 - 3.76 (m, 1H), 3.63 - 3.59 (m, 1H), 2.79 - 2.76 (m, 1H), 2.71 - 2.69 (m, 1H), 2.25 (m, 1H), 1.92 - 1.91 (m, 2H), 1.86 - 1.72 (m, 1H), 1.34 - 1.30 (m, 3H).

### Step 5: Synthesis of compound WX008-10

To a pre-dried reaction flask were added **WX008-8** (6.35 g, 33.03 mmol, 1 eq) and DCM (120 mL). The reaction system was replaced with hydrogen three times. 2,6-Lutidine (8.85 g, 82.57 mmol, 9.62 mL, 2.5 eq) was slowly added dropwise thereto. Then **WX008-9** (20.24 g, 66.06 mmol, 17.76 mL, 2 eq) was dropwise added thereto, and the mixture was stirred at 25°C for 16 hours. After the reaction was completed, the reaction mixture was quenched with water (120 mL). The aqueous phase was extracted with dichloromethane (150 mL * 3). The organic phases were combined, washed with saturated brine (100 mL * 2). The resulting organic phase was collected, dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1) to obtain **WX008-10.** ¹H NMR (400MHz, chloroform-d) δ 7.04 - 7.00 (m, 1H), 6.68 - 6.63 (m, 2H), 4.78 - 4.77 (m, 1H), 3.72 - 3.68 (m, 1H), 3.52 - 3.48 (m, 1H), 2.84 (m, 1H), 2.70 - 2.68 (m, 1H), 2.31 - 2.28 (m, 1H), 2.10 (m, 1H), 1.69 (m, 1H), 1.47 (m, 1H), 1.36 - 1.34 (m, 3H), 1.21 - 1.19 (m, 3H), 1.17 - 1.12 (m, 18H).

### Step 6: Synthesis of compound WX008-11

To a pre-dried reaction flask were added **WX008-10** (11 g, 31.56 mmol, 1 eq) and acetonitrile (220 mL), then added NBS (6.18 g, 34.71 mmol, 1.1 eq). The reaction system was replaced with nitrogen three times, and stirred at 25°C for 2 hours. After the reaction was completed, the reaction mixture was quenched with water (50 mL). The aqueous phase was extracted with ethyl acetate (60 mL * 3). The organic phases were combined, washed with saturated brine (100 mL * 2). The resulting organic phase was collected, dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1)to obtain **WX008-11.** ¹H NMR(400MHz, chloroform-d) δ 7.31 - 7.29 (m, 1H), 6.58 - 6.56 (m, 1H), 4.77 - 4.75 (m, 1H), 3.72 - 3.68 (m, 1H), 3.51 - 3.47 (m, 1H), 2.90 - 2.88 (m, 1H), 2.51 (m, 1H), 2.30 - 2.29 (m, 1H), 2.26 (m, 1H), 1.75 (m, 1H), 1.55 (s, 1H), 1.44 - 1.32 (m, 3H), 1.21 - 1.18 (m, 3H), 1.17 - 1.11 (m, 18H).

### Step 7: Synthesis of compound WX008-12

To a dry reaction flask were added **WX008-11** and THF (20 mL). The reaction system was replaced with nitrogen, cooled to -78°C, dropwise added with n-BuLi (2.5 M, 2.06 mL, 1.1 eq), and reacted at -78°C for 1 hour. The mixture was then added with a solution of **WX001-4** in THF (10 mL), and reacted at -78°C for 1 hour. After the reaction was completed, the reaction mixture was quenched with 20 mL of saturated ammonium chloride solution, and extracted with 30 mL of ethyl acetate. After separating the phases, the organic phase was collected, and the aqueous phase was extracted with ethyl acetate. The resulting organic phases were combined, washed with saturated brine (20 mL * 3), dried over anhydrous sodium sulfate, and filtered. The filtrate was concentrated under reduced pressure to obtain a crude product. The crude product was purified by an automatic column chromatography machine (petroleum ether: ethyl acetate = 1:0 to 5:1) to obtain **WX008-12.**

### Step 8: Synthesis of compound WX008-13

To a reaction flask were added **WX008-12** (680 mg, 1.21 mmol, 1 eq), dioxane (7 mL), then added **WX001-7** (241.36 mg, 1.33 mmol, 223.48 µL, 1.1 eq) and cesium carbonate (591.70 mg, 1.82 mmol, 1.5 eq). The reaction system was first replaced with nitrogen, then added with Pd₂(dba)₃ (49.89 mg, 54.48 µmol, 0.045 eq) and Xantphos (31.52 mg, 54.48 µmol, 0.045 eq), and again replaced with nitrogen three times. The mixture was heated to 100°C, and stirred for 12 hours. After the reaction was completed, the reaction system was cooled to room temperature, and the reaction mixture was filtered. The filtrate was concentrated under reduced pressure to obtain a crude product. The crude product was purified by an automatic column chromatography machine (petroleum ether: ethyl acetate = 1:0 to 5:1) to obtain **WX008-13.**

### Step 9: Synthesis of compound WX008-14

To a pre-dried reaction flask were added **WX008-13** (470 mg, 709.99 µmol, 1 eq) and THF (8 mL). The reaction system was replaced with nitrogen three times, cooled to 0°C, and added with dilute hydrochloric acid (2 M, 354.99 µL, 1 eq). The mixture was stirred at 20°C for 0.5 hours. After the reaction was completed, the reaction mixture was added with a saturated solution of sodium bicarbonate to adjust the pH to around 7. The aqueous phase was extracted with ethyl acetate (10 mL * 3). The organic phases were combined, and washed with saturated brine (10 mL * 2). The resulting organic phase was collected, dried over anhydrous sodium sulfate, and filtered. The filtrate was concentrated under reduced pressure to obtain a crude product. The crude product was purified by an automatic column chromatography machine (petroleum ether: ethyl acetate = 1:0 to 3:1 to 2:1) to obtain **WX008-14.**

### Step 10: Synthesis of compound WX008-15

To a pre-dried reaction flask introduced with argon were added Pd/C (10 mg, content of 10%), ethyl acetate (18 mL), glacial acetic acid (2 mL), and **WX008-14** (175 mg, 351.56 µmol, 1 eq). The reaction system was replaced with hydrogen, and stirred at 25°C for 4 hours at 15 psi. After the reaction was completed, the reaction mixture was directly filtered through diatomite. The filtrate was concentrated under reduced pressure to obtain a crude product. The crude product was directly used in the next step. Thus **WX008-15** was obtained. ¹H NMR (400 MHz, chloroform-d) δ 6.50 - 6.44 (m, 3H), 6.27 (d, *J* = 8.3 Hz, 1H), 4.81 (s, 1H), 3.77 - 3.73 (m, 1H), 3.73 - 3.68 (m, 1H), 3.65 (s, 1H), 3.59 (s, 1H), 3.56 - 3.51 (m, 1H), 2.92 (dd, *J* = 5.8, 17.2 Hz, 1H), 2.58 (ddd, *J* = 6.7, 11.6, 17.7 Hz, 1H), 2.37 - 2.26 (m, 1H), 2.17 - 2.09 (m, 1H), 2.07 (s, 6H), 1.89 - 1.76 (m, 1H), 1.30 - 1.26 (m, 3H), 1.22 (t, *J=* 7.1 Hz, 3H), 1.11 (dd, *J* = 7.5, 11.2 Hz, 18H).

### Step 11: Synthesis of compound WX008-16

To a dry reaction flask were added **WX008-15** (260 mg, 539.66 µmol, 1 eq), THF (0.3 mL), then added a solution of **WX001-10** (120.21 mg, 809.49 µmol, 1.5 eq) in THF (0.3 mL), and triethylamine (163.82 mg, 1.62 mmol, 225.34 µL, 3 eq). The mixture was stirred at 20°C for 1 hour. After the reaction was completed, the reaction mixture was quenched with 10 mL of saturated solution of sodium bicarbonate, and extracted with 30 mL of ethyl acetate. After separating the phases, the organic phase was collected, and the aqueous phase was extracted with ethyl acetate (20 mL * 3). The organic phases were combined, sequentially washed with saturated brine (20 mL * 3), dried over anhydrous sodium sulfate, and filtered. The filtrate was concentrated under reduced pressure to obtain a crude product. The crude product was purified by an automatic column chromatography machine (petroleum ether: ethyl acetate = 1:0 to 2:1) to obtain **WX008-16.** ¹H NMR (400 MHz, chloroform-d) δ 8.49 (br s, 1H), 7.23 (br s, 2H), 6.42 (br d, J = 8.4 Hz, 1H), 6.16 (br d, J = 8.4 Hz, 1H), 4.78 (br s, 1H), 3.77 - 3.68 (m, 1H), 3.67 - 3.59 (m, 1H), 3.58 - 3.47 (m, 2H), 3.27 (q, J = 7.4 Hz, 1H), 2.89 - 2.79 (m, 1H), 2.29 (br d, J = 12.9 Hz, 2H), 2.10 (br s, 1H), 2.05 (br d, J = 3.1 Hz, 6H), 1.81 (br d, J = 1.6 Hz, 1H), 1.26 (td, J = 3.8, 7.3 Hz, 3H), 1.22 - 1.18 (m, 3H), 1.06 (dd, J = 7.4, 11.6 Hz, 18H).

### Step 12: Synthesis of compound WX008-17

To a dry reaction flask were added **WX008-16** (270 mg, 454.68 µmol, 1 eq) and THF (0.3 mL), then added triethylamine trihydrofluoride (366.49 mg, 2.27 mmol, 370.57 µL, 5 eq). The reaction system was replaced with nitrogen three times, and stirred at 50°C for 12 hours. After the reaction was completed, the reaction system was cooled to room temperature, and the reaction mixture was quenched with 30 mL of water, and extracted with 20 mL of ethyl acetate. After separating the phases, the resulting organic phase was collected, and the aqueous phase was extracted with ethyl acetate (20 mL * 3). The organic phases were combined, sequentially washed with saturated brine (20 mL * 3), dried over anhydrous sodium sulfate, and filtered. The filtrate was concentrated under reduced pressure to obtain a crude product. The crude product was found to have poor solubilities in dichloromethane and ethyl acetate upon inverting the bottle, so a small amount of methanol was used to dissolve it. The resulting solution was concentrated and evaporated to dryness by rotary evaporation. Upon analysis, it was found that the ethoxy group had been converted to a methoxy group. The crude product was purified (chromatographic column: Phenomenex C18 80 * 40 mm * 3 µm; mobile phase: [water (ammonium bicarbonate) - acetonitrile]; acetonitrile%: 25% to 45%, 8 minutes) to obtain **WX008-17.**

### Step 13: Synthesis of compound WX008 and WX009

**WX008-17** (60 mg, 101.04 µmol) was subjected to chiral resolution (chromatographic column: DAICEL CHIRALPAK AD (250 mm*30 mm, 10 µm); mobile phase: A: carbon dioxide B: [0.1% ammonia water - ethanol]; B%: 35% to 35%, 14 minutes) to obtain **WX008** and **WX009.** The compounds were detected by a chiral method (method: chromatographic column: Chiralpak AD-3, 50 × 4.6 mm I.D., 3 µm; mobile phase: A: carbon dioxide B: ethanol (0.1% IPAm, v/v); flow rate: 2.5 mL/min; column temperature: 35°C; pressure: 2000 psi), showing **WX008** (ee% = 98.64%, retention time of 3.482 minutes), and **WX009** (ee% = 97.98%, retention time of 3.831 minutes). The product **WX009** was then purified (chromatographic column: Phenomenex C18 75 * 30 mm * 3 µm; mobile phase: [water (ammonia water + ammonium bicarbonate) - acetonitrile]; acetonitrile%: 15% to 45%, 8 minutes) to obtain **WX009.**

**WX008:** ¹H NMR (400 MHz, methanol-d4) δ 7.41 (s, 2H), 6.44 (d, *J* = 8.1 Hz, 1H), 6.19 (d, *J=* 8.6 Hz, 1H), 4.67 (t, *J=* 3.2 Hz, 1H), 3.86 - 3.67 (m, 2H), 3.47 (s, 3H), 2.97 - 2.87 (m, 1H), 2.67 - 2.54 (m, 1H), 2.29 - 2.22 (m, 1H), 2.14 (s, 6H), 2.03 - 1.94 (m, 1H), 1.91 - 1.84 (m, 1H), 1.65 - 1.55 (m, 1H).

**WX009:** ¹H NMR (400 MHz, methanol-d4) δ 7.42 (s, 2H), 6.45 (d, *J* = 8.3 Hz, 1H), 6.20 (d, *J=* 8.3 Hz, 1H), 4.67 (t, *J* = 2.8 Hz, 1H), 3.86 - 3.66 (m, 2H), 3.47 (s, 3H), 2.98 - 2.86 (m, 1H), 2.61 (ddd, *J* = 6.2, 11.4, 17.6 Hz, 1H), 2.32 - 2.20 (m, 1H), 2.14 (s, 6H), 2.05 - 1.96 (m, 1H), 1.92 - 1.80 (m, 1H), 1.66 - 1.55 (m, 1H).

### Example 8

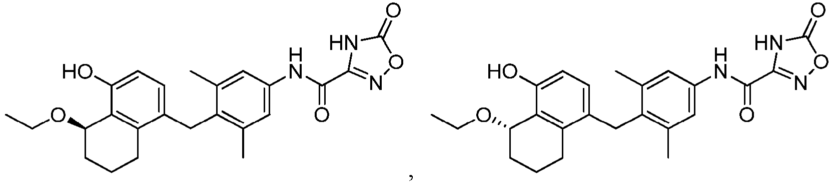

### Synthetic route:

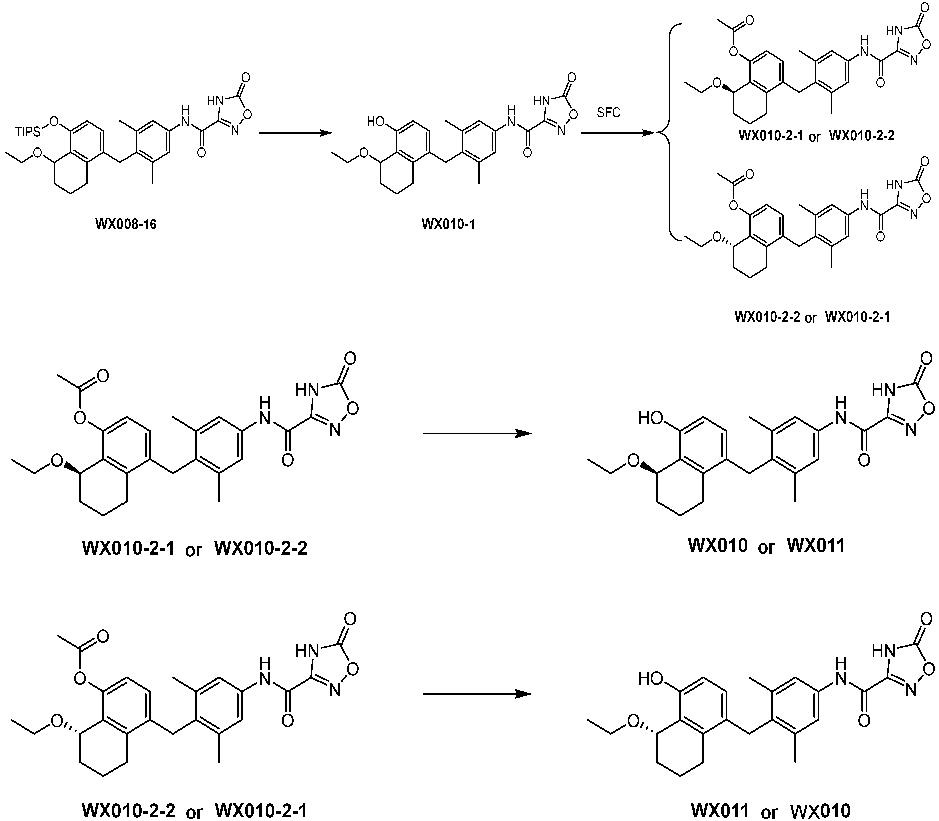

### Step 1: Synthesis of compound WX010-1

To a pre-dried reaction flask were sequentially added **WX008-16** (200 mg, 336.80 µmol, 1 eq), THF (5 mL), and tetrabutylammonium fluoride (1 M, 404.16 µL, 1.2 eq). The reaction system was replaced with nitrogen three times, and stirred at 20°C for 1 hour. After the reaction was completed, the reaction mixture was directly concentrated under reduced pressure to obtain a crude product. The crude product was purified by pre-TLC (dichloromethane: methanol = 10:1) to obtain **WX010-1.**

### Step 2: Synthesis of compounds WX010-2-1 and WX010-2-2

To a pre-dried reaction flask were added **WX010-1** (70 mg, 160.00 µmol, 1 eq) and pyridine (2 mL), then added acetic anhydride (245.02 mg, 2.40 mmol, 224.79 µL, 15 eq). The mixture was stirred at 25°C for 1 hour. After the reaction was completed, the reaction mixture was added with 0.5 N hydrochloric acid to adjust the pH to between 4 and 5, and extracted with dichloromethane (5 mL). The organic phase was collected, and the aqueous phase was extracted with dichloromethane (5 mL * 2). The resulting organic phases were combined, sequentially washed with 0.5 M hydrochloric acid (10 mL * 2), washed with saturated brine (20 mL * 2), dried over anhydrous sodium sulfate, and filtered. The filtrate was concentrated under reduced pressure to obtain a crude product. The crude product was purified by supercritical fluid chromatography (chromatographic column: DAICEL CHIRALPAK AD (250 mm * 30 mm, 10 µm); mobile phase: A: carbon dioxide B: [0.1% ammonia water - ethanol]; B%: 25% to 25%, 11 minutes) to obtain **WX010-2-1** and **WX010-2-2.** The compounds were detected by chiral method (method: chromatographic column: Chiralpak AD-3, 150 × 4.6 mm I.D., 3 µm; mobile phase: A: carbon dioxide B: methanol (0.1%IPAm, v/v); flow rate: 2.5 mL/min; column temperature: 35°C; pressure: 2000 psi), showing that the retention time for **WX010-2-1** was 2.893 minutes, with an ee% of 100%; the retention time for **WX010-2-2** was 3.190 minutes, with an ee% of 91.06%.

### Step 3: Synthesis of compounds WX010 and WX011

To a pre-dried reaction flask were sequentially added **WX010-2-1** (16 mg, 33.37 µmol, 1 eq) and MeOH (4 mL), then added potassium carbonate (9.22 mg, 66.73 µmol, 2 eq). The reaction system was replaced with nitrogen three times, and stirred at 20°C for 4 hours. After the reaction was completed, the reaction mixture was filtered, and concentrated under reduced pressure to obtain a crude product. The crude product was purified by HPLC (chromatographic column: Phenomenex C18 75 * 30 mm * 3 µm; mobile phase: [water (ammonium bicarbonate) - acetonitrile]; acetonitrile%: 25% to 55%, 8 minutes) to obtain **WX010.** ¹H NMR (400MHz, methanol-d4) δ 7.42 (s, 2H), 6.44 (d, J = 8.3 Hz, 1H), 6.19 (d, J = 8.1 Hz, 1H), 4.78 (t, J = 2.9 Hz, 1H), 3.85 - 3.75 (m, 2H), 3.75 - 3.67 (m, 2H), 2.98 - 2.88 (m, 1H), 2.68 - 2.56 (m, 1H), 2.26 - 2.18 (m, 1H), 2.16 - 2.12 (m, 6H), 2.08 - 1.99 (m, 1H), 1.92 - 1.83 (m, 1H), 1.68 - 1.58 (m, 1H), 1.21 (t, J = 7.0 Hz, 3H); MS-ESI *m*/*z:* 436.2 [M-H]⁺.

To a pre-dried reaction flask were sequentially added **WX010-2-2** (10.00 mg, 20.85 µmol, 1 eq) and MeOH (2.5 mL), then added potassium carbonate (5.76 mg, 41.71 µmol, 2 eq). The reaction system was replaced with nitrogen three times, and stirred at 20°C for 4 hours. After the reaction was completed, the reaction mixture was filtered, and concentrated under reduced pressure to obtain a crude product. The crude product was purified by HPLC (chromatographic column: Phenomenex C18 75 * 30 mm * 3 µm; mobile phase: [water (ammonium bicarbonate) - acetonitrile]; acetonitrile%: 25% to 55%, 8 minutes) to obtain **WX011.** ¹H NMR (400MHz, methanol-d4) δ 7.42 (s, 2H), 6.44 (d, J = 8.4 Hz, 1H), 6.19 (d, J = 8.4 Hz, 1H), 4.78 (t, J = 3.1 Hz, 1H), 3.84 - 3.76 (m, 2H), 3.74 - 3.68 (m, 2H), 3.00 - 2.87 (m, 1H), 2.69 - 2.55 (m, 1H), 2.25 - 2.19 (m, 1H), 2.14 (s, 6H), 2.07 - 2.00 (m, 1H), 1.93 - 1.82 (m, 1H), 1.67 - 1.59 (m, 1H), 1.21 (t, J = 7.1 Hz, 3H); MS-ESI *m*/*z:* 436.1 [M-H]⁻.

### Example 9

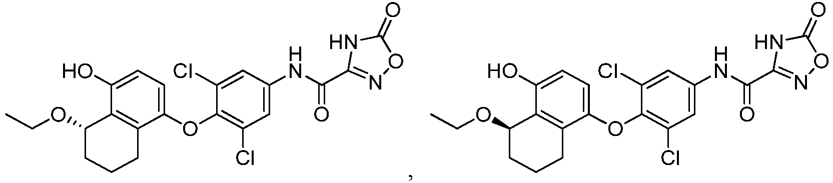

### Synthetic route:

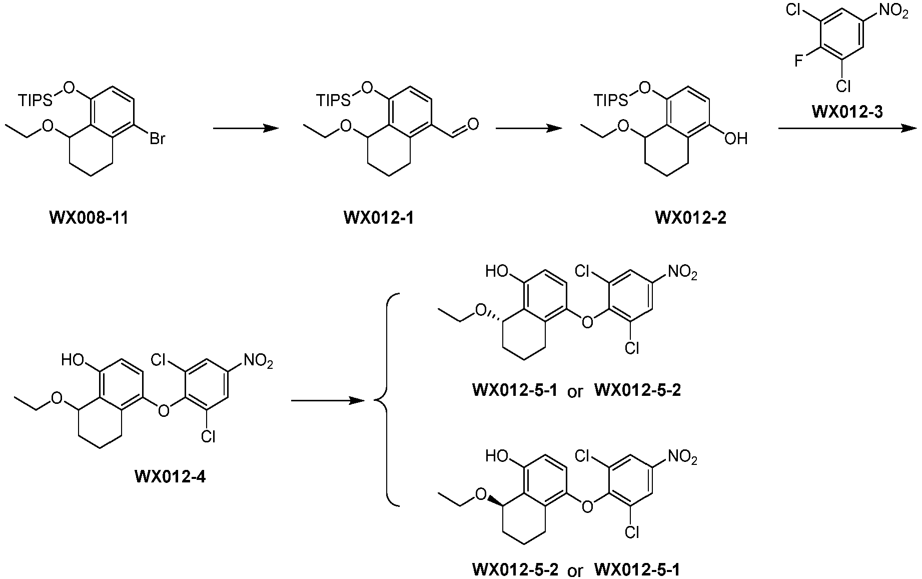

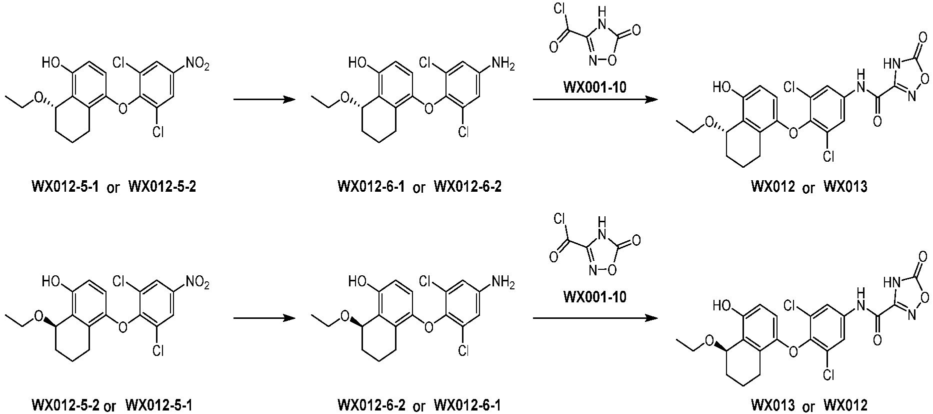

### Step 1: Synthesis of compound WX012-1

To a pre-dried reaction flask were added **WX008-11** (5 g, 11.70 mmol, 1 eq) and THF (100 mL). The reaction system was replaced with nitrogen three times and placed at -78°C. DMF (3.42 g, 46.78 mmol, 3.60 mL, 4 eq) was slowly added dropwise thereto, and the mixture was stirred at -78°C for 0.5 hours. The mixture was dropwise added with n-butyllithium (2.5 M, 5.15 mL, 1.1 eq) at -78°C, and stirred at -78°C for another 1 hour. After the reaction was completed, the reaction mixture was poured into a saturated solution of ammonium chloride (20 mL), and added with ethyl acetate (20 mL) to separate the phases. The organic phase was collected, and the aqueous phase was extracted with ethyl acetate (20 mL * 2). The resulting organic phases were combined, sequentially washed with saturated brine (20 mL * 2), dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1 to 5:1) to obtain **WX012-1.** ¹H NMR (400MHz, chloroform-d) δ 10.107 (s, 1H), 7.66 - 7.63 (m, 1H), 6.80 - 6.78 (m, 1H), 4.79 (s, 1H), 3.74 - 3.71 (m, 1H), 3.54 - 3.48 (m, 1H), 2.94 (m, 1H), 2.34 (m, 1H), 2.05 (m, 1H), 1.80 (m, 1H), 1.55 (s, 1H), 1.45 - 1.39 (m, 3H), 1.37 - 1.35 (m, 3H), 1.22 - 1.19 (m, 18H).

### Step 2: Synthesis of compound WX012-2

To a pre-dried reaction flask were added **WX012-1** (1 g, 2.66 mmol, 1 eq) and DCM (10 mL), then added 3-chloroperoxybenzoic acid (1.13 g, 6.55 mmol, 2.47 eq). The reaction system was replaced with nitrogen three times, and stirred at 25°C for 16 hours. After the reaction was completed, the reaction mixture was quenched with the addition of a saturated solution of sodium bicarbonate (20 mL), stirred for 30 minutes, and added with dichloromethane (20 mL) to separate the phases. The organic phase was collected, and the aqueous phase was extracted with dichloromethane (20 mL * 2). The organic phases were combined, washed with saturated brine (20 mL * 2), dried over anhydrous sodium sulfate, and filtered. The filtrate was concentrated under reduced pressure to obtain an intermediate. To a pre-dried reaction flask were added the intermediate (1 g, 2.66 mmol, 1 eq), DCM (5 mL), and MeOH (5 mL), then added TEA (1.42 g, 14.07 mmol, 1.96 mL, 5.3 eq). The reaction system was replaced with nitrogen three times, and stirred at 25°C for another 40 minutes. After the reaction was completed, the reaction mixture was diluted with dichloromethane (10 mL), and washed with 2 N hydrochloric acid (10 mL). The organic phase was collected, washed with a saturated solution of sodium bicarbonate (10 mL), then washed with saturated brine (20 mL), dried over anhydrous sodium sulfate, and filtered. The filtrate was concentrated under reduced pressure to obtain a crude product. The crude product was purified by column chromatography (petroleum ether: ethyl acetate = 1:0 to 20:1 to 10:1 to 5:1) to obtain **WX012-2.** ¹H NMR (400MHz, chloroform-d) δ 6.56 - 6.55 (m, 2H), 4.76 - 4.75 (s , 1H), 4.34 (s, 1H), 3.73 - 3.69 (m, 1H), 3.53 - 3.49 (m, 1H), 2.77 (m, 1H), 2.46 - 2.44 (m, 1H), 2.29 - 2.26 (m, 1H), 2.06 (m, 1H), 1.75 (m, 1H), 1.55 (s, 1H), 1.42 - 1.31 (m, 3H), 1.22 - 1.20 (m, 3H), 1.18 - 1.11 (m, 18H).

### Step 3: Synthesis of compound WX012-4

To a pre-dried reaction flask were added **WX012-2** (690 mg, 1.89 mmol, 1 eq) and DMF (14 mL), then added potassium carbonate (392.34 mg, 2.84 mmol, 1.5 eq). The reaction system was replaced with nitrogen three times, stirred at 25°C for 0.5 hours, slowly added with **WX012-3** (516.63 mg, 2.46 mmol, 1.3 eq), and replaced with nitrogen three times. The mixture was stirred at 100°C for 15.5 hours. After the reaction was completed, the reaction mixture was quenched with water (30 mL). The phases were separated. The aqueous phase was extracted with ethyl acetate (50 mL * 3). The organic phases were combined, washed with saturated brine (100 mL * 3). The resulting organic phase was collected, dried over anhydrous sodium sulfate, and filtered. The filtrate was concentrated under reduced pressure to obtain a crude product. The crude product was purified by column chromatography (petroleum ether: ethyl acetate = 1:0 to 10:1 to 5:1) to obtain **WX012-4.** ¹H NMR (400MHz, chloroform-d) δ 8.31(m, 1H), 8.28 (m, 2H), 6.03 - 6.01 (m, 1H), 5.97- 5.95 (m, 1H), 4.90 (s, 1H), 3.85 - 3.81 (m, 1H), 3.73 - 3.69 (m, 1H), 3.23 - 3.18 (m, 1H), 2.72 - 2.71 (m, 1H), 2.37 - 2.33 (m, 1H), 2.15 (m, 1H), 1.95 - 1.94 (m, 1H), 1.70 - 1.68 (m, 1H), 1.29 - 1.26 (m, 3H).

### Step 4: Synthesis of compounds WX012-5-1 and WX012-5-2

**WX012-4** (500 mg, 1.26 mmol, 1 eq) was purified supercritical fluid chromatography (chromatographic column: DAICEL CHIRALPAK AD (250 mm * 30 mm, 10 µm); mobile phase: A: carbon dioxide B: [0.1% ammonia water + isopropanol]; B%: 30% to 30%, 10 minutes) to obtain **WX012-5-1** and **WX012-5-2.** The compounds were detected by chiral method (instrument: CAS-TJ-ANA-SFC-H (Waters UPCC with SQ Detector 2); chromatographic column: Chiralpak AD-3, 50 × 4.6 mm I.D., 3 µm; mobile phase: A: food-grade supercritical carbon dioxide; B: isopropanol (0.1% isopropylamine, by volume); gradient: The content of B was increased from 5% to 50% in 1.2 minutes, and held for 1 minute, then increased from 50% to 5% within 0.4 minutes; flow rate: 3.4 mL/min; column temperature: 35°C; detection wavelength: 220 nm; system back pressure: 100 ba). The retention time for **WX012-5-1** was 1.197, with an ee% of 100%; the retention time for **WX012-5-2** was 1.295, with an ee% of 98.56%. **WX012-5-1:** ¹H NMR (400MHz, dimethyl sulfoxide-d6) δ 8.52 (s, 2H), 8.50 - 8.48 (m, 1H), 6.29 - 6.21 (m, 1H), 6.19 - 6.14 (m, 1H), 4.80 (s, 1H), 3.82 - 3.68 (m, 1H), 3.65 - 3.52 (m, 1H), 3.06 (br dd, J = 3.1, 17.8 Hz, 1H), 2.74 - 2.64 (m, 1H), 2.33 (td, J = 1.8, 3.6 Hz, 1H), 2.29 - 2.18 (m, 1H), 1.99 - 1.79 (m, 1H), 1.69 - 1.54 (m, 1H), 1.14 (t, J = 7.0 Hz, 3H). **WX012-5-2:** ¹H NMR (400MHz, dimethyl sulfoxide-d6) δ 8.51 (s, 2H), 8.51 - 8.47 (m, 1H), 6.29 - 6.21 (m, 1H), 6.20 - 6.11 (m, 1H), 4.80 (s, 1H), 3.83 - 3.69 (m, 1H), 3.60 (dd, J = 7.1, 8.9 Hz, 1H), 3.06 (br dd, J = 3.4, 17.9 Hz, 1H), 2.72 - 2.59 (m, 1H), 2.35 - 2.21 (m, 1H), 1.97 - 1.80 (m, 2H), 1.67 - 1.56 (m, 1H), 1.14 (t, J = 6.9 Hz, 3H).

### Step 5: Synthesis of compounds WX012-6-1 and WX012-6-2

To a pre-dried reaction flask were added **WX012-5-1,** glacial acetic acid (1 mL), isopropanol (2 mL), and water (1 mL). At 50°C, iron powder (98.17 mg, 1.76 mmol, 7 eq) was slowly added thereto in batches. The reaction system was replaced with nitrogen three times, and the mixture was stirred at 90°C for 2 hours. After the reaction was completed, the reaction mixture was cooled to room temperature, and filtered through diatomite. The filtrate was concentrated under reduced pressure. The concentrate was added with a saturated solution of sodium bicarbonate to adjust the pH to between 7 and 8. The aqueous phase was extracted with ethyl acetate (10 mL * 3). The organic phases were combined, sequentially washed with saturated brine (20 mL * 2), dried over anhydrous sodium sulfate, and filtered. The filtrate was concentrated under reduced pressure to obtain a crude product. The crude product was purified by pre-TLC (petroleum ether: ethyl acetate = 1:1) to obtain **WX012-6-1.** ¹H NMR (400MHz, dimethyl sulfoxide-d6) δ 6.67 (s, 2H), 6.15 - 6.07 (m, 1H), 6.05 - 5.96 (m, 1H), 5.58 (s, 2H), 4.76 (br s, 1H), 3.78 - 3.66 (m, 1H), 3.65 - 3.55 (m, 1H), 3.00 (br dd, J = 4.3, 18.0 Hz, 1H), 2.70 - 2.57 (m, 1H), 2.24 - 2.11 (m, 1H), 1.93 - 1.72 (m, 2H), 1.59 - 1.46 (m, 1H), 1.12 (t, J = 7.0 Hz, 3H).

To a pre-dried reaction flask were added **WX012-5-2** (150 mg, 376.66 µmol, 1 eq), glacial acetic acid (1.5 mL), isopropanol (3 mL), and water (1.5 mL). At 50°C, iron powder (147.26 mg, 2.64 mmol, 7 eq) was slowly added thereto in batches. The reaction system was replaced with nitrogen three times, and the mixture was stirred at 90°C for 2 hours. After the reaction was completed, the reaction mixture was cooled to room temperature, and filtered through diatomite. The filtrate was concentrated under reduced pressure. The concentrate was added with a saturated solution of sodium bicarbonate to adjust the pH to between 7 and 8. The aqueous phase was extracted with ethyl acetate (10 mL * 3). The organic phases were combined, sequentially washed with saturated brine (20 mL * 2), dried over anhydrous sodium sulfate, and filtered. The filtrate was concentrated under reduced pressure to obtain a crude product. The crude product was purified by pre-TLC (petroleum ether: ethyl acetate = 1:1) to obtain **WX012-6-2.** ¹H NMR (400MHz, dimethyl sulfoxide-d6) δ 6.67 (s, 2H), 6.19 - 6.07 (m, 1H), 6.05 - 5.96 (m, 1H), 5.58 (s, 2H), 4.76 (br s, 1H), 3.79 - 3.52 (m, 2H), 3.00 (br dd, J = 3.9, 17.8 Hz, 1H), 2.69 - 2.53 (m, 1H), 2.18 (br d, J = 12.8 Hz, 1H), 1.95 - 1.73 (m, 2H), 1.61 - 1.47 (m, 1H), 1.17 - 1.05 (m, 3H).

### Step 6: Synthesis of compounds WX012 and WX013

To a dry reaction flask was added a solution of **WX001-10** (48.39 mg, 325.86 µmol, 1.5 eq) in THF (1.6 mL). **WX012-6-1** (80 mg, 217.24 µmol, 1 eq) was dissolved in THF (1 mL) and the solution was added to the reaction flask. TEA (65.95 mg, 651.72 µmol, 90.71 µL, 3 eq) was added thereto. The mixture was stirred at 20°C for 1 hour. After the reaction was completed, the reaction mixture was poured into water (5 mL), and extracted with ethyl acetate (5 mL) to separate the phases. The organic phase was collected, and the aqueous phase was extracted with ethyl acetate (5 mL * 2). The resulting organic phases were combined, sequentially washed with saturated brine (20 mL * 2), dried over anhydrous sodium sulfate, and filtered. The filtrate was concentrated under reduced pressure to obtain a crude product. The crude product was purified by HPLC (chromatographic column: Waters Xbridge BEH C18 100 * 30 mm * 10 µm; mobile phase: [water (ammonium bicarbonate) - acetonitrile]; acetonitrile%: 20% to 65%, 10 minutes) to obtain **WX012.** ¹HNMR(400MHz, methanol-d4) δ 7.94 (br s, 2H), 6.21 - 6.14 (m, 1H), 6.13 - 6.06 (m, 1H), 5.00 (br s, 1H), 3.92 - 3.78 (m, 2H), 3.22 (br dd, J = 5.0, 17.9 Hz, 1H), 2.73 (ddd, J = 6.0, 11.9, 18.2 Hz, 1H), 2.40 - 2.31 (m, 1H), 2.13 - 1.91 (m, 2H), 1.76 - 1.63 (m, 1H), 1.31 - 1.22 (m, 3H).

To a dry reaction flask was added a solution of **WX001-10** (66.54 mg, 448.06 µmol, 1.5 eq) in THF (2 mL). **WX012-6-2** (110 mg, 298.71 µmol, 1 eq) was dissolved in THF (1 mL) and the solution was added to the reaction flask. TEA (90.68 mg, 896.12 µmol, 124.73 µL, 3 eq) was added thereto. The mixture was stirred at 20°C for 1 hour. After the reaction was completed, the reaction mixture was poured into water (5 mL), and extracted with ethyl acetate (5 mL) to separate the phases. The organic phase was collected, and the aqueous phase was extracted with ethyl acetate (5 mL * 2). The resulting organic phases were combined, sequentially washed with saturated brine (20 mL * 2), dried over anhydrous sodium sulfate, and filtered. The filtrate was concentrated under reduced pressure to obtain a crude product. The crude product was purified by HPLC (chromatographic column: Waters Xbridge BEH C18 100 * 30 mm * 10 µm; mobile phase: [water (ammonium bicarbonate) - acetonitrile]; acetonitrile%: 20% to 65%, 10 minutes) to obtain **WX013.** ¹H NMR(400MHz, methanol-d4) δ 7.94 (br s, 2H), 6.22 - 6.15 (m, 1H), 6.12 - 6.12 (m, 1H), 6.15 - 6.09 (m, 1H), 5.00 (br s, 1H), 3.92 - 3.77 (m, 2H), 3.22 (br dd, J = 4.5, 18.1 Hz, 1H), 2.73 (ddd, J = 6.3, 11.9, 18.0 Hz, 1H), 2.43 - 2.29 (m, 1H), 2.14 - 1.91 (m, 2H), 1.77 - 1.63 (m, 1H), 1.31 - 1.24 (m, 3H).

### Bioassay

### Experimental example 1: Testing agnostic activity of compounds of the present disclosure on thyroid hormone receptors at cellular level

### Experimental principle:

The present experiment employs the GeneBLAzer^{®} TR alpha/beta-UAS-bla HEK 293T Cell-based Assay developed by ThermoFisher. The underlying principle is that TR alpha-UAS-bla HEK 293T Cell and TR beta-UAS-bla HEK 293T Cell express β-lactamase, a reporter gene controlled by the upstream UAS sequence. When a compound enters the cell and binds to THR, the receptor binds to the DNA binding region to form a complete GAL4 dimer that utilizes the GAL4-UAS system to activate β-lactamase expression, which then decomposes the substrate CCF4-AM (coumarin). The resulting product, when excited at 409 nm, emits fluorescence at a wavelength of 447 nm. If β-lactamase is not expressed, the product generates fluorescence at a wavelength of 520 nm through FRET under excitation at 409 nm. By detecting the ratio of the two fluorescences (447 nm/520 nm), the binding status of the compound to the protein can be determined, thus allowing the calculation of the EC₅₀ of the compound.

### Experimental method:

The ECHO liquid handling workstation was used to transfer the compound to a 384-well plate, with 10 gradient concentrations for each compound, 3-fold dilution, and duplicate wells. 1.5×10⁴ cells (TR beta-UAS-bla HEK 293T Cell) or 1.0×10⁴ cells (TR alpha-UAS-bla HEK 293T Cell) were seeded onto the 384-well plate. HEK 293T-TR beta cells were incubated for 16 hours in a 37°C incubator, and HEK 293T-TR alpha cells were incubated for 22 hours. The LiveBLAzer^{™}-FRET B/G (CCF4-AM) substrate was added to the cell plate and incubated for 2 hours at room temperature, shielded from light. The Flexstation 3 instrument was utilized to detect the fluorescence values with the wavelength of 460 nm/530 nm emitted by the product under the excitation at 409 nm. By detecting the ratio of the two fluorescences (460 nm/530 nm), the EC₅₀ of the compound was calculated using Graphpad Prism software. In each experiment, the reference compound triiodothyronine (T3) was taken as a positive control. The Z-factor (greater than 0.5) is calculated to monitor the stability of each experiment.

**Table 1. Activity of THRα and THRβ in each examples**

| Compound | THRα EC₅₀ (nM), maximum agnostic capability | THRβ EC₅₀ (nM), maximum agnostic capability |
|---|---|---|
| T3 | 0.057, 100% | 0.2, 100% |
| WX001 | 121.9, 24% | 39.42, 103% |
| WX002 | 999.6, 101% | 85.55, 99% |
| WX003 | 149.1, 88% | 27.62, 93% |
| WX004 | 76.24, 96% | 9.073, 94% |
| WX005 | 155.1, 85% | 57.84, 96% |
| WX006 | 435, 86% | 104.9, 90% |
| WX008 | 402.6, 79% | 118.6, 94% |
| WX009 | 772.9, 83% | 154.2, 93% |
| WX010 | 93.05, 90% | 9.95, 94% |
| WX011 | 65.53, 73% | 13, 97% |

Experimental conclusion: The compounds of the present disclosure exhibit significant THRβ activity and selectivity.

### Experimental example 2: Study on inhibition of cytochrome P450 isoenzymes

### Experimental purpose:

To determine the inhibitory effect of the test compound WX001 on the activity of human liver microsomal cytochrome P450 isoenzymes (CYP1A2, CYP2C9, CYP2C19, CYP2D6, and CYP3A4).

### Experimental procedure:

First, the test compound (10.0 mM) was diluted to prepare a working solution (100 × the final concentration) with a concentration of 1.00 mM. Simultaneously, working solutions were respectively prepared for positive inhibitors of the P450 isoenzymes (CYP1A2, CYP2C9, CYP2C19, CYP2D6, CYP3A4 (with midazolam as a probe substrate), and CYP3A4 (with testosterone as a probe substrate)) and specific probe substrates thereof. Human liver microsomes, stored in a refrigerator at a temperature lower than -60°C, were thawed on ice, and once fully dissolved, were diluted with PB to prepare a working solution of a specific concentration (0.127 mg/mL). 20.0 µL of probe substrate was added to the reaction plate (20.0 µL of PB was added to the Blank wells), followed by the addition of 158 µL of human liver microsomal working solution to the reaction plate. The reaction plate was then placed on ice for later use. Then, 2.00 µL of the test compound (N=1) and specific inhibitors (N=2) were added to the corresponding wells. In groups without inhibitors (either with test compound or positive inhibitors), the corresponding organic solvent was added. The organic phases for the test compound control samples and positive control samples were DMSO and MeOH at a ratio of 1 to 1, and DMSO and MeOH at a ratio of 1 to 9, respectively. After pre-incubating in a 37°C water bath for 10 minutes, 20.0 µL of the cofactor (NADPH) solution was added to the reaction plate. For the CYP3A4 metabolic reaction using midazolam as the probe substrate, the reaction lasted 3 minutes; for the CYP2C19 reaction using (S)-mephenytoin as the probe substrate, and the CYP2D6 reaction using dextromethorphan as the probe substrate, the reaction lasted 20 minutes; for all other reactions, the reaction lasted 10 minutes. The reaction was then terminated by adding 400 µL of pre-cooled acetonitrile solution (containing 200 ng/mL of internal standards Tolbutamide and Labetalol). Placed on a shaker, the reaction plate was shaken and evenly mixed for 10 minutes, then centrifuged at 4°C and 4000 rpm for 20 minutes. 200 µL of the supernatant was taken and added to 100 µL of water for sample dilution. Lastly, the plate was sealed, shaken to ensure the content was thoroughly mixed, and analyzed by LC/MS/MS.

### Experimental results:

### As shown in Table 2.

**Table 2. Results of inhibition effects of test compound on activity of human liver microsomal cytochrome P450 isoenzyme**

| Compound | IC₅₀ (µM) | | | | |
|---|---|---|---|---|---|
| | CYP1A2 | CYP2C9 | CYP2C19 | CYP2D6 | CYP3A4-M |
| WX001 | 40.9 | 3.27 | 5.09 | 41.6 | 25.4 |

Experimental conclusion: Compound WX001 exhibits no inhibitory effects on CYP1A2, CYP2C19, CYP2D6, and CYP3A4, but demonstrates moderate inhibition against CYP2C9.

### Experimental example 3: In vivo pharmacokinetics study

Pharmacokinetic study of oral and intravenous administration of WX001 in rats

Male SD rats were selected, and given the test compound as detailed in Table 3.

**Table 3. Administration and blood collection of compounds of the present disclosure**

| Compound | Route of administration | Dosage mpk | Vehicle | Concentration (mg/mL) | Time points for blood collection (hour) |
|---|---|---|---|---|---|
| WX001 | Intravenous | 3 | 10% Dimethyl sulfoxide/10% Solutol/80% water | 1.5 | 0.0833, 0.250, 0.500, 1.00, 2.00, 4.00, 8.00, 12.0, 24.0 |
| WX001 | Oral | 30 | 0.5% Sodium carboxymethyl cellulose + 0.2% Tween 80 aqueous solution | 6 | 0.250, 0.500, 1.00, 2.00, 4.00, 8.00, 12.0, 24.0 |

Whole blood was collected for a certain period of time and prepared into plasma. The drug concentration was analyzed by the LC-MS/MS method, and the pharmacokinetic parameters were calculated using Phoenix WinNonlin software (Pharsight, USA). Experimental results: As shown in Table 4 and Table 5:

**Table 4. Pharmacokinetic results of compound of the present disclosure through intravenous administration**

| Compound | Route of administration | Dosage mpk | Half-life T_{1/2} (h) | Clearance CL (mL/min/kg) | Apparent volume of distribution Vdₛₛ (L/kg) | Area under the plasma concentration -time curve / Dosage DNAUC₀₋ₗₐₛₜ (nM.h/mpk) |
|---|---|---|---|---|---|---|
| WX001 | Intravenous | 3 | 1.66 | 7.93 | 0.411 | 5343 |

**Table 5. Pharmacokinetic results of compound of the present disclosure through oral administration**

| Compound | Route of administration | Dosage (mpk) | Half-life T_{1/2} (h) | Peak concentration Cₘₐₓ (nM) | Area under the plasma concentration-time curve / Dosage DNAUC₀₋ₗₐₛₜ (nM.h/mpk) | Bioavailability |
|---|---|---|---|---|---|---|
| WX001 | Oral | 30 | 1.6 | 3 | 1939 | 36% |

Conclusion: The compound of the present disclosure exhibits high exposure and good oral bioavailability.

### Experimental example 4: In vivo pharmacokinetics study

### Experimental purpose:

To test the in vivo efficacy of the compound to be tested using a rat model induced by cholesterol and bile acid-supplemented feed.

### Experimental method:

Male SD rats, aged 9-10 weeks, were selected and allowed to acclimate for 3-7 days upon arrival at the facility. During the acclimation period, the health of the animals was observed daily, and regular feed was provided. After the acclimation period, rats were fed with a high-cholesterol diet (1.5% cholesterol and 0.5% bile acid) for modeling, while the blank control group of rats continued to be provided with regular feed. After the rats were fed with the high-cholesterol diet for two weeks, blood was collected, and serum was separated to detect LDL-C levels. The high cholesterol model rats were randomly grouped according to LDL-C levels in serum, and then administered orally for 7 consecutive days, once a day, as detailed in Table 6. One week after the administration, rat serum was collected to detect LDL-C level and assess the drug efficacy.

**Table 6. Animal grouping**

| Group | Administration group | Vehicle | Feed | Number of animals | Route of administration | Frequency and period of administration |
|---|---|---|---|---|---|---|
| 1 | blank control | 0.5% sodium carboxymethyl cellulose + 0.2% Tween 80 aqueous solution | regular feed | 3 | oral gavage | QD, 7 days |
| 2 | vehicle control | | high-cholesterol diet | 6 | oral gavage | QD, 7 days |
| 3 | WX001, 1 mpk | | high-cholesterol diet | 6 | oral gavage | QD, 7 days |
| 4 | WX001, 3 mpk | | high-cholesterol diet | 6 | oral gavage | QD, 7 days |

### Experimental results:

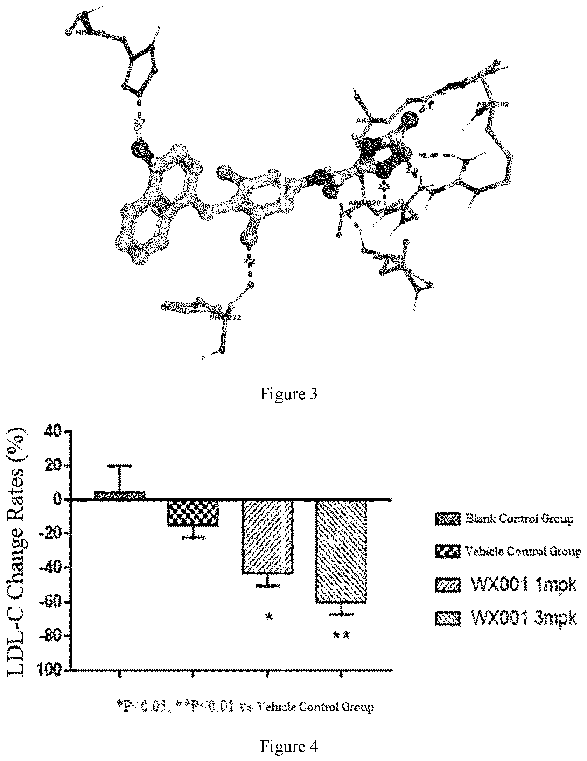

See Figure 4, where P < 0.05 indicates a statistically significant difference in the pharmacodynamic index between the administration group and the vehicle group.

Experimental conclusion: The compound of the present disclosure can significantly reduce the LDL-C levels in rat plasma.

## Claims

1. A compound of formula (I) or a pharmaceutically acceptable salt thereof, 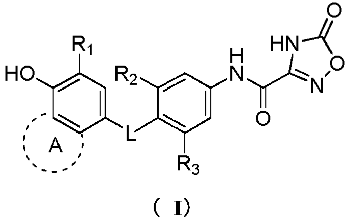 wherein
R₁ is independently selected from H, F, Cl, Br, I, and C₁₋₃ alkyl, and the C₁₋₃ alkyl is optionally substituted by 1, 2, or 3 Rₐ;
R₂ and R₃ are each independently selected from H, F, Cl, Br, I, OH, NH₂, CN, C₁₋₃ alkyl, and C₁₋₃ alkoxy, and the C₁₋₃ alkyl and C₁₋₃ alkoxy are optionally substituted by 1, 2, or 3 R_{b};
L is selected from -O- and -CH₂-;
ring A is selected from phenyl, 5- to 6-membered heteroaryl, 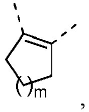 and 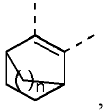 and the phenyl, 5- to 6-membered heteroaryl, 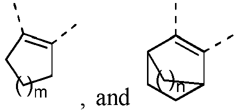 are optionally substituted by 1, 2, or 3 R_{c};
n and m are each independently selected from 0, 1, and 2;
Rₐ and R_{b} are each independently selected from F, Cl, Br, and I;
R_{c} is independently selected from F, Cl, Br, I, =O, =N-C₁₋₃ alkoxy, C₁₋₃ alkyl, and C₁₋₃ alkoxy, and the =N-C₁₋₃ alkoxy, C₁₋₃ alkyl, and C₁₋₃ alkoxy are optionally substituted by 1, 2, or 3 R;
R is independently selected from F, Cl, Br, and I.

2. The compound or the pharmaceutically acceptable salt thereof according to claim 1, wherein R₁ is independently selected from H, F, Cl, Br, I, and CH₃, and the CH₃ is optionally substituted by 1, 2, or 3 Rₐ.

3. The compound or the pharmaceutically acceptable salt thereof according to claim 2, wherein R₁ is independently selected from H, F, Cl, Br, I, CH₃, and CF₃.

4. The compound or the pharmaceutically acceptable salt thereof according to any one of claims 1 to 3, wherein R₂ and R₃ are each independently selected from H, F, Cl, Br, I, OH, NH₂, CN, CH₃, and OCH₃, and the CH₃ and OCH₃ are optionally substituted by 1, 2, or 3 R_{b}.

5. The compound or the pharmaceutically acceptable salt thereof according to claim 4, wherein R₂ and R₃ are each independently selected from H, F, Cl, Br, I, OH, NH₂, CN, CH₃, CH₂F, CHF₂, CF₃, and OCH₃.

6. The compound or the pharmaceutically acceptable salt thereof according to any one of claims 1 to 3, wherein R_{c} is independently selected from F, Cl, Br, I, =O, =N-O-CH₃, =N-O-CH₂CH₃, CH₃, OCH₃, and OCH₂CH₃, and the =N-O-CH₃, =N-O-CH₂CH₃, CH₃, OCH₃, and OCH₂CH₃ are optionally substituted by 1, 2, or 3 R.

7. The compound or the pharmaceutically acceptable salt thereof according to claim 6, wherein R_{c} is independently selected from F, Cl, Br, I, =O, =N-O-CH₃, =N-O-CH₂CH₃, CH₃, OCH₃, and OCH₂CH₃.

8. The compound or the pharmaceutically acceptable salt thereof according to any one of claims 1 to 3, wherein ring A is selected from 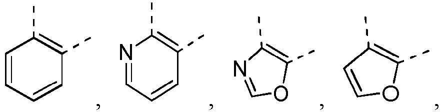 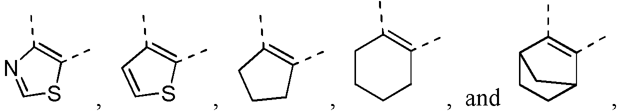 and the 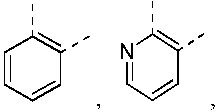 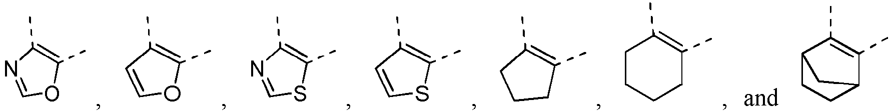 are optionally substituted by 1, 2, or 3 R_{c}.

9. The compound or the pharmaceutically acceptable salt thereof according to claim 8, wherein ring A is selected from

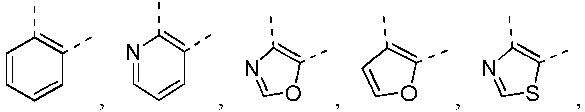

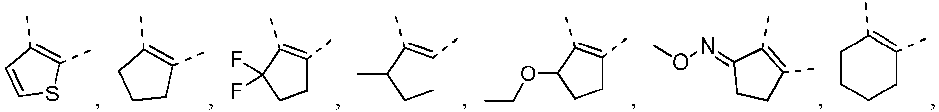

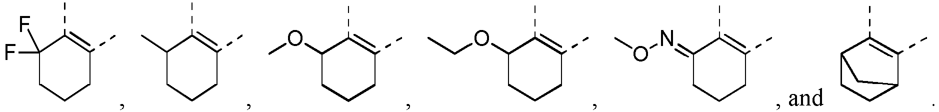

10. The compound or the pharmaceutically acceptable salt thereof according to any one of claims 1 to 3, and 9, wherein the structural moiety 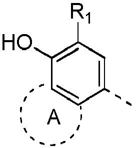 is selected from

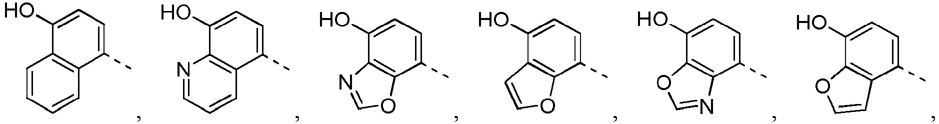

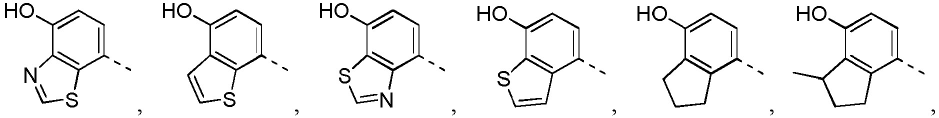

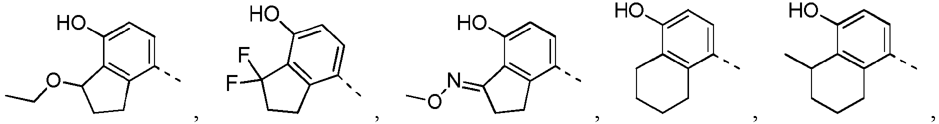

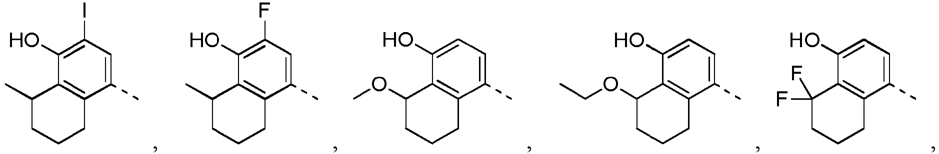

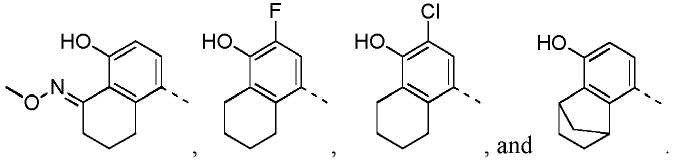

11. The compound or the pharmaceutically acceptable salt thereof according to any one of claims 1 to 10, selected from: 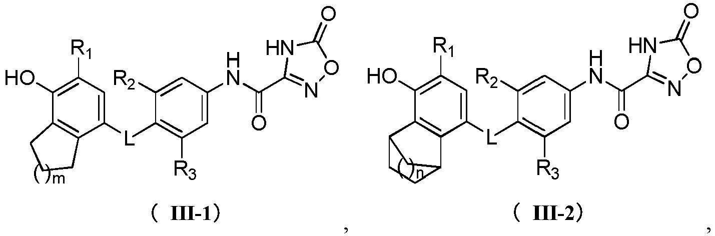 wherein R₁, R₂, R₃, L, m, and n are as defined in any one of claims 1 to 10.

12. The compound or the pharmaceutically acceptable salt thereof according to any one of claims 1 to 10, selected from: 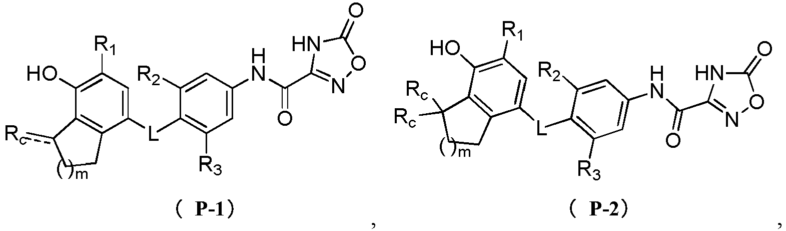 wherein
when 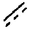 is a single bond, R_{c} is F, Cl, Br, I, C₁₋₃ alkyl, and C₁₋₃ alkoxy, and the C₁₋₃ alkyl and C₁₋₃ alkoxy are optionally substituted by 1, 2, or 3 halogens;
when 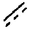 is a double bond, R_{c} is =O or =N-C₁₋₃ alkoxy, and the =N-C₁₋₃ alkoxy is optionally substituted by 1, 2, or 3 halogens;
R₁, R₂, R₃, L, and m are as defined in any one of claims 1 to 10.

13. The compound or the pharmaceutically acceptable salt thereof according to claim 1, selected from:

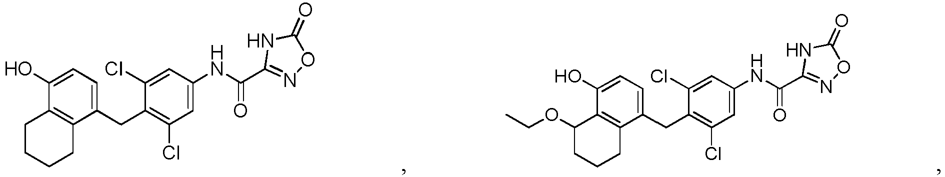

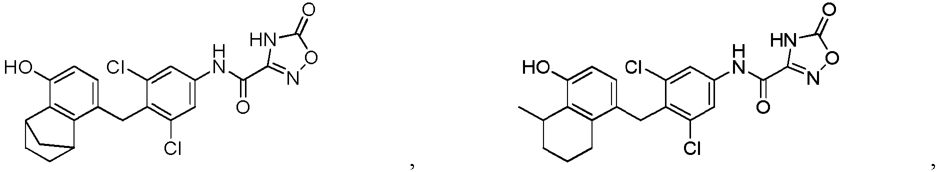

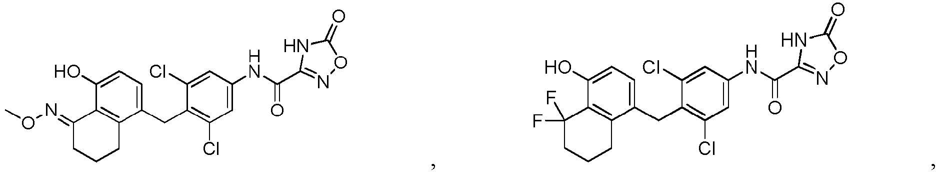

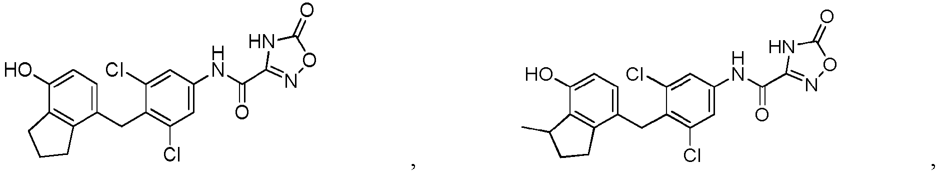

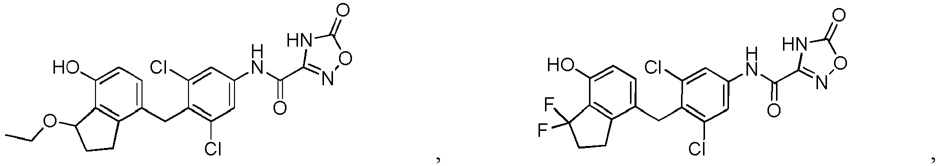

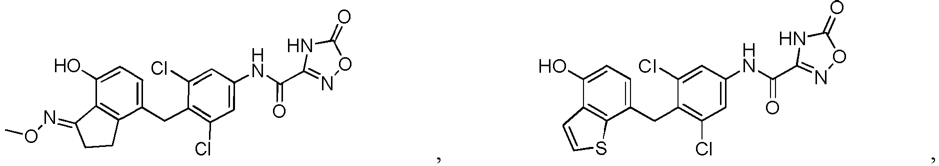

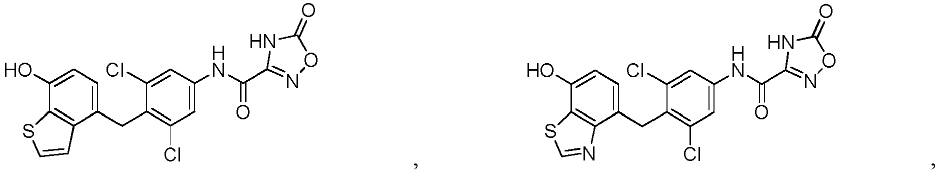

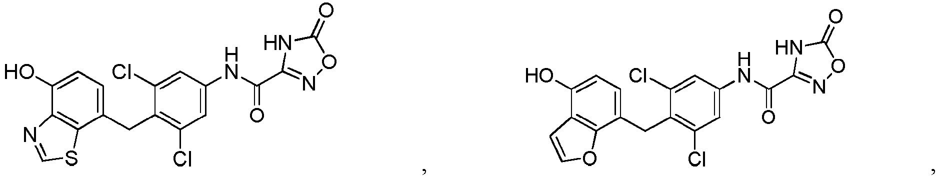

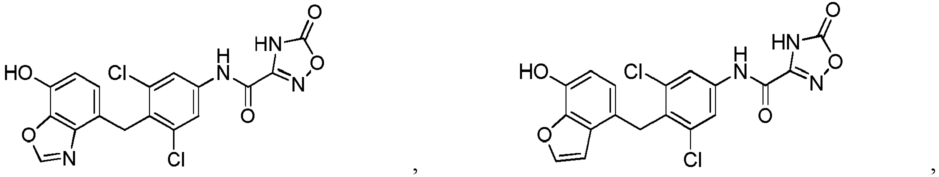

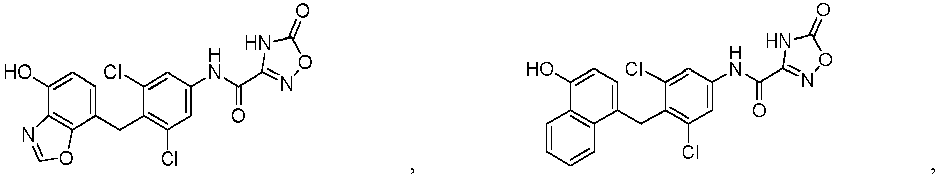

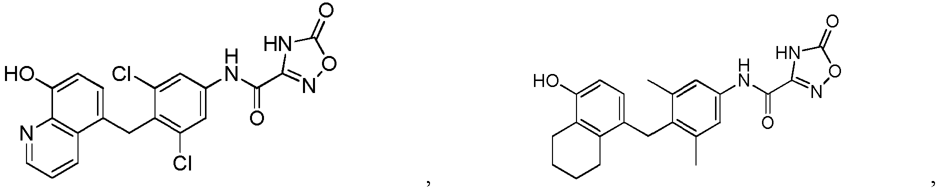

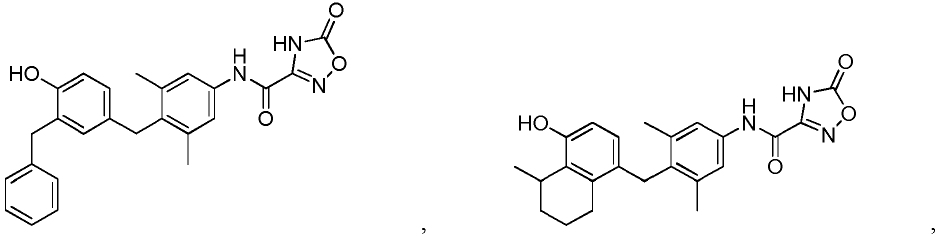

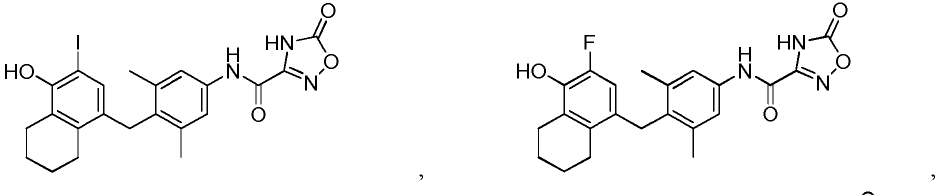

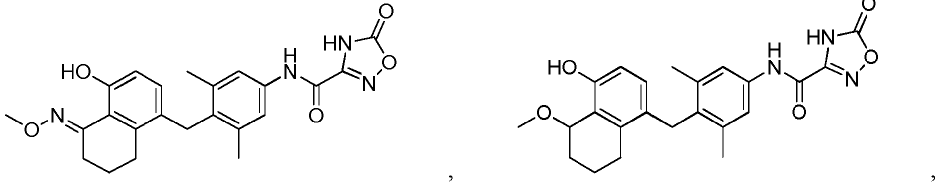

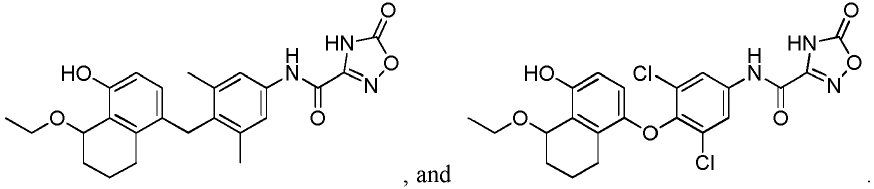

14. The compound or the pharmaceutically acceptable salt thereof according to claim 13, selected from:

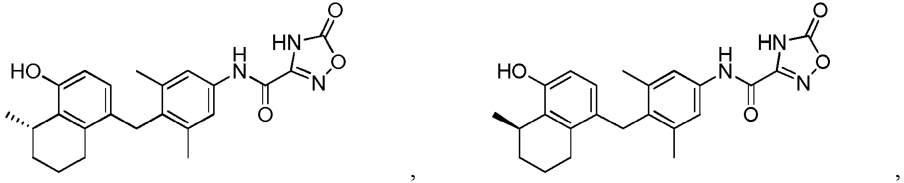

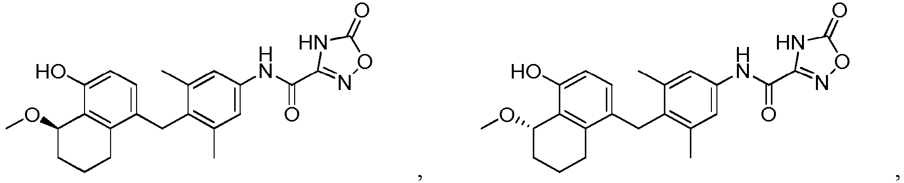

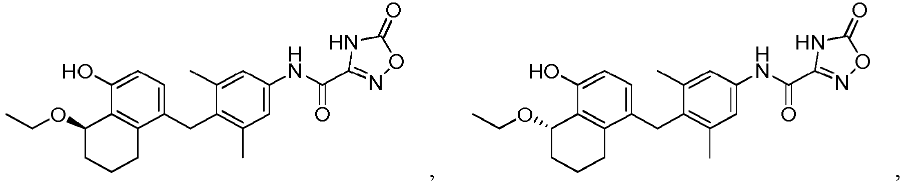

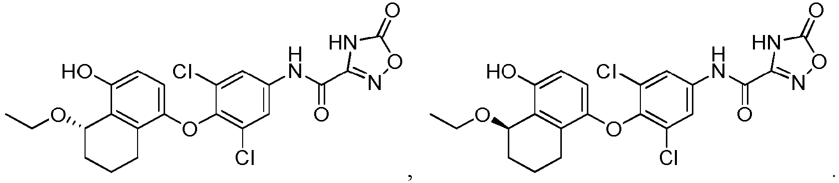

15. The compound or the pharmaceutically acceptable salt thereof according to any one of claims 1 to 14 for use in the treatment of non-alcoholic steatohepatitis.

## Patentansprüche

1. Verbindung nach Formel (I) oder pharmazeutisch akzeptables Salz davon, 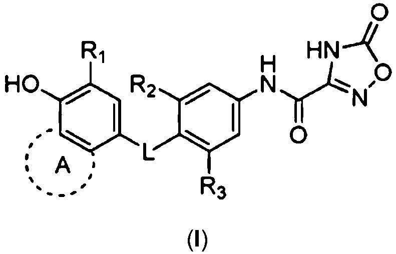 wobei
R₁ unabhängig aus H, F, Cl, Br, I und C₁₋₃-Alkyl ausgewählt ist und das C₁₋₃-Alkyl optional durch 1, 2 oder 3 Rₐ substituiert ist;
R₂ und R₃ jeweils unabhängig aus H, F, Cl, Br, I, OH, NH₂, CN, C₁₋₃-Alkyl und C₁₋₃-Alkoxy ausgewählt sind und das C₁₋₃-Alkyl und C₁₋₃-Alkoxy optional durch 1, 2 oder 3 R_{b} substituiert sind;
L aus -O- und -CH₂- ausgewählt ist;
Ring A aus Phenyl, 5- bis 6-gliedrigem Heteroaryl, 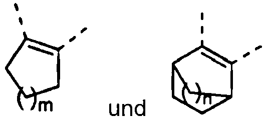 ausgewählt ist und das Phenyl, 5- bis 6-gliedrige Heteroaryl, 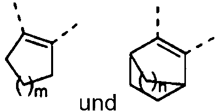 optional durch 1, 2 oder 3 R_{c} substituiert sind;
n und m jeweils unabhängig aus 0, 1 und 2 ausgewählt sind;
Rₐ und R_{b} jeweils unabhängig aus F, Cl, Br und I ausgewählt sind;
R_{c} unabhängig aus F, Cl, Br, I, =O, =N-C₁₋₃-Alkoxy, C₁₋₃-Alkyl und C₁₋₃-Alkoxy ausgewählt ist und das =N-C₁₋₃-Alkoxy, C₁₋₃-Alkyl und C₁₋₃-Alkoxy optional durch 1, 2 oder 3 R substituiert sind;
R unabhängig aus F, Cl, Br und I ausgewählt ist.

2. Verbindung oder pharmazeutisch akzeptables Salz davon nach Anspruch 1, wobei R₁ unabhängig aus H, F, Cl, Br, I und CH₃ ausgewählt ist und das CH₃ optional durch 1, 2 oder 3 Rₐ substituiert ist.

3. Verbindung oder pharmazeutisch akzeptables Salz davon nach Anspruch 2, wobei R₁ unabhängig aus H, F, Cl, Br, I, CH₃ und CF₃ ausgewählt ist.

4. Verbindung oder pharmazeutisch akzeptables Salz davon nach einem der Ansprüche 1 bis 3, wobei R₂ und R₃ jeweils unabhängig aus H, F, Cl, Br, I, OH, NH₂, CN, CH₃ und OCH₃ ausgewählt sind und das CH₃ und OCH₃ optional durch 1, 2 oder 3 R_{b} substituiert sind.

5. Verbindung oder pharmazeutisch akzeptables Salz davon nach Anspruch 4, wobei R₂ und R₃ jeweils unabhängig aus H, F, Cl, Br, I, OH, NH₂, CN, CH₃, CH₂F, CHF₂, CF₃ und OCH₃ ausgewählt sind.

6. Verbindung oder pharmazeutisch akzeptables Salz davon nach einem der Ansprüche 1 bis 3, wobei R_{c} unabhängig aus F, Cl, Br, I, =O, =N-O-CH₃, =N-O-CH₂CH₃, CH₃, OCH₃ und OCH₂CH₃ ausgewählt ist und das =N-O-CH₃, =N-O-CH₂CH₃, CH₃, OCH₃ und OCH₂CH₃ optional durch 1, 2 oder 3 R substituiert sind.

7. Verbindung oder pharmazeutisch akzeptables Salz davon nach Anspruch 6, wobei R_{c} unabhängig aus F, Cl, Br, I, =O, =N-O-CH₃, =N-O-CH₂CH₃, CH₃, OCH₃ und OCH₂CH₃ ausgewählt ist.

8. Verbindung oder pharmazeutisch akzeptables Salz davon nach einem der Ansprüche 1 bis 3, wobei Ring A ausgewählt ist aus 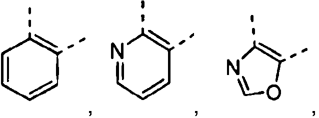 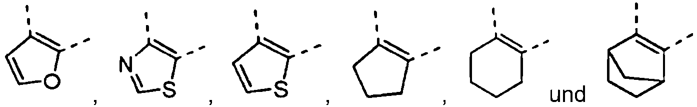 und das 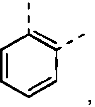 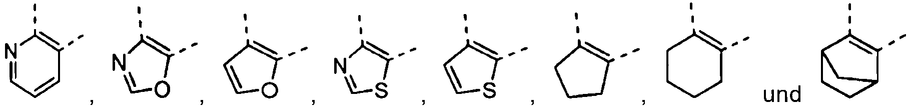 optional substituiert sind durch 1, 2 oder 3 R_{c}.

9. Verbindung oder pharmazeutisch akzeptables Salz davon nach Anspruch 8, wobei Ring A ausgewählt ist aus 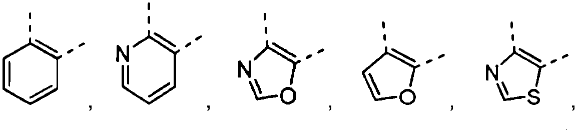 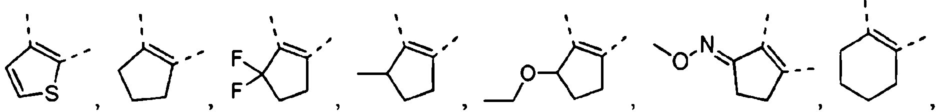 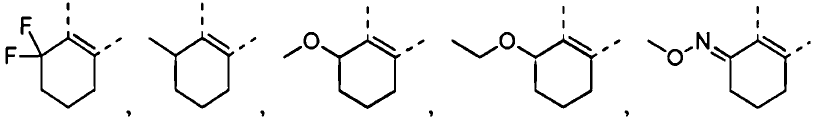 und

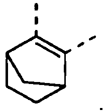

10. Verbindung oder pharmazeutisch akzeptables Salz davon nach einem der Ansprüche 1 bis 3 und 9, wobei die strukturelle Einheit 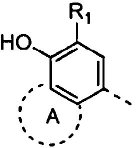 ausgewählt ist aus

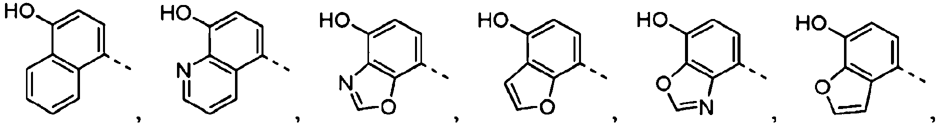

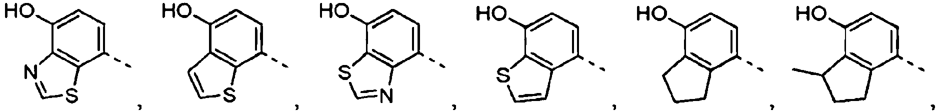

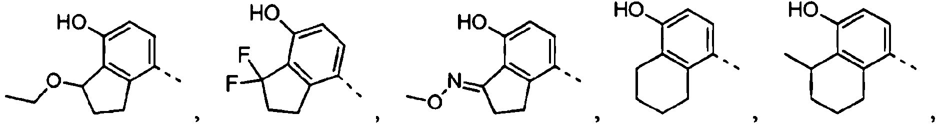

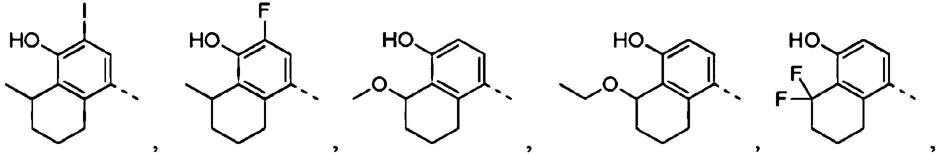

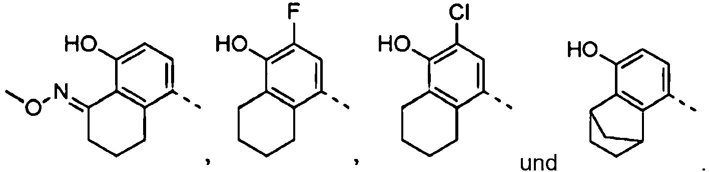

11. Verbindung oder pharmazeutisch akzeptables Salz davon nach einem der Ansprüche 1 bis 10, ausgewählt aus: 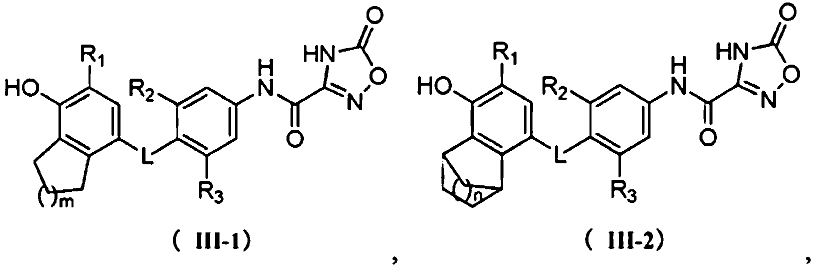 wobei R₁, R₂, R₃, L, m und n so sind, wie sie in einem der Ansprüche 1 bis 10 definiert sind.

12. Verbindung oder pharmazeutisch akzeptables Salz davon nach einem der Ansprüche 1 bis 10, ausgewählt aus: 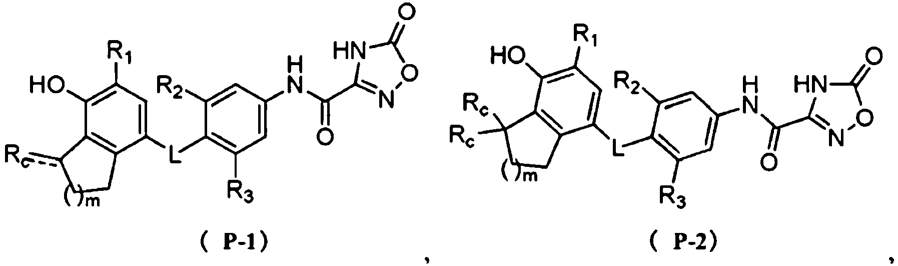 wobei
wenn 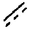 eine Einfachbindung ist, R_{c} F, Cl, Br, I, C₁₋₃-Alkyl und C₁₋₃-Alkoxy ist und das C₁₋₃-Alkyl und C₁₋₃-Alkoxy optional durch 1, 2 oder 3 Halogene substituiert sind;
wenn 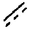 eine Doppelbindung ist, R_{c} =O oder =N-C₁₋₃-Alkoxy ist und das =N-C₁₋₃-Alkoxy optional substituiert ist durch 1, 2 oder 3 Halogene;
R₁, R₂, R₃, L und m so sind, wie in einem der Ansprüche 1 bis 10 definiert ist.

13. Verbindung oder pharmazeutisch akzeptables Salz davon nach Anspruch 1, ausgewählt aus:

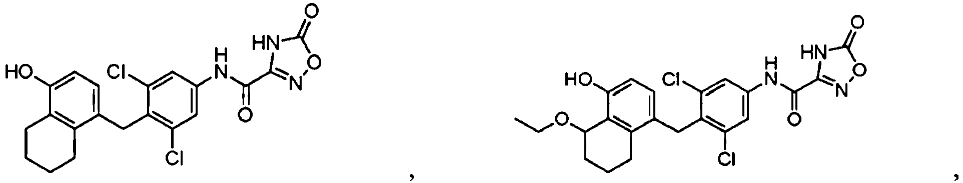

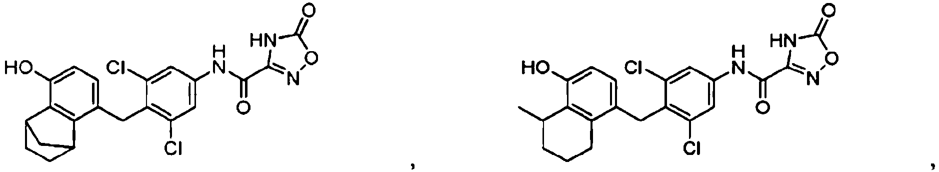

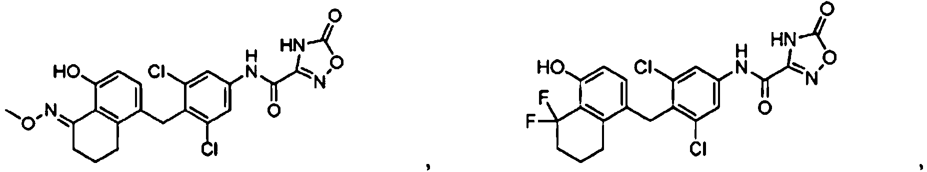

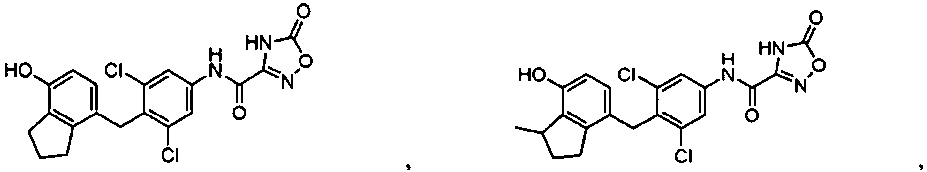

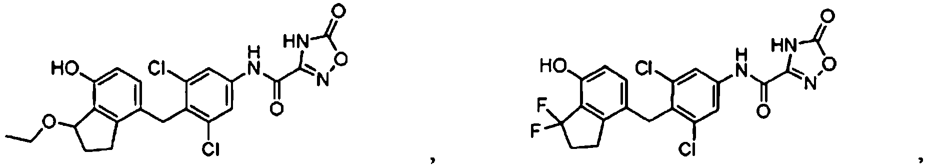

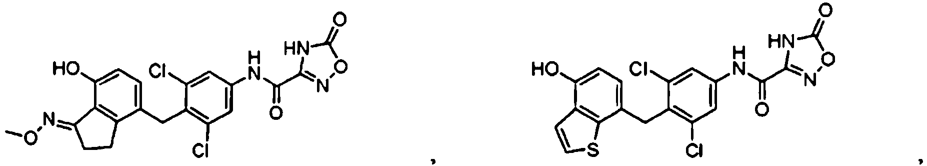

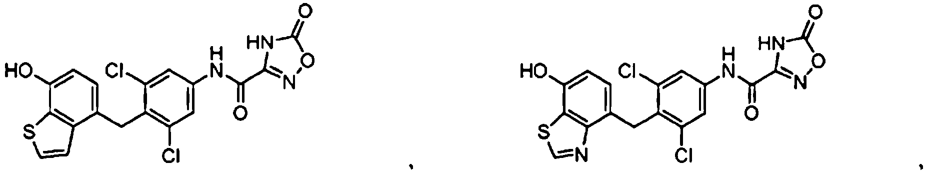

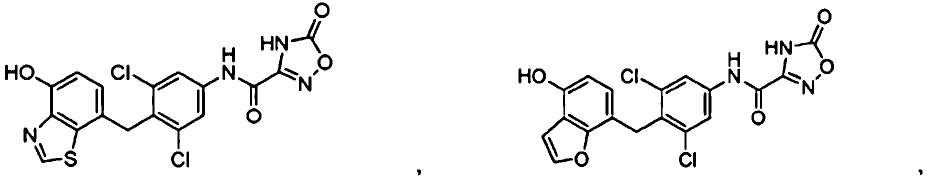

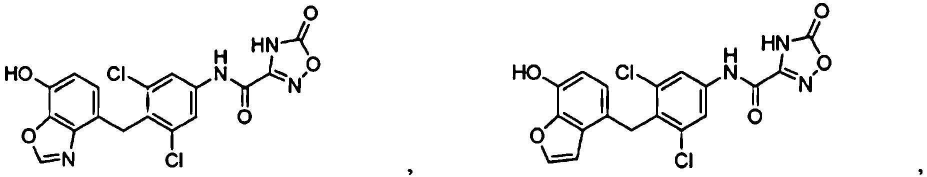

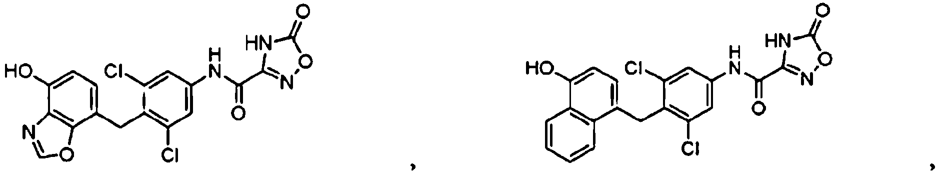

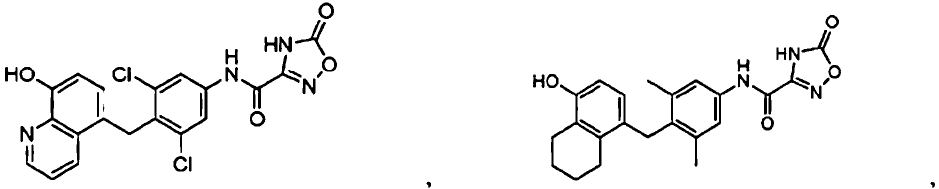

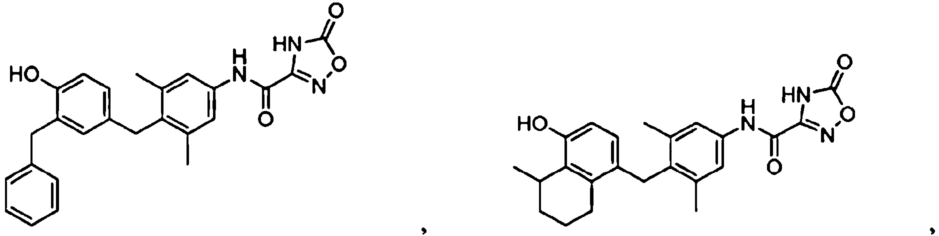

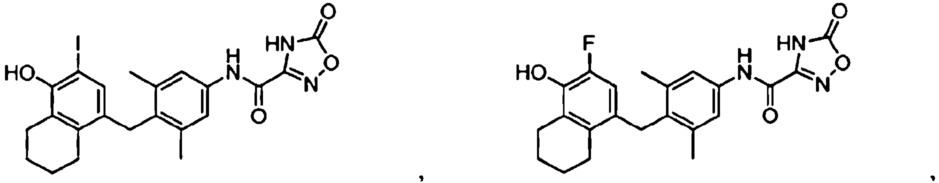

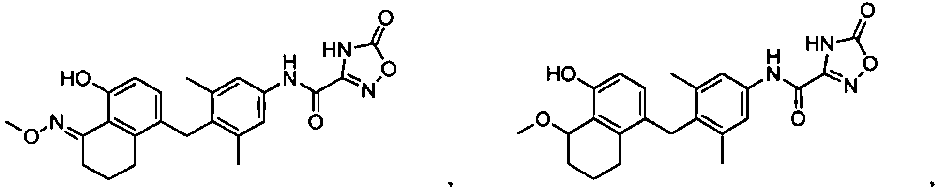

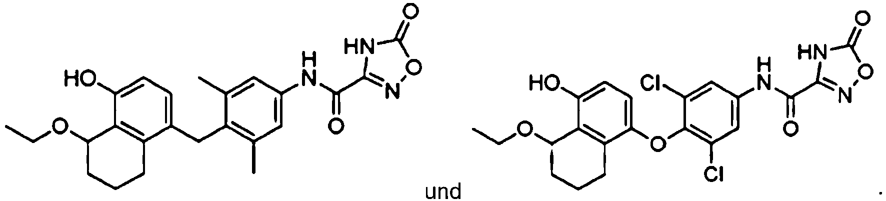

14. Verbindung oder pharmazeutisch akzeptables Salz davon nach Anspruch 13, ausgewählt aus:

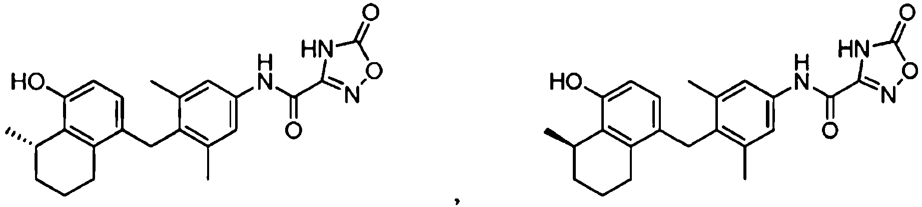

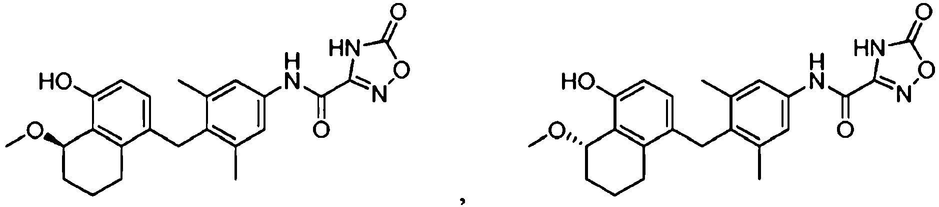

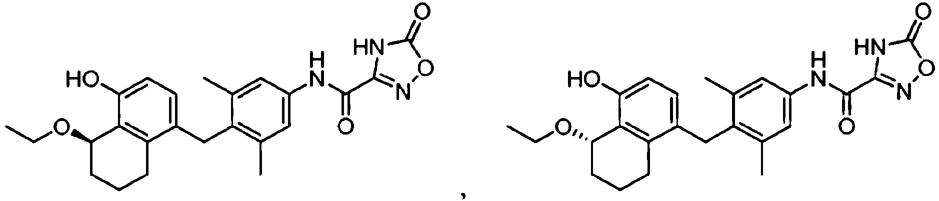

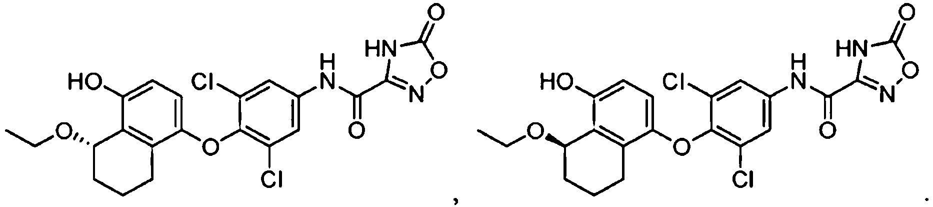

15. Verbindung oder pharmazeutisch akzeptables Salz davon nach einem der Ansprüche 1 bis 14 zur Verwendung in der Behandlung von nichtalkoholischer Steatohepatitis.

## Revendications

1. Composé de formule (I) ou sel pharmaceutiquement acceptable de celui-ci, 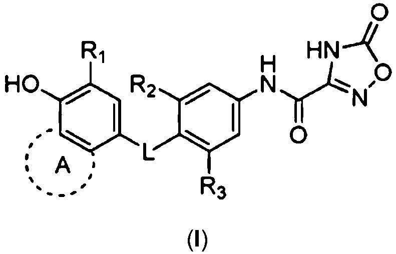 dans lequel
R₁ est indépendamment sélectionné parmi H, F, Cl, Br, I et alkyle en C₁₋₃, et l'alkyle en C₁₋₃ est facultativement substitué par 1, 2, ou 3 Rₐ ;
R₂ et R₃ sont chacun indépendamment sélectionnés parmi H, F, CI, Br, I, OH, NH₂, CN, alkyle en C₁₋₃ et alcoxy en C₁₋₃, et l'alkyle en C₁₋₃ et l'alcoxy en C₁₋₃ sont facultativement substitués par 1, 2, ou 3 R_{b};
L est sélectionné parmi -O- et -CH₂- ;
le cycle A est sélectionné parmi phényle, hétéroaryle à 5 ou 6 chaînons, 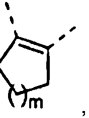 et 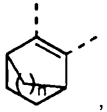 et le phényle, l'hétéroaryle à 5 ou 6 chaînons, 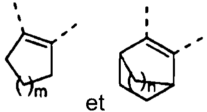 sont facultativement substitués par 1, 2, ou 3 R_{c};
n et m sont chacun indépendamment sélectionnés parmi 0, 1 et 2 ;
Rₐ et R_{b} sont chacun indépendamment sélectionnés parmi F, Cl, Br et I ;
R_{c} est indépendamment sélectionné parmi F, Cl, Br, I, =O, =N-alcoxy en C₁₋₃, alkyle en C₁₋₃ et alcoxy en C₁₋₃ , et le =N-alcoxy en C₁₋₃, l'alkyle en C₁₋₃ et l'alcoxy en C₁₋₃ sont facultativement substitués par 1, 2, ou 3 R ;
R est indépendamment sélectionné parmi F, Cl, Br et I.

2. Composé ou sel pharmaceutiquement acceptable de celui-ci selon la revendication 1, dans lequel R₁ est indépendamment sélectionné parmi H, F, CI, Br, I et CH₃, et le CH₃ est facultativement substitué par 1, 2 ou 3 Rₐ.

3. Composé ou sel pharmaceutiquement acceptable de celui-ci selon la revendication 2, dans lequel R₁ est indépendamment sélectionné parmi H, F, Cl, Br, I, CH₃ et CF₃.

4. Composé ou sel pharmaceutiquement acceptable de celui-ci selon l'une quelconque des revendications 1 à 3, dans lequel R₂ et R₃ sont chacun indépendamment sélectionnés parmi H, F, Cl, Br, I, OH, NH₂, CN, CH₃, et OCH₃, et le CH₃ et l'OCH₃ sont facultativement substitués par 1, 2, ou 3 R_{b}.

5. Composé ou sel pharmaceutiquement acceptable de celui-ci selon la revendication 4, dans lequel R₂ et R₃ sont chacun indépendamment sélectionnés parmi H, F, CI, Br, I, OH, NH₂, CN, CH₃, CH₂F, CHF₂, CF₃, et OCH₃.

6. Composé ou sel pharmaceutiquement acceptable de celui-ci selon l'une quelconque des revendications 1 à 3, dans lequel R_{c} est indépendamment sélectionné parmi F, CI, Br, I, =O =N-O-CH₃, =N-O-CH₂CH₃, CH₃, OCH₃, et OCH₂CH₃, et les =N-O-CH₃, =N-O-CH₂CH₃, CH₃, OCH₃, et OCH₂CH₃ sont facultativement substitués par 1, 2, ou 3 R.

7. Composé ou sel pharmaceutiquement acceptable de celui-ci selon la revendication 6, dans lequel R_{c} est indépendamment sélectionné parmi F, CI, Br, I, =O, =N-O-CH₃, =N-O-CH₂CH₃, CH₃, OCH₃, et OCH₂CH₃.

8. Composé ou sel pharmaceutiquement acceptable de celui-ci selon l'une quelconque des revendications 1 à 3, dans lequel le cycle A est sélectionné parmi 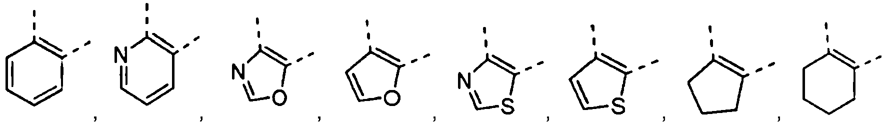 et 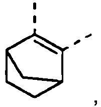 et les 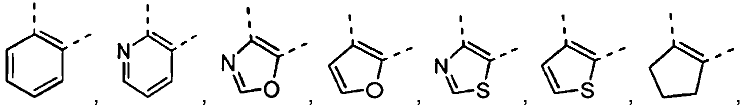 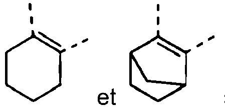 sont facultativement substitués par 1, 2 ou 3 R_{c}

9. Composé ou sel pharmaceutiquement acceptable de celui-ci selon la revendication 8, dans lequel le cycle A est sélectionné parmi 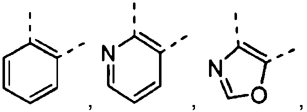 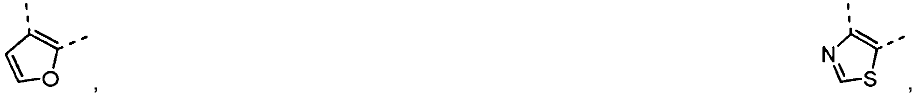 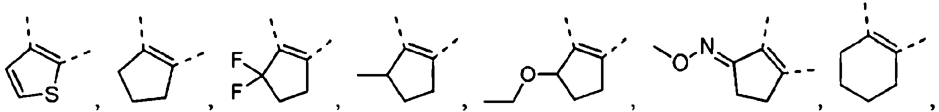 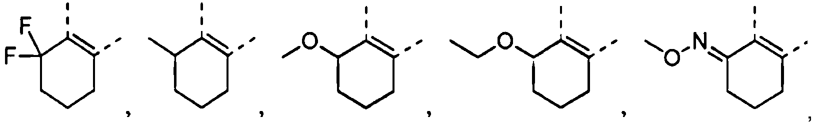 et

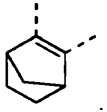

10. Composé ou sel pharmaceutiquement acceptable de celui-ci selon l'une quelconque des revendications 1 à 3 et la revendication 9, dans lequel le groupement structural 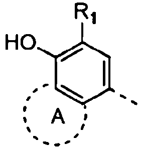 est sélectionné parmi

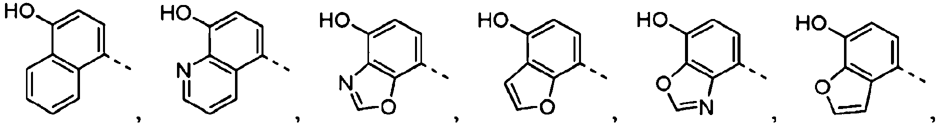

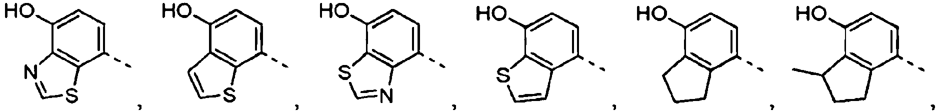

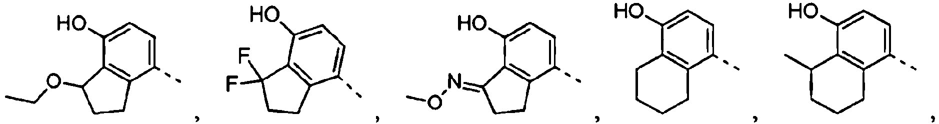

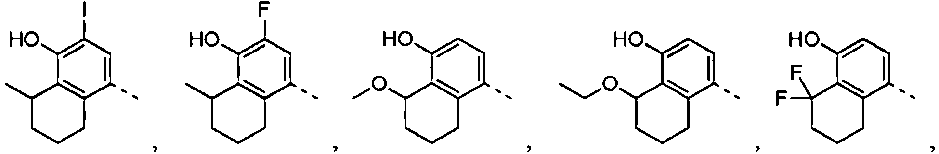

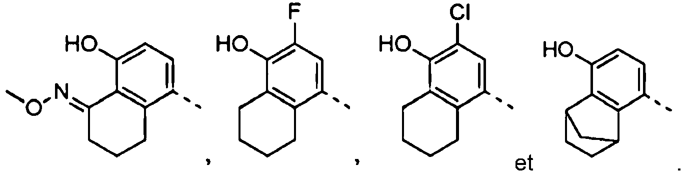

11. Composé ou sel pharmaceutiquement acceptable de celui-ci selon l'une quelconque des revendications 1 à 10, sélectionné parmi : 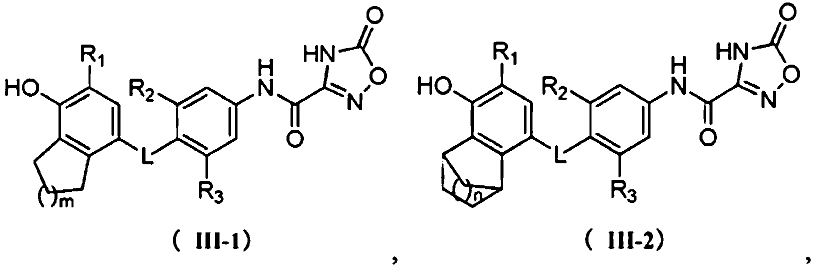 dans lequel R₁, R₂, R₃, L, m et n sont tels que définis dans l'une quelconque des revendications 1 à 10.

12. Composé ou sel pharmaceutiquement acceptable de celui-ci selon l'une quelconque des revendications 1 à 10, sélectionné parmi : 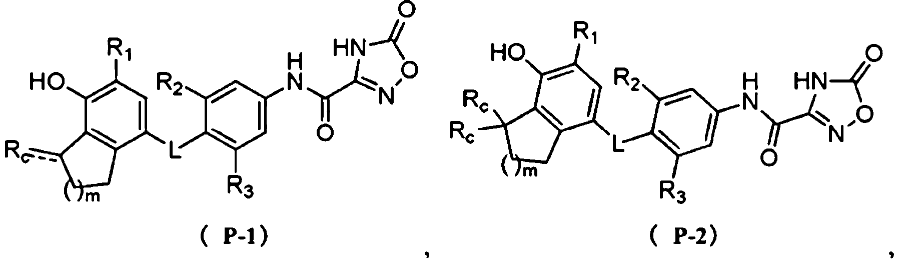 dans lequel
lorsque 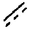 est une liaison simple, R_{c} est F, CI, Br, I, alkyle en C₁₋₃ et alcoxy en C₁₋₃, et l'alkyle en C₁₋₃ et l'alcoxy en C₁₋₃ sont facultativement substitués par 1, 2 ou 3 halogènes ;
lorsque 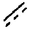 est une double liaison, R_{c} est =O ou =N-alcoxy en C₁₋₃, et le =N-alcoxy en C₁₋₃ est facultativement substitué par 1, 2, ou 3 halogènes ;
R₁, R₂, R₃, L et m sont tels que définis dans l'une quelconque des revendications 1 à 10.

13. Composé ou sel pharmaceutiquement acceptable de celui-ci selon la revendication 1, sélectionné parmi :

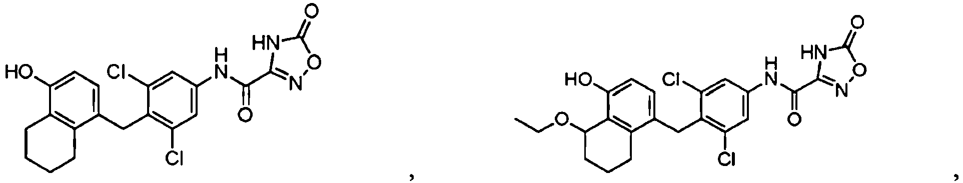

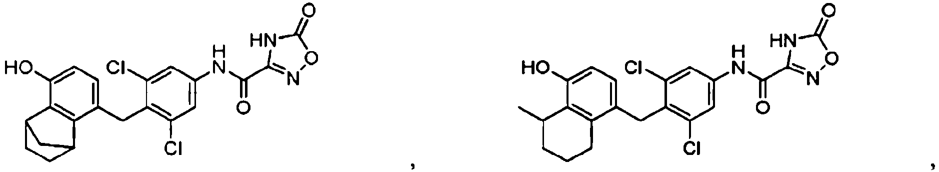

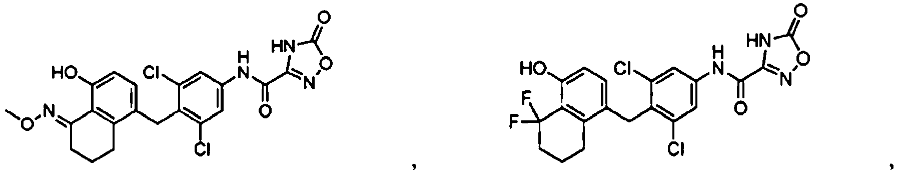

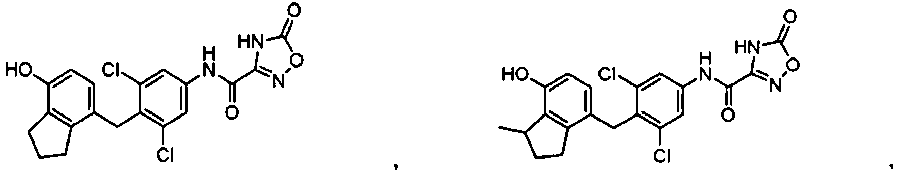

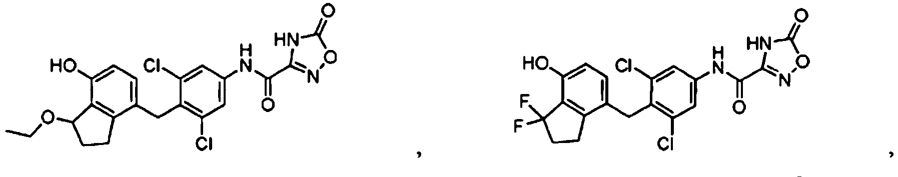

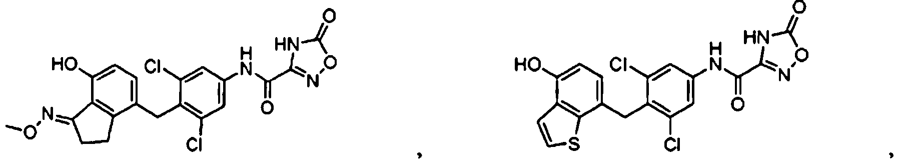

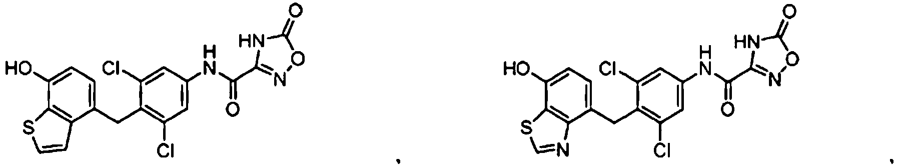

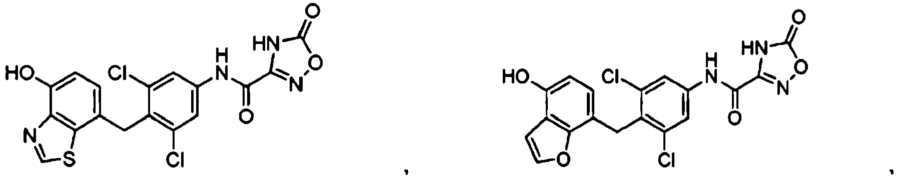

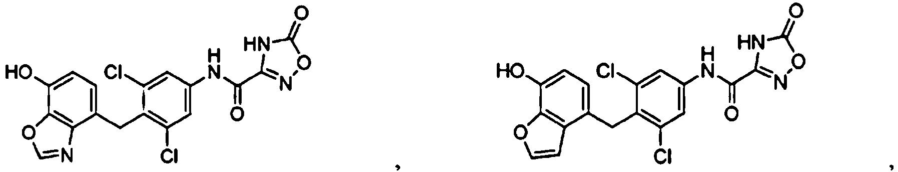

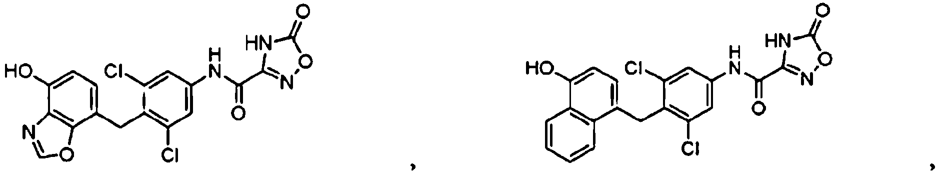

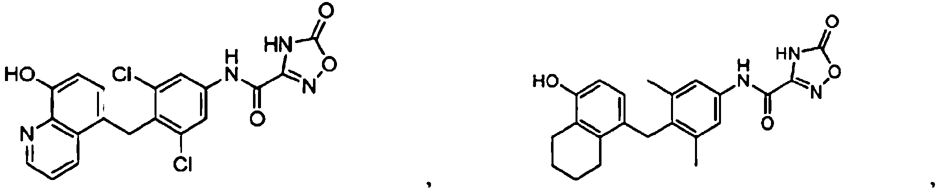

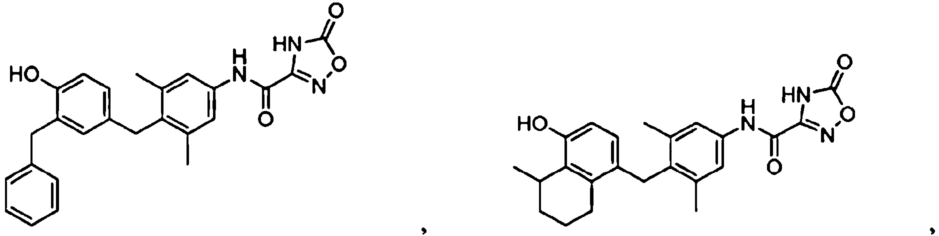

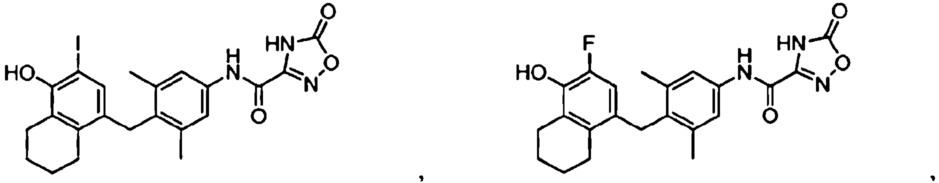

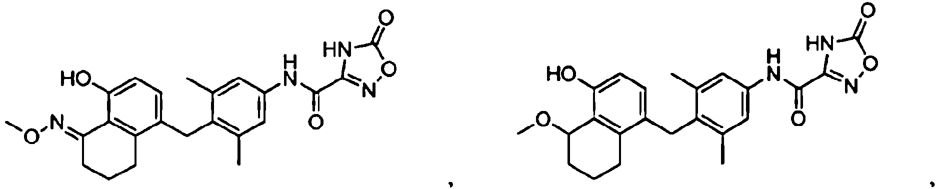

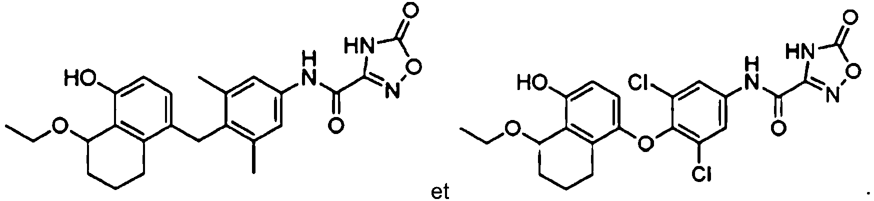

14. Composé ou sel pharmaceutiquement acceptable de celui-ci selon la revendication 13, sélectionné parmi :

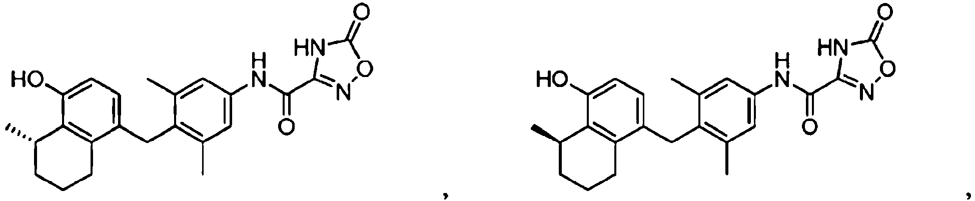

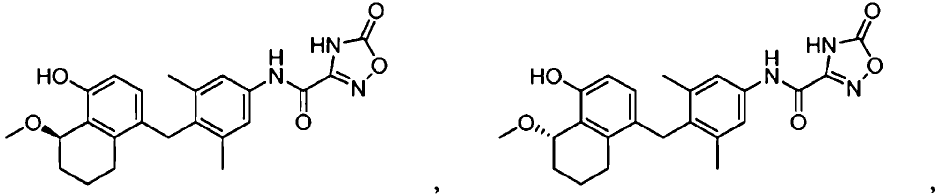

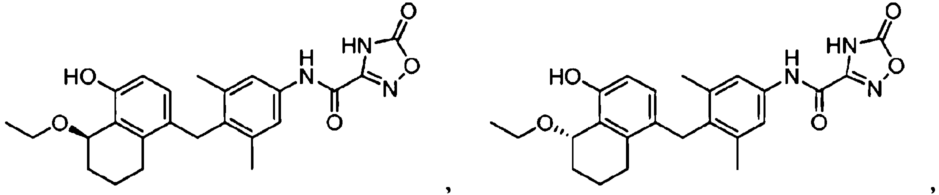

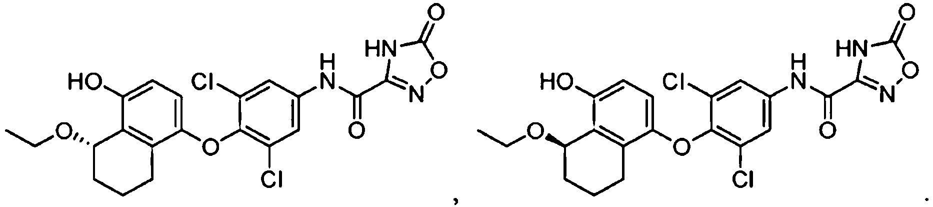

15. Composé ou sel pharmaceutiquement acceptable de celui-ci selon l'une quelconque des revendications 1 à 14 pour une utilisation dans le traitement de la stéatohépatite non alcoolique.
